# EUROPEAN PATENT SPECIFICATION

(11) **EP 3 359 259 B1**
(45) Date of publication and mention of the grant of the patent: **15.11.2023**
(21) Application number: 16854140.7
(22) Date of filing: 03.10.2016
(51) Int. Cl.: A61P 39/00, A23L 33/13, A23L 33/175, A61K 36/48, A61K 9/00, A61K 9/20, A61K 31/198, A61K 31/706, A61K 36/258, A61K 33/04, A61P 29/00, A61P 37/04, A61K 33/40, A61K 31/714, A61K 31/045, A61K 31/205

(54) **RESETTING BIOLOGICAL PATHWAYS FOR DEFENDING AGAINST AND REPAIRING DETERIORATION FROM HUMAN AGING**
ZURÜCKSETZEN BIOLOGISCHER WEGE ZUR ABWEHR UND BEHEBUNG VON ALTERUNGSERSCHEINUNGEN BEIM MENSCHEN
RÉINITIALISATION DE VOIES BIOLOGIQUES DE DÉFENSE CONTRE ET DE RÉPARATION D'UNE DÉTÉRIORATION CAUSÉE PAR LE VIEILLISSEMENT HUMAIN

(30) Priority: 07.10.2015 US 201562238338 P
(43) Date of publication of application: 15.08.2018
(73) Proprietor: Huizenga, Joel, La Jolla, California 92037 (US)
(72) Inventor: Huizenga, Joel, La Jolla, California 92037 (US)
(74) Representative: Dehns
(86) International application number: PCT/US2016/055173
(87) International publication number: WO 2017/062311

(56) References cited:
- WO-A1-2016/158212
- US-A1- 2004 204 746
- US-A1- 2007 253 919
- US-A1- 2009 214 628
- US-A1- 2015 072 950
- Sue Dunlevy: "University of NSW research finds compound that can reverse ageing", news.com.au/lifestyle/health, 19 December 2013 (2013-12-19), pages 1-2, XP055570000, Internet Retrieved from the Internet: URL:https://www.news.com.au/lifestyle/heal th/university-of-nsw-research-finds-compou nd-that-can-reverse-ageing/news-story/2db5 2e293fb786faf08acf57da7d0bb7 [retrieved on 2019-03-15]
- MARIA GIULIA BACALINI ET AL: "Present and future of anti-ageing epigenetic diets", MECHANISMS OF AGEING AND DEVELOPMENT., vol. 136-137, 1 March 2014 (2014-03-01), pages 101-115, XP055570022, CH ISSN: 0047-6374, DOI: 10.1016/j.mad.2013.12.006
- E. K. GO ET AL: "Betaine Suppresses Proinflammatory Signaling During Aging: The Involvement of Nuclear Factor- B via Nuclear Factor-Inducing Kinase/I B Kinase and Mitogen-Activated Protein Kinases", JOURNALS OF GERONTOLOGY, SERIES A, BIOLOGICAL SCIENCES ANDMEDICAL SCIENCES, vol. 60, no. 10, 1 October 2005 (2005-10-01), pages 1252-1264, XP055570016, US ISSN: 1079-5006, DOI: 10.1093/gerona/60.10.1252
- ALARCON DE LA LASTRA CATALINA ET AL: "RESVERATROL AS AN ANTI-INFLAMMATORY AND ANTI-AGING AGENT: MECHANISMS AND CLINICAL IMPLICATIONS", MOLECULAR NUTRITION & FOOD RESEARCH, WILEY - VCH VERLAG, WEINHEIM, DE, vol. 49, no. 5, 1 May 2005 (2005-05-01), pages 405-430, XP009077313, ISSN: 1613-4125, DOI: 10.1002/MNFR.200500022

## Description

### FIELD

The disclosed subject matter generally relates to compositions for defending against and repairing the effects of aging.

### BACKGROUND

Early human ancestors did not have enough energy to do everything they could benefit from doing. Energy availability limitations during early human evolution led to biological tradeoff mechanisms governing energy use, which limits energy use for defense and repair of human cellular damage. Cellular damage has been proposed to be causal for human biological aging (Olson C 1987, Holliday R 2004, Kirkwood T 2005, Gavrilov L 2001). Human biological aging has been proposed to be causal for the human "Diseases of Aging" (Cutler R 2006).

Sue Dunlevy ("University of NSW research finds compound that can reverse ageing", news.com.au/lifestyle/health, 2013, pages 1-2, XP05570000) discloses NMN as an agent for anti-ageing and anti-inflammation.

Maria Giulia Bacalini et al. ("Present and future of anti-ageing epigenetic diets", Mechanisms of Ageing and Development, Vol. 136-137, 2014, pages 101-115), discloses the effects of certain diets on inflammation and age-related diseases.

E. K. Go et al. ("Betaine suppresses proinflammatory signaling during aging: The Involvement of Nuclear Factor B via Nuclear Factor-Inducing Kinase/I B Kinase and Mitogen-Activated Protein Kinases", Journals of Gerontology, Series A, Biological Sciences and Medical Sciences, Vol 60, No. 10, 2005, pages 1252-1264) discloses that betaine is an important human nutrient found in many foods. Specifically, betaines are described as supressing NF-kB activities and having anti-inflammatory and anti-aging effects.

Alarcon de la Lastra Catalina et al. ("Resveratrol as an anti-inflammotory and anti-aging agent: mechanisms and clinical implications", Molecular Nutrition and Food Research, Vol.49, No. 5, 2005, pages 405-430) relates to the possible anti-inflammatory mechanisms of resveratrol.

WO2016/158212 concerns a food composition comprising resveratrol and nicotinamide mononucleotide.

### Feedback loops are a part of energy tradeoff controls

Feedback loops in biochemical synthetic pathways turn off energy expenditures in areas of non-use to increase efficiency of total organismic energy use. Use of energy and nutrients in evolution needed to be balanced with the ability to obtain these calories and nutrients from the environment, which was varied and limited. In part this was achieved by what in medicine is known as "The use it or lose it principle," which is adaptive upregulation / down-regulation based on need. An example is that of the antioxidant enzymes systems that are turned to lower settings if they do not receive, over time, new oxidative pulses to keep the antioxidant enzymes on higher settings.

### Cellular repair involves energy trade offs

Energy that is used for the repair of cellular damage is energy that is not available to use for other functions that are beneficial for cellular function and life. Cellular repair systems and the complex human immune systems represent two such competing energetically demanding systems that compete for energy use. In their "Disposable Soma Theory of Aging" Kirkwood and Rose (Kirkwood and Rose 1991) proposed that to optimize energy use, biological systems may invest most of their energy in growth and development and little in damage control and repair for non-germline (soma) cells.

### Energy availability from diet effects energy tradeoffs

The second law of thermodynamics teaches that the state of entropy in a closed system can only change in one direction over time. Animals need to eat food, thus maintaining an open system, to improve, repair, or maintain their structure over time at the expense of the food they eat, which gains in entropy becoming fecal matter.

In evolution, food and its nutrients and energy were often limiting and only sporadically available. Evolution had to adapt for this. In calorie restricted times, energy pathways adapted for these limitations. These pathways have benefits. The beneficial effect of calorie restriction is seen by recent research to be directed by Sirtuin enzymes. Sirtuin enzymes are involved in human cellular repair. There are 7 known human Sirtuin enzymes. All 7 of these human Sirtuin enzymes use NAD+ (Imai S 2000). Nicotinamide is the end product of these Sirtuin reactions.

An example of a feedback loop in Sirtuin pathways is that the end product, nicotinamide, is able to bind to the Sirtuin enzymes and decrease their enzymatic properties. The feedback loop changes if the nicotinamide is methylated by the human nicotinamide-N-methyltransferase (NNMT) enzyme in the cell using S-5' Adenosyl-L- methionine (SAM). The new methylated nicotinamide is then unable to bind in the nicotinamide binding site because of steric hindrance of the physical size of the newly attached methyl group to the nicotinamide (Schmeisser K 2013). With this methylation change the Sirtuin enzymes are able to keep working instead of stopping their activity.

### Defenses against pathogens comes with energy tradeoffs

People with disease, especially chronic disease, age faster. The innate immune system (example white blood cells) when throwing oxidants (example Cl-) at pathogens to kill them creates background damage in its own cells that leads to faster aging for the organism. Pathogens have been a major killer of humans, so without energy for fighting these pathogens, individuals would be more quickly removed from evolution. Making this trade off of how much energy to expend in a pathogen attack, how much energy to use to repair the damage from a pathogen attack, and even the energy to use to heighten the immune system to be ready for an immune attack, are all important tradeoffs in evolution.

An example of this tradeoff is seen in a study of 684 individuals over 100 and 536 individuals 85 to 99 years old in Japan (Arai Y 2015). Lower levels of inflammation (4 immune variable composite score) was the best predictor of who was going to continue to live (life-span) and be physically and cognitively healthy (health-span). Immune markers (a simple index of serum interleukin-6 (IL-6) and tumor necrosis factor alpha (TNF-alpha) which were two of the Arai 4 markers) were found to be the best predictor of mortality in 1,155 older adults in a 10 year all-cause mortality study after adjusting for variables already known to cause death (Varadhan R 2014). Just one immune marker, Serum IL-6, predicted all-cause mortality, cancer, cardiovascular disease and liver disease in a 1843 person prospective cohort study (Lee JK 2012). These studies confirmed results in smaller prior studies (Derhovanessian E 2010, Reuben DB 2002, Taaffe DR 2000).

The biological cellular mechanism of this tradeoff may be due to when Nrf2 releases Keap1, it is available to capture IKKBeta thus inhibiting NF-kB target genes. This interaction correlates the expression of antioxidant enzymes by Nrf2 and the turning on and off of the immune system by NF-kB.

### Sexual animals have energy use tradeoffs that asexual animals do not have

Asexual animals like sea anemones do not age. There is no apparent senescence in asexually reproducing Hydra, but there are signs of aging when Hydra reproduce sexually (Yoshida K 2006). Hydra share 6071 genes with humans (Wenger Y 2013) and at least 80% of known human aging genes are shared with Hydra (Tomczyk S 2014). Research has shown sexual animals, like humans, age faster in somatic cells after puberty and less if sexual hormones are lowered. A human example is eunuchs in India and Korea, with no testicles, live on average 9 to 13 years longer. In flat worms, a research model named C. elegans, the heat shock response (HSR), essential for proteostasis and cellular health, is repressed after sexual maturity in somatic (non-sexual) cells by germline (sexual) cells by triple methylation at stress gene loci. This competition between the interest of the germline and the soma cells (Kirkwood TL 2000) determines the rate of aging in sexually mature individuals (Labbadia J 2015). Research also shows trade-offs between ability to bear children and aging. An example is use of low dose RU-486, the abortion drug, gives lower fertility and longer life span on average (Landis G. 2015). Bearing children, and especially bearing children later in life (Sun F 2015, Perls TT 1997), has been linked to increases in the probability of a longer life in women, although cause and effect is still uncertain. Time of menopause has also been correlated to rate of aging.

### Energy use tradeoffs seen with chronological age

In youth, humans have an excess or a reservoir of ability and energy in excess of what is needed by cells and organs on average but this decreases with age. In youth one has less knowledge and wisdom and smaller body size but evolution makes up this deficit with a higher metabolism allowing more energy expenditure (especially per body mass) thus one is able to live more per unit of time. Higher metabolism is correlated with faster aging generally across species, although humans are known to age faster after puberty showing this need not be a hard and fast rule. The "Rate of Living" theory (Pearl R 1928) was updated to "Livingness" (Sohal R 2012) to include temperature, hibernation, fecundity and metabolic potential from initial oxygen-use observations (Rubner M 1908). Older individuals have more experience, knowledge and wisdom and are thus able to still maintain themselves in life with less energy consumption. This lower energy production may at least partially be due to the decline of the quantity and function of mitochondria to produce energy during one's life span.

### Extra brain energy used in humans comes with energy tradeoffs

Animals are known to trade off larger brain size with smaller fat reserves and smaller musculature. Humans have done both in evolution for the increased energy needed (the human brain uses about 30% of the organism's energy) by our larger (and 3X more dense with cells) brain (per body size). This indicates energy was in short supply in evolution for human ancestors. Cooking food to make its energy more available helped with this energy equation as well.

### Exercise comes with energy tradeoffs

Because of the "use it or lose it" principle of biological feedback loops, more exercise will continue the energy flow to tissue and biological systems like muscles and anti-oxidant defense systems when they are used in excess of the normal amount. When they are not used the body turns off energy flow to them to conserve energy. It has been known by medical science for a while that exercise in the long term is "good" for humans, but exercise in the short term is "bad" for humans.

The mechanism of this effect is seen as that the "bad" of exercise comes from the release of oxidants, including the oxidants from energy production in the mitochondria. This pulse of oxidation turns on defense and repair mechanism and then this in turn benefits the cells and body during the non-exercising hours of the day, this is called oxidative preconditioning.

### Sleep is an energy use tradeoff

All animals with neurons sleep. With sleep, one gets more time to repair cellular damage, and thus extend one's life quality and length at the expense of the things that cannot be accomplished during sleep hours.

### Biological aging vs / chronological aging

The amount of human biological aging has been shown to vary during a chronological year. In a study (Belsky DW 2015) of 954 "young" humans in their third and fourth decade of life (at studies end, 38 chronologically years old and without signs of the diseases of aging), all born in a one year time period in the same New Zealand town, aged at rates (biologically years old) that varied from 1 biological year per chronological year to nearly 3 biological years per chronological year as determined by a grouping of 10 diagnostic tests measured at 3 time points. 3 of the 954 even appeared to have reversed biological age in the time period. This variation in the amount of human biological aging in a chronological year indicates that the rate of biological aging in humans is not fixed and has the possibility to be changed.

### A "Unified Theory of Aging"

Over the years, four major theories of aging have developed. These four general theories have arisen from numerous branches of scientific inquiry. The four major theories of aging are:
- The calorie restriction theory of aging, (McCay C 1935)
- The free radical theory of aging (now called Redox), (Harmon D 1956)
- The methylation theory of aging in 1967 (Vanyushin B 2005), and
- The somatic mutation theory of aging (Szilard L 1959).

Others aging theories include:
- Rate of Living Theory of Aging (Pearl R 1928, Rubner M 1908, Sohal R 2012)
- Disposable Soma Theory of Aging (Kirkwood and Rose 1991)
- Redox Stress Hypothesis of Aging (Sohal R 2012)
- Inflammaging (Franceschi C 2007, 2007, 2014) Para-inflammation (Medzhitov R 2008)
- "Metchnikoff's Hypothesis of Aging" (Metchnikoff E 1901)

There are connections and overlaps between all these nine theories of aging, and the compounds, compositions, formulations, and compositions for use disclosed herein further support these theories and in fact provide for their unification.

### Caloric restriction (CR) theory of aging

In 1935, Clive McCay first discovered that caloric restriction (CR) increased life span in animals. CR is the practice of reducing caloric consumption without inducing malnutrition. This requires an organism receive adequate amounts of water, vitamins, minerals, and protein, but limits carbohydrate and fat calories (to less than the recommended dietary allowance (RDA) for humans). CR can be done safely without harmful health effects with total caloric restrictions in the range of 10-40% less than RDA recommendations. In 1986, Richard Weindruch showed that restricting calories to 2/3rds of the normal amount in mice increased lifespan by 40%. To date, a large number of experiments in animal models have corroborated these results. Animal models of CR have also helped researchers discover the molecular biology pathways that account for the increase in life span and health span (Colman RJ 2014). A randomized controlled two year calorie restriction study in humans (Ravussin E 2015) showed feasibility and effects on predictors of health-span and longevity (life-span).

### Sirtuins and Caloric Restriction

In the 1990s, a MIT research team led by Leonard Guarente discovered that a certain enzyme found in yeast was a "nutrient sensor" and could possibly be the molecular mechanism that would explain the effects of caloric restriction (Guarente L 2000). In yeast, caloric restriction increased the life span of yeast by 40%. When this enzyme, called Sirtuin, was "knocked out", the yeast did not live longer in response to caloric restriction.

### Sirtuins, NAD+, and the solution to the rate-limiting step of NAD+ biosynthesis

All Sirtuin enzymes required a cofactor called nicotinamide adenine dinucleotide (NAD+) (Imai S 2000). This compound is naturally occurring, found in all cells and is one of the "energy currencies" of the cell; much like ATP. NAD+ is the "depleted energy form" of NADH, which is the actual "energy currency form" of the molecule. Thus NAD+ is a "signal" that the cell is out of energy and this "signal" activates and is used by the Sirtuin enzymes. This explains how caloric restriction, which is an "energy depletion state", can activate the cell to trigger cellular stress pathways to promote survival. All 7 of the Sirtuins found in humans appear to be triggered by cellular nutritional stress. NAD+ is the trigger for this response. NAD+ is produced from nicotinamide mononucleotide (NMN) and NMN can be made by an enzyme called NAMPT. NAD+ has a half-life of 3 to 5 hours in unstressed cells (Suave A lab: reported in Canto C 2013). Unfortunately, in humans there does not seem to be not enough NAD+ being made in the body due to energy-use-regulation. In 2011, it was shown that the regulatory stopping point in the synthesis of NAD+ is the enzyme NAMPT, which converts the precursor of NMN into the compound NMN. When NMN is given to mice, they create NAD+ out of the NMN in 15 minutes. Thus the solution for the "NAD+ synthesis restriction problem" is to bypass the rate-limiting step, which was the production of NMN. This was demonstrated in 2011 (Jun Yoshino and Kathryn Mills 2011).

### Human Sirtuins 1, 2, 3, 4, 5, 6, 7

### Sirtuin 1 (Sirt1)

Sirtuin 1 (Sirt1) is localized in the nucleus and the cytoplasm. It is extremely sensitive to H₂O₂ oxidation inhibition. Extracellular concentrations as low as 1 µM of H₂O₂ inhibit Sirt1 by oxidizing critical cysteine residues in the Sirtuin active center (Jung S-B 2013). In addition, the RNA-binding protein HUR binds to the 3' untranslated region of the mRNA encoding Sirt1, leading to its stabilization and increased levels. H₂O₂ triggers the dissociation of HUR from the HUR-Sirt1 mRNA complex, promoting Sirt1 mRNA decay, reducing Sirt1 abundance, a process that seems to be regulated by Chk2 kinase (Abdelmohsen K 2007). Redox Factor-1 (REF-1) was found to chemically reduce Sirt1 cysteine residues, stimulating its activities (Jung S-B 2013). REF-1, which maintains sulfhydryl (thiol) groups of cysteine residues in Sirt1 in reduced form protecting Sirt1 from H₂O₂ oxidation, has also been called APE1 (Apurinic/Apyrmidinic endonuclease)-1 because in a separate active site on the enzyme it is the rate limiting enzyme in mammalian base excision repair pathway. Sirtuin 1 is the most studied human sirtuin to date.

### Sirtuin 2 (Sirt2)

Sirt2 is mainly in the cytoplasm (Yudoh K 2015, Gomes P 2015). Sirt2 is important in regulation of the cell cycle (Nie H 2014). It has been shown to be a histone deacetylase (Moscardo A 2015). It has been shown to maintain faithful chromosome division and replication (Kim HS 2011). A reported mechanism for this is the Sirt2 deacetylation of ATR-interacting protein (ATRIP) at lysine 32 in response to replication stress. BubR1, a mitotic checkpoint kinase, is a deacetylation target of Sirt2. By deacetylation of lysine 668, Sirt2 stabilizes BubR1 and keeps it from ubiquitination and degradation. This leads to a striking 58% (122% for male) median life span increase and 21% maximal life span increase in mice (North BJ 2014).

Sirt2 activity has been correlated to a decrease in depression (in a rat model system that created depression through stress) possibly by increasing neurogenesis (Liu R 2015).

### Sirtuin 3 (Sirt3)

Sirtuin 3 is localized in the mitochondrial inner membrane and is an important regulator of cellular energy homeostasis (Nogueiras R 2012). A specific Sirt3 allele activated enhanced activity level and has been shown to be necessary for a life span over 90 in humans (Rose G 2003, Bellizzi D 2005, Halaschek-Wiener J 2009). Sirt 3 is the dominant mitochondrial deacetylase activity (Lombard DB 2007). Sirt3 expression, in the liver, increases after fasting (Hirschey MD 2010). Sirt3 expression in the muscle increases after exercise (Hokari F 2010), fasting, and caloric restriction and decreases with chronic high fat eating (Palacios OM 2009). Overall these studies indicate Sirt3 acts as a master switch that is adaptive to energy shortage (Cho E-H 2014) to maintain ATP production, including the metabolic switch known as the Warburg effect (Guarente L 2014). Sirt3 deacetylates at lysine 926 and 931 to activate OPA1, a mitochondrial fusion protein, elevating its GTPase activity. About 20% of mitochondrial proteins can be acetylated. Protein acetylation / deacetylation is thought to be a major regulatory mechanism in the mitochondria (Kim SC 2006). The role of Sirt3 in regulating mitochondrial biogenesis via activation of the PGC-alpha/ERR-alpha complex has been demonstrated (Giralt A 2012, Hirschey MD 2011, Kong X 2010).

Sirt3-dependent pathways are a putative molecular link between sleep-loss and neurodegeneration (Fifel K 2014, Zhang J 2014). Sirt3 mediates reduction of oxidative damage and prevention of age-related hearing loss (Someya S 2010) with OPA1 (Leruez S 2013). Sirt3 has also been implicated in Alzheimer's disease, Huntington's disease, Parkinson's disease, amyotropic lateral sclerosis (Kincaid B 2013) and Non-alcoholic fatty liver disease (Cho E-H 2014).

### Sirtuin 4 (Sirt4)

Sirtuin 4 is localized in the mitochondria. It is a cellular lipoamidase (or delipoylase), removing lipoyl modifications from lysine residues of substrates. Sirt4 delipoylates and modulates the activity of pyruvate dehydrogenase complex (PHD), which in turn inhibits the production of acetyl-CoA (Mathias RA 2014). It deacetylates malonyl-CoA decarboxylase (MCD) to regulate lipid catabolism (Laurent G 2013). It also performs ADP-ribosylation on glutamate dehydrogenase (GLUDI) (Haigis MC 2006).

### Sirtuin 5 (Sirt5)

Sirtuin 5 is localized in the mitochondria. Sirt5 desuccinylates, demalonylates, and deglutarylates protein substrates such as carbamoyl phosphate synthase 1 (CPS1) to regulate the urea cycle (Du J 2011, Peng C 2011, Tan M 2014). Sirt5's deacetylating activity is weak (Du J 2011, Tan M 2014). Sirt5 has been proposed to regulate ammonia production and ammonia-induced autophagy and mitophagy by regulating glutamine metabolism (Polletta L 2015).

### Sirtuin 6 (Sirt6)

Sirtuin 6 is localized in the nucleus and is a chromatin associated histone deacetylase (Kugel S 2014). It can deacetylase histone H3 lysine 9 (H3K9) thus participating in regulation of telomeric chromatin and cellular senescence (Michishita E 2008). When it deacetylases histone H3 lysine 56 (H3K56) it decreases the chromatin accessibility for transcription factors such as NF-kB, Foxo3, and HIF1-alpha to their target promoters thereby inhibiting the expression of their target genes (Kugel S 2014). Sirt6 deacetylates histone H4K16 which regulates the meiotic apparatus in the oocyte (Han L 2015). Sirt6 has been linked to the regulation of life-span and health-span (Kanfi Y 2012, Cardus A 2013, Shen J 2013, Liu R 2014, Sharma A 2013). Activation of Sirt6 is postulated to reduce atherosclerotic vascular diseases. Sirt6 expression suppresses cellular senescence and NF-kB mediated inflammatory responses, like TNF-alpha, in the human knee which leads to osteoarthritis development (Wu Y 2015). Increasing Sirt6 activity has also been implicated as a therapy in idiopathic pulmonary fibrosis (IPF) (Minagawa S 2011).

### Sirtuin 7 (Sirt7)

Sirtuin 7 is localized in the nucleolus. Sirt7 has been functionally linked to transcriptional regulation. It positively controls ribosome production through direct interaction with the Poll machinery (Ford E 2006, Grob A 2009, Chen S 2013). Conversely Sirt7 negatively regulates transcription of genes outside rDNA repeats via histone H3K18 deacetylation (Barber MF 2012). Sirt7 targets acetylated lysine in the N-terminal tail of histone H3 (H3K18Ac). Sirt7 is downstream of Sirt1 and Sirt6 in the DNA damage signaling cascade. Sirt7 recruitment to DNA damage sites is dependent of PARP1 activity. There it can deacetylate H3K18Ac. H3K18Ac affects recruitment of damage response factor 53BP1 to double stranded breaks in DNA leading to their end joining and genome stability.

### Cyclic Adenosine Monophosphate (cAMP)

CAMP discovery as a second messenger led to a 1971 Nobel Prize. Calorie restriction increases cAMP. CAMP decreases with age. Higher levels of cAMP has now been correlated to longer life. CAMP performs a variety of metabolic-related hormone signaling processes. NAD+ contains an AMP moiety. CAMP interacts with the Sirtuin NAD+ binding pocket. This binding increases the hydrolysis of NAD+ into NAM and 2'-O-acetyl-ADP-ribose. Thus cAMP is a promoter of the enzymatic activity of Sirtuins (Wang Z 2015) acting as a reinforcement to the energy depletion signal of NAD+.

### Phosphorylation

Sirt1 can be phosphorylated at the highly conserved Serine 434 location which is in the Sirtuin catalytic site. Phosphorylation at S434 increases the Sirt1 deacetylase activity. Protein Kinase A (PKA) or a kinase downstream of PKA is thought to phosphorylate Sirt1. This phosphorylation regulation is thought to regulate the Sirtuin activity on a shorter time frame (5 to 15 minutes) than normal measures that increase NAD+ levels. The shorter time frame allows the cAMP/ PKA induction for short term fatty acid utilization (Gerhart-Hines Z 2011). In addition, Sirt1 transcriptional levels are regulated by the competition for promoter site binding by cyclic AMP response-element-binding protein (CREB) and carbohydrate response-element-binding protein (ChREBP). CREB itself can be phosphorylated which leads to its nuclear import leading to it being a better competitor for the promoter site on Sirt1 and Sirt1 transcription. This mRNA reaches a maximum in 12 to 18 hours and returns to basal levels at 24 hours (Noriega LG 2011) pointing to the desirability of not eating for 12 hours each day (Chaix A 2014).

The Sirt1 protein has several other phosphorylation sites on serine amino acid side chains. Ser27 is one of the sites that gets phosphorylated indirectly by JNK2 activation. When the Ser27 site on Sirt1 is phosphorylated, the Sirt1 protein becomes much more resistant to proteasome-mediated degradation. Thus it increases the half-life of the Sirt1 protein from less than 2 hours to greater than 9 hours. This is a very important part of maintaining Sirt1 protein levels within the cell (Ford J 2008).

Keap1 serves as a negative regulator of Nrf2, described later, which activates antioxidant enzymes. Keap1 degradation in response to antioxidants is controlled by tyrosine phosphorylation (Kasper JW 2011).

### Nicotinamide Mononucleotide (NMN) as an anti-aging compound

Only NAD+ activates all 7 Sirtuins. In 2008 it was demonstrated that NMN, a precursor of NAD+, produced age-reversal effects in mice (Ramsey K and Mills K 2008). Then in 2009 it was shown that NMN had a powerful effect on reversing the effects of obesity-induced diabetes (Imai S 2009) as well as age-induced diabetes. In 2013 it was shown that high dose NMN reversed muscle aging with one week of NMN administration (Gomes A 2013).

### CD 38

CD38 is a NADase as well as a NADPase. CD38 can be extracellular (a type II plasma membrane enzyme) and intracellular. CD38 transforms NAD+ into nicotinamide and cADPR. cADPR is a second messenger involved with cell function. Nicotinamide, as previously noted, feeds back to inhibit both the sirtuin enzymes as well as the PARP enzymes discussed in the next section. CD38 is found in many cell populations. CD38 is associated with the innate immune system as well as the adaptive immune system. CD38 is highly expressed in inflammatory cells and the loss of CD38 is associated with impaired immune responses. CD38 and CD157 are thought to enable energy recovery of energetically costly products which would otherwise be wasted. Two CD38 alleles are known in the Caucasiod population (Malavasi F 2007). CD157 is a second member of this family, although CD157's catalytic efficiency is several hundredfold lower than that of CD38 (Hussain AM 1998). CD38 and CD157 can be in monomeric or dimeric forms. CD38 is a master Ca⁺⁺ regulator that catalyzes the formation of endogenous Ca⁺⁺ (Lee S 2015). Ca⁺⁺ releases can stimulate the production of IL-6 (Adebanjo OA 1999, Sun L 2003). IL-6 was shown to be lowered in "Example" section herein.

NAD+ is known to decline with age. CD38's protein levels, mRNA levels and enzymatic activity all increase (in all tissues tested: liver, white adipose tissue, spleen, skeletal muscle, ilium, jejunum, and kidney) with increase age. This increase in CD38 is required for age related NAD decline. Other proteins that use NAD do not appear to be the cause of NAD+ decrease with age; examples include PARP1 and Sirt1 both of these decrease with age. An excellent inverse correlation coefficient was observed between CD38 activity and NAD+ decline in aging. CD38 is also able to degrade NAD+ precursor nicotinamide mononucleotide (NMN) (Grozio A 2013). The kcat for NMN+ was 5-fold higher than that of NAD+ and has the greatest reported kcat of any substrate for CD38 (Sauve AA 1998). When CD38 lowered NAD+ in cells, this led to the loss of mitochondrial function without changes in levels of Sirt3.

CD38 is induced by:
i. Oxidation is associated with CD38 activation (Zhang AY 2004, Kumasaka S 1999, Wilson HL 2001, Dipp M 2001,Okabe E 2000, Ge Y 2010)
   This is the opposite of Sirt1, where reduction is needed for Sirt1 to stay on, and oxidation turns it off. Oxidation also activates PARP-1 (Bai P 2011).
ii. TNF-alpha is a potent inducer of CD38 expression in cells (Barata H 2004, Lee CU 2012).
   Note: The triple therapy in "Example" herein demonstrates the reduction of both TNF-alpha and IL-6.
   a) CD38 has a TNF receptor (Prasad GS 1996);
   b) TNF-alpha also induces a two-fold activation of the CD38 promoter.
So TNF controls both the (transcriptional regulation) RNA levels and the protein activity. The mechanism of this regulation is that TNF-alpha increase binding to the NF-kB site and to some of the AP-1 binding sites (Tirumurugaan K 2008).

CD38 appears not to be effected by an end product feedback loop:
i. Nicotinamide rescues CD38 from inhibition of synthetic inhibitors (Sauve AA 2002, Sauve AA 1998). Nicotinamide inhibits Sirt1 and PARP (other users of NAD+). When Nicotinamide is methylated then this does not feedback to Sirtuins and PARPs and does not turn off these NAD+ using enzymes (due to steric hindrance).

CD38 is inhibited by:
ii. There are nicotinamide mononucleotide look-alike-molecules for example flavonoids luteolinidin, kuromanin, and luteolin (Kellenberger E 2011) these inhibit CD38, but these would probably also inhibit other reactions involving the three enzymes that make NAD+ from NMN as well as the other enzymes that use NAD+ like SIRT and PARP.
iii. Methylation of CD38's gene may be a part of its regulation (Ferrero E 1999). This in addition to the effect of methylation of nicotinamide to methyl-nicotinamide altering the feedback loop of the Sirtuin and PARP enzymes. This gene methylation (epigenetics) may well be why CD38 increase with age.
iv. Apigenin inhibits CD38. It also turns on Nrf2. Apigenin effectively reversed the hypermethylated status of the 15 CpG sites in the Nrf2 promoter in a dose-dependent manner. Apigenin enhanced the nuclear translocation of Nrf2 and increased the mRNA and protein expression of Nrf2 and Nrf2 downstream target gene NQ01. Apigenin restored NRF2 from the silenced status by CpG demethylation (Parededs-Gonzalez X 2014).
v. Reduction turns off CD38. Reduction of Cys 118-Cys 201 disulphide in CD38 leads to inactivation (Prasad GS 1996). A disulphide is involved with the bifunctional activity at hinge region of the enzyme and the three dimensional structure dependent on the 10 cysteine residues (Lin Q 2005, Prasad GS 1996). By contrast Sirt1 is kept on by reduction.
vi. There is a potential binding site upstream from the CD38 transcription start site for trans-interactions with IL-6 (Note, triple therapy in "Example" reduces IL-6).

### Poly (ADP-ribose) polymerase (PARPs)

DNA breakage repair takes a lot of energy and energy devoted to this is allocated. DNA breakage increases with age on average, in part, because energy is not allocated to its repair, even though the DNA damage can be repaired. Poly (ADP-ribose) polymerases (PARPs) are NAD+ dependent enzymes that repair DNA (an ancient and evolutionarily conserved biochemical reaction Otto H 2005) and are responsible for other biological functions as well. Nicotinamide is released as an end product of these PARP reactions. Thus not only do the Sirtuin enzymes compete for available NAD+ with the PARPs and CD38, but the Sirtuins are also inhibited by the end product of the use of NAD+, which is the nicotinamide from Sirtuin use of NAD+, the PARP use of NAD+, the CD38 use of NAD+ as well as other uses of NAD+. As stated before, NAD+ has been seen to decrease with aging (Braidy N 2011 and Massudi H 2012). There are 17 PARP enzymes in humans (Ame J 2004). PARP-1, 2, and 3 (and tankyrases) are all involved with DNA repair. Sirt1 and Sirt6 have been shown to be involved with DNA repair as well. The majority of DNA - induced PARP activity is covered by PARP-1 (85-90%) while PARP-2 is considered to be responsible for the rest (Szanto M 2012). PARP-1 is regulated by a) nicotinamide feedback, b) redox balance, H2O2 oxidation activates PARP-1, c) reversible methylation and d) PARP-1 is turned off by Sirt1 deacetylation. Prolonged PARP activation can deplete cellular NAD+ pools, thus the large drop in NAD+ with excess DNA damage. PARP-1 displays high catalytic turnover of NAD+ compared to Sirt1 (Bai P 2012). The Km (20-60 µM - Ame J 1999) is 5-fold lower and PARP-1 has a much stronger Vₘₐₓ than Sirtuin-1 (Houtkooper R 2010). The affinity of PARP-2 with NAD+ and its degradation is about the same as with Sirt1. PARP-2 binds to DNA in the proximal region of the Sirt1 promoter. The Kₘ of most Sirtuins for NAD+ are in the range of 100-300 µM and fluctuations of NAD+ are reported at 200 to 500 µM. NAD+ levels generally fluctuate within a two-fold range (Chen D 2008). In addition NAD+ fluctuates in a circadian fashion (Ramsey K 2009). Although these measurements have several shortcomings it does appear that the activity of Sirtuins can be rate limited by NAD+ availability.

### Free Radical Theory of Aging

In 1956, Denham Harmon, studying the effects of X-ray radiation, proposed that the cause of aging was due to reactive oxygen species called "free radicals," and today is known as the "Free Radical Theory of Aging" (Harman D 2009). From his observations of the effects of X-ray radiation on animals, Dr. Harmon proposed that just like in X-radiation induced free radical production, normal aging generated free radicals and had similar effects on the organism. At that time, the source of these "free radicals" with normal aging was unknown. Subsequent efforts have confirmed that cells produce their own reactive oxygen species (i.e., free radicals). In fact, free radicals are produced in every cell from before birth until death. Many cellular biochemical reactions create reactive oxygen species within the cell. Aging is not due to the presence of these free radicals, per se, but rather due to the damaging excess of free radicals, because of the lack of free radical scavenging by the many enzymes that quench these reactive oxygen species, cause to the cells. Control of Reactive Oxygen Species (ROS) is modified in muscles of old animals and release of ROS (superoxide) is reduced in old muscles. (Jackson M. 2011). This Free Radical field is now called the field of Redox Biology (Nathan C 2013) and there are now a growing number of reports detailing advantageous biological effects of free radicals involved in the modulation of cell signaling pathways (Powers and Jackson 2008). The "Redox Stress Hypothesis of Aging" conceptually shifts the importance of redox to signal transduction and gene regulation with a pro-oxidizing shift in the redox state of cells with age that leads to the over-oxidation of redox-sensitive protein thiols and the consequent disruption of the redoxregulated signaling mechanisms. Support for this theory comes from a) observations that oxidative byproducts increase from 25 to 100% from puberty to adulthood b) protein carbonyls increase with age and decrease with calorie restriction and c) average lifespan is proportional to protein carbonyls (Sohal R 2012).

### Oxidation-Sensitive Protein Thiol Groups

Changing the redox potential of oxidation-sensitive protein thiols can allow the switch between distinct catabolic and anabolic processes as well as activate survival pathways. Protein methionine and cysteine residues are particularly sensitive to oxidative modification. Methionine is the step prior to SAM synthesis in the methylation pathway. Thus methionine is connected to the methylation pathway and regulated by redox balance. The percentage of cysteine residues increases with organism complexity but their prevalence are still significantly lower than occurrence simply based on codon usage. Cysteines that occur in clusters are highly conserved in evolution and usually are structurally or functionally important. pKa values for the thiol groups are influenced by their local environment. Oxidation states can range from the fully reduced thiol / thiolate anion to the fully oxidized sulfonic acid (Cremers CM 2013). The reaction rate of protein thiols with oxidants such as hydrogen peroxide (H₂O₂) spans over 7 orders of magnitude without any detectable correlation to the acidity of the respective active site thiol (Ferrer-Sueta G 2011).

There are reversible and irreversible cysteine modifications. Oxidation of cysteine thiol (RSH/RS-) by ROS, RNS, or RCS leads to the formation of highly reactive sulfenic acid (RSOH), which can react either with another thiol to form a disulfide bond (RSSR) or with GSH to become S-glutathionylated (RSSG). These oxidative modifications are reversible, and reduction is catalyzed by the Trx and/or Grx system. Further oxidation of sulfenic acid to sulfinic acid (RSO₂H) and sulfonic acid (RSO₃H) is thought to be generally irreversible in vivo. Many of the thiol redox regulated proteins act as transcriptional regulators (e.g., OxyR, Yap1p) which rapidly induce expression of genes involved with antioxidant defenses (Zheng M 1998, Tachibana T 2009), others are involved with signal transduction cascades (Gopalakrishna R 2000 and Dinkova-Kostova AT 2005). (See Supplement 1 from Cremer CM 2013 for more examples.)

An example of an enzyme with thiols in the active site is GAPDH which plays a crucial role in glycolysis. Oxidation of the GAPDH thiols blocks glycolysis and contributes to the generation of NADPH instead of NADH (Shenton D 2003). Another example is the oxidation of thiols in active sites that inactivate the phosphatase activity of SHP1/2, PTEN, Cdc25 enhancing signaling intensity achieved by substrate phosphorylation, this leads to activation of signaling pathways like NF-kB-inducing kinase/IxB, causing expression of genes involved in antioxidant defense (Jung KJ 2009). A third example is the Sirtuin thiol groups in Sirtuin active sites that are very sensitive to oxidation, which inhibit Sirtuin activity when oxidized. Human Sirtuin-1 has 3 (Cys-67, Cys-268, and Cys-623) of 5 cysteines exposed to possible reversible thiol modification via redox balance (Autiero I 2008). Cys-67 and Cys-623 are consistent with post-translational regulation of these terminal regions, Cys-268 lies in the NAD+ binding region in all the members of the Sirtuin family of which the catalytic core is highly conserved. The binding of NAD+ results in changes in the Sirtuin conformation that allows catalysis to proceed (Zee R 2010).

### Redox Biology Main Components

There are different types of ROS and RNS (reactive nitrogen species). Together they are referred to as RONS. They include: superoxide, hydrogen peroxide, hydroxyl radicals, singlet oxygen, nitric oxide, peroxynitrite, hyperchlorite, and also lipid peroxidations "PUFA"s. There is different specificity of ROS. ROS display a type of specificity that is atomic rather than molecular. ROS most often reversibly reacts in cell signaling with Sulphur, which is one of the least abundant atoms in biological macromolecules and mostly with side chains of cysteine or methionine residues in peptides or proteins (Nathan C 2013). Endogenous enzymatic sources of ROS (multiple isoforms allow more sensitivity and specificity in regulation) include seven isoforms of NADPH oxidases (NOXs) that are differentially expressed (regulated) in diverse cells and species as well as a list of other sources (see Box 1 page 2 Nathan C 2013).

The main types of anti-oxidants enzymes (multiple isoforms allow more sensitivity and specificity in regulation) (need control and use of transition metals) are Superoxide Dismutase (SOD) 3 isoforms, Glutathione Peroxidase (GPX) 5 isoforms, and Catalase. There are also Thioredoxin (TRX) 2 isoforms (with thioredoxin reductase) Thioredoxin may be recycled by interaction with REF-1 (REF-1 keeps Sirtuin thiols reduced), Glutaredoxin (GRX) 3 isoforms, Peroxiredoxin (PRX) 6 isoforms (responsible for reduction of 90% of Eukaryote mitochondrial and more than that of cytosol HzOz. This can be turned on and off with a functional loop of regulation allowing redox signaling (Sies H. 2014). Peroxiredoxin makes up a phylogenetically ancient family of proteins whose primary role is detoxification of HzOz. These also create a redox rhythm. It is thought that catalytic cycle of peroxiredoxin hyperoxidation and recycling by sulfiredoxin may form the basis of a transcription-independent circadian clock (Rey, G. 2013). NAD+ levels are correlated to the biological clock with 2 peaks in the day 12 hours apart. It is thought that because NAD+/NADH with a cellular ratio of >1 is higher than NADP+/NADPH cellular ratio of <0.01 in the cytosol that this allows the cell to segregate antioxidant and biosynthetic reducing equivalents (NADPH) from those destined for mitochondrial ATP generation (NADH). The phosphate of NADPH confers different substrate specificity but has the same electron transfer properties. Hyperoxidation of peroxiredoxins can induce chaperone function as well as signal transduction.

Antioxidant small molecules include: Glutathione (GSH), Uric acid, Bilirubin, Ascorbic Acid (Vitamin C) Vitamin E, also carotenoids, Co-Enzyme Q10, N-acetylcyteine (NAC). NAC acts as a reduced thiol donor and counters H₂O₂ that oxidizes thiols.

### Methylation Theory of Aging

Not all genes are expressed in all cells. This "selective gene expression" control of the 21,800 genes that code for proteins in human cells determines if the cell becomes a brain cell or a heart cell. This system of gene regulation is referred to as "epigenetics" (Kundaje A 2015). Epigenetics controls the rate of aging (Reynolds L 2014). One of the methods by which genes are regulated is the methylation of certain DNA residues called "cytosines." In 1967, Boris Vanyushin showed that DNA loses its methylation with aging (Vanyushin BF 2005). There are other epigenetic mechanisms involved besides DNA methylation, such as histone protein modifications, microRNA, and chromatin remodeling (heterochromatin vs euchromatin) (Kundaje A 2015). In addition, some DNA cytosines increase their methylation with aging and other sites decrease their methylation with aging. It is clear is that DNA methylation is the form of epigenetic gene regulation that correlates with aging. This has most recently been shown by Steven Horvath, who showed that a "DNA methylation clock" can be constructed from the analysis of only 353 cytosine residues and that this "DNAm clock" (Bocklandt S 2014) has a 0.96 correlation with aging. More importantly, this "clock" keeps time much better than any other known measure of aging besides birth date. DNA methylation profiling of mesenchymal stem cells (MSCs) obtained from individuals aged from 2 to 92 years identified 18,735 hypermethylated and 45,407 hypomethylated CpG sites associated with aging. Most importantly, hypomethylated CpG sites were strongly enriched in the active chromatin mark H3K4me1 in stem and differentiated cells, suggesting this is a cell type-independent chromatin signature of DNA hypomethylation during aging. These results indicated that the dynamics of DNA methylation during aging depends on a complex mixture of factors that include the DNA sequence, cell type, and chromatin context involved and that, depending on the locus, the changes can be modulated by genetic and/or external factors (Fernandez AF 2015). It has been shown that calorie restriction prevents the age-related changes in DNA methylation in mice (Chouliaras L 2012). Two of the 7 Sirtuin enzymes have been shown to indirectly affect DNA methylation through their effects on histone deacetylation (Sirt1 and Sirt6). It was also shown that the end-product of the Sirtuin reaction, nicotinamide, needs to be methylated to 1-methylnicotinamide, otherwise the end product nicotinamide will bind inside the Sirtuin enzyme and stop its enzymatic activity (Schmeisser K 2013). Sirtuin-1 decreases the activity of NF-kB which increases tri-methylation of lysine 36 on histone 3 (H3K36me3). This correlates to accelerated DNA methylation. Genes with a dramatic expression change during aging are marked with low or even undetectable levels of H3K36me3 in their gene bodies irrespective of their corresponding mRNA abundance (Pu M 2015). In human cells a global loss of tri-methylation of H3K9 (H3K9me3) recapitulates accelerated cellular senescence and changes in heterochromatin architecture. These findings also correlated to people ages 7 to 72 heterochromatin's disorganization with increasing age (Zhang W 2015). In January 30, 2015 DNA-methylation-age of blood was used to predict all-cause mortality in later age of humans independently of health status, lifestyle factors and known genetic factors (Marioni RE 2015). On February 19, 2015 the journal Nature published the results of 111 human epigenomes allowing future comparisons and references to be made by others (Kundaje A 2015).

### Methylation Pathway

Methionine is particularly sensitive to oxidative modification. Methionine is the step after homocysteine synthesis and prior to SAM synthesis in the methylation pathway. Thus methionine is a part of the methylation pathway and is regulated by redox balance. Cysteine is synthesized from methionine and is the main precursor of hydrogen sulfide (H₂S). Elevated homocysteine levels are associated with inhibition of endogenous hydrogen sulfide (H₂S) generation (Tang X 2011). Hydrogen sulfide (H₂S) ameliorates methionine induced oxidative stress (Tyagi N 2009). Homocysteine (Hcy) can be irreversibly degraded to hydrogen sulfide (H₂S) by a transsulfuration pathway which is activated by oxidative stress. H₂S has protective functions in hyperhomocysteinemia (Ohashi, R. 2006, Chang L 2008). Adipose tissue is an important organ of methionine metabolism and is also an insulinsensitive organ. Increases of H₂S in adipose tissue increases insulin sensitivity (Feng X 2009). High pancreatic H₂S suppresses insulin release (Wu L 2009). Blood levels of H₂S are lower in type 2 diabetes than age matched healthy subjects (Jain S 2010). Aspirin is an arachidonate inhibitor and may influence the methionine-homocysteine cycle and associated one carbon metabolism and thereby both methylation and redox balance (Lupoli R 2015). H₂S therapy with H₂S donors; Na₂S or NaSH inhibits aspirin in a dose dependent manner (Zanardo RC 2006).

There is also a methylation inhibitor: S-adenosylhomocysteine (SAH). When methionine is abundant, NNMT regulates only SAH not SAM (Ulanovskaya OA 2013).

Radical SAM enzymes are a diverse superfamily of proteins that use radical chemistry (5'-dAdo) to effect substrate modification. Substrates of these enzymes are distinct from the nucleophilic substrates that undergo methylation by a polar mechanism. There are 4 known subclasses of these enzymes (A, B, C, D).

As a general rule age-related hypo-methylation of DNA is the dominant event leading to increased expression of genes, but hyper-methylation is common in some promoter regions of DNA with age leading to promoter repression. There is a close relationship between redox balance and methylation balance (Metes-Kosik N 2012).

There is a relationship of methylation to redox balance with homocysteine going to the antioxidant glutathione when under oxidative conditions and going to SAM and methylation under reduction conditions (Mosharov E 2000).

### Somatic Mutation Theory of Aging

Somatic cells are cells that, when cloned, can grow to a full animal that ages normally. Somatic cells in sexual organisms live to support germline cells' attempt to have their DNA reach the next generation. Somatic cells are known to give up their own cellular lives for the good of the organism. One way they do this is via programmed cell death also called apoptosis where the somatic cell dies in a way that is organized and less harmful than necrosis cell death to its cellular neighbors. Sirt1 inhibits apoptosis. Sirt2 is indicated to be involved in the regulation of necroptosis, a somewhat more organized version of necrosis (Narayan N 2012). Some viruses like vaccinia have anti-apoptosis genes so other methods of cell death are needed. Another way is the culling of somatic cells that are not as vital as their neighboring cells. In this specific selection, cells with higher anabolic capacity and higher relative c-Myc than their neighboring cells are selected for and relatively unfit cells with lower c-Myc than their neighboring cells are eliminated (Merino M 2015). Sirt1 in humans regulates c-Myc and thus this process, as well as apoptosis. Of note is that c-Myc concentration change is opposite in direction to IL-6 level changes (Hoffman-Liebermann B 1991). Note: the "Example" herein lowers IL-6 concentrations in serum. Results from the reduction in expression of myc in mice has led to its suggestion as an anti-aging therapy (Alic N 2015).

### Autophagy

Autophagy generates amino acids, sugars, fatty acids, and nucleosides that are recycled for macromolecular synthesis and energy production which is important during starvation and stress for cell survival. NAD+ is intimately correlated to autophagy and NAD+ and its metabolism can influence autophagy. The mechanism of control of autophagy by NAD+ include pathways involving: a) NAD+/ NADH b) NADPH, c) PARylation, d) Deacetylation, e) NAADP and f) cADPR/ADPR. NAD+ dependent deacetylation by Sirt1 regulates multiple autophagy processes. NAD+ metabolites catalyzed by CD38 are also involved in multiple autophagy processes. Sirt1 regulates autophagy via p53 which has a pivotal role in sensing cellular stress, including DNA damage and oxidative stress. A link between p53 and necrosis has also been reported (Tu H 2009). Autophagy is a process of self-degradation of cellular components in which doublemembrane autophagosomes sequester organelles or portions of cytosol and fuse with lysosomes or vacuoles for breakdown by resident hydrolases. Deacetylation modification of the autophagy machinery proteins is also required for autophagy and the deacetylation process is dependent on the NAD-dependent deacetylase Sirt1 (He C 2009).

### Connections between various theories of aging

The various theories of aging, discussed herein have connections between them. For example, the Calorie Restriction/Sirtuin Theory of aging is connected to the Methylation Theory of Aging by the methylation of nicotinamide. Nicotinamide is produced from the use of NAD+ by Sirtuins, and by methylating nicotinamide, methylated-nicotinamide cannot inhibit the Sirtuins in a negative feedback loop. PARPs and CD-38 also use NAD+ and produce nicotinamide as an end product of their reactions, which inhibits PARP and Sirtuin activity through a negative feedback loop. Thus, methylation of nicotinamide can prevent the negative feedback loops of Sirtuin and PARP enzymes.

In addition, the Calorie Restriction/Sirtuin Theory of aging is connected to the Free Radical (now Redox) theory of aging by Sirtuins inhibition of NF-κB, a major component of inflammation and immune defense. This process increases tri-methylation of DNA which increases DNA wrapping which decrease all-cause mortality. The thiol groups at the Sirtuin active site have to be reduced for the Sirtuin to be active directly connecting the two theories.

Furthermore, the Calorie Restriction/Sirtuin Theory of aging also connects to the Somatic Mutation Theory of Aging. If cellular damage is not repaired, it accumulates, affects the fitness of cells, and if the cellular performance falls below a critical level the individual dies. This is called the Somatic Mutation theory of aging (Kennedy S 2012 and Szilard L 1959). Sirt1 as well as other Sirtuins effect the expression of Myc. Mammals are able to specifically select for cells with high anabolic capacity and elimination relatively unfit cells from their relative Myc activity (Mareno E 2014). This ability to select for more fit cells and eliminate unfit cells prolongs lifespan of flies 35% over calorie restriction (Merino M 2015).

The Methylation Theory of aging is connected to the Free Radical (now Redox) theory of aging as seen when homocysteine progresses in the synthetic pathway to S-Adenosyl-methionine (SAM), which is needed to make 1-methylnicotinamide in the example above, under the reducing effect of the antioxidant defense system but is diverted to the synthesis of glutathione, an antioxidant, when under oxidative stress.

The other theories of aging feed into the above theories as discussed in the text. The previously discussed "Rate of Living Theory of Aging" (Pearl R 1928, Rubner A 1908, Sohal R 2012) and the previously discussed "Disposable Soma Theory of Aging" (Kirkwood and Rose 1991) both feed into the "Calorie Restriction" and "Sirtuin use of NAD+ discussions. The Redox Stress Hypothesis of Aging (Sohal R 2012) feeds into the Free Radical Theory discussion as noted in that section. It has also been noted that Redox balance itself is related to inflammation. The results demonstrated in the "Example" herein where IL- 6 and TNF- alpha are decreased in plasma then correlate the "Inflammaging Theory of Aging" (Franceschi C 2007, 2007, 2014) also called Para-inflammation (Medzhitov R 2008) and the "Metchnikoff's Hypothesis of Aging" which concerns the intestinal lining permeability to bacteria and their products leading to aging (Metchnikoff E 1901) and this permeability to bacteria can be reduced by fasting via a pathway involving Crtc a molecule that interacts with CREB, which is linked to Sirt1 pathways, discussed here which are activated by fasting. It appears there is a link between the immune system's attack on bacteria to its attack on mitochondria proteins such as cardiolipin which is need for N1rp3 inflammasome activation (Iyer SS 2013).

### Cellular damage is causal in aging and aging is causal in the "Diseases of Aging"

In addition to the aging itself there are diseases of aging (Goldman DP 2013). In these diseases of aging, aging is a causative factor in the disease. Diseases of aging include: inflammation, heart disease (heart attack and heart failure), stroke, neurodegenerative disease such as Alzheimer's disease, diabetes, cancer, respiratory disease, systemic autoimmune disease, and muscle wasting.

What are needed are new compositions and approaches for addressing the effects of aging. The compositions and compositions for use disclosed herein address this need.

### SUMMARY

In one aspect, there is provided a nutritional composition according to claim 1.

In a further aspect, there is provided a tablet comprising a composition according to claim 13.

In further aspects, there is provided a composition for use in reducing inflammatation in a subject according to claim 14.

In another aspect, there is provided a product for use in reducing inflammation in a subject according to claim 21.

### DETAILED DESCRIPTION

The study of caloric restriction led to the discovery of Sirtuins, which are activated by the "depleted energy" version of NADH, which is called NAD+. NADH is not used by sirtuins enzymes and is only inhibitory at concentrations far greater that those predicted for cells. NADH is also not used for generation of NADP+ by the cytosolic NADK enzyme and this generated NADP+ is rapidly turned into NADPH (Pollak N 2007). Caloric restriction induces a "nutritional stress" that results in a depletion of the cells energy stores (ATP, NADH, etc.). The "depleted energy forms" of this stored energy are cAMP and NAD+.

NAD+ activates a set of enzymes called Sirtuins as well as PARPs. What the data disclosed herein shows is that by providing NAD+ or compounds or compositions having a similar activity, immune system markers are reduced, which has been shown to be associated with anti-aging. These data are consistent with an increased activation of Sirtuins, through interaction with NAD+, or similar acting molecules. However, also disclosed herein, the positive effect of NAD+ can level off, presumably because of other reactions taking place in the organism, including in the active site of the Sirtuins themselves.

Therefore, what has been additionally shown by the disclosed compositions for use and compositions is that by adding additional molecules along with NAD+ or similar acting molecules, the beneficial effects can be extended by, for example, a continued, enhanced, and maintained reduction in inflammation markers, which has been linked to anti-aging. This information has led to compositions and formulations, which contain three categories of compositions, or compositions/products for use where three different categories of molecules are administered, alone, in conjunction, or in combination to a subject.

Increasing life-span and health-span by repairing cellular damage and preventing the age-related changes that can occur are disclosed. The data provided herein show that to reduce markers for inflammation three broad goals to defend against and repair deterioration from aging should be sought:
I. NAD+ should be available to turn on and be used by Sirtuins,
II. methyl donors should be available to methylate DNA and other entities needing methylation like the reaction of nicotinamide to 1-methylnicotinamide by the nicotinamide-N-methyltransferase (NNMT) enzyme, and
III. a reducing balance should be provided so that important enzymes, such as Sirtuins, can have the thiol (sulfur) groups in their reactive sites maintained in a reduced state.

Disclosed herein are compositions, formulations, and compositions/products for use that reduce markers of inflammation related to aging, and are consistent with enhancing these three goals.

Meeting these three goals is possible if oxidation, in the form pulsed low level HzOz, is available to turn on pre-conditioning of the anti-oxidant defense and repair system. By turning this system on, the system is protected against the down regulation of the antioxidant defense and repair system, which is an energy saving mechanism. In this way, when the antioxidant defense system is challenged with an oxidative assault from a larger oxidative burst, it is able to defend against this oxidation that would lead to cell damage and destruction.

In one embodiment, one provides enough oxidation from H₂O₂ to provide preconditioning from signaling to turn on the anti-oxidant defense and repair system but not enough to create oxidized damage like oxidizing the thiol groups in the Sirtuin active site that turns the Sirtuin enzymes activities off. The APE-1/ Ref-1 is a molecule that protects the thiol groups of amino acids in the Sirtuin active site from oxidation by H₂O₂. This can be kept active. It is theorize that the same or a similar process is needed for the nicotinamide-N-methyltransferase (NNMT) enzyme to make 1-methylnicotinamide from nicotinamide and thus to stop this feedback loop from shutting off the Sirtuin enzyme by cutting off the supply of nicotinamide that can fit into the Sirtuin enzyme and stop it's activity.

Disclosed is a usable solution for reversal of human aging by resetting the human endogenous defense and repair pathways and mechanisms. These mechanisms are normally set to preserve energy due to molecular settings set by and for evolutionary energy insufficiency, evolutionary sexual selection, and pathogen defense by diverting more usable energy and resources from defense and repair mechanisms. Through administration of the disclosed compounds, compositions, and formulations these pathways can be reset for increased repair and defense.

It is demonstrated herein that dietary NMN drunk by itself in water does turn into NAD+ and turns on Sirtuins in humans, but these effects are ephemeral. It is also demonstrated herein that Hormesis / feedback loops effected benefits in humans until these benefits are plateaued or reversed and even overshoot the initial beneficial effects within a three month time frame. This discovery solves this deterioration of beneficial effect problem by turning on the beneficial effects of Sirtuin enzymes, optimizing their beneficial effects, and keeping these beneficial effects turned on.

Disclosed herein are compounds, compositions, formulations, and compositions/products for use, which turn on, enhance, and in some formulations keep on, the human defense and repair mechanisms involving the Sirtuin enzymes. These compounds, compositions and formulations comprise one or more items from each of three (3) categories alone or in combination, and can be administered through ingestion, injection, inhalation, application to the skin, or any other means.

When administered, the disclosed compounds, compositions, and formulations, can perform at least one of the following activities:
A) Protect against further cellular damage from the aging process
B) Repair cellular damage from the aging process
C) Delay the onset of the diseases of aging where aging is a causal factor.

Diseases of aging include: inflammation, heart disease (including heart attack and heart failure), stroke, neurodegenerative disease such as Alzheimer's disease, diabetes, cancer, respiratory disease, systemic autoimmune disease (including arthritis) and muscle wasting.
D) Promote weight loss / reduce hunger
E) Promote more productive sleep, waking more rested

### Compounds, Compositions, and Formulations

The compositions comprise at least one compound from within each of the following three general categories:
Category 1 which are Repair System Activators
Category 2 which are Methyl Donors, and
Category 3 which are Antioxidant Defense Activators

The first compound (repair system activator) is chosen from nicotinamide adenine dinucleotide (NAD+), nicotinamide mononucleotide (NMN), nicotinamide riboside (NR), nicotinic acid riboside (NAR), nicotinic acid adenine mononucleotide (NaMN), nicotinic acid adenine dinucleotide (NaAD), 1-methylnicotinamide (MNM), cyclic adenosine monophosphate (cAMP), and any combination thereof. The second compound (methyl donor) is chosen from S-5'-adenosyl-L-methionine (SAM), methionine, betaine, choline, folate, vitamin B12, and any combination thereof. The third compound (antioxidant defence activator) is chosen from HzOz, hydrogen sulfide (H₂S), sodium hydrosulfide (NaHS), sodium sulfide (Na₂S), metformin, curcumin, sulforaphane, quercetin, apigenin, pterostilbene, resveratrol, zinc, and any combination thereof, and optionally, water.

In one embodiment the first compound comprises NMN. In one embodiment the composition lowers a Surrogate Marker for Aging. In one embodiment the surrogate marker is CMV IgG, C-Reactive Protein, Tumor Necrosis Factor-alpha, or Interleukin-6 Serum. In one embodiment the composition comprises water. In one embodiment the composition is formulated for injection. In one embodiment the composition is in concentrate form for dissolving in a liquid. In one embodiment the composition is in tablet form or aerosol. In one embodiment the composition comprises at least 1 × 10⁻⁸ moles of the first compound, at least 1 × 10⁻⁸ moles of the second compound, and at least 1 × 10⁻⁹ moles of the third compound.

### Category 1, Repair System Activators

The turning on and maintaining of Sirtuin activity provides the beneficial effects disclosed herein. Sirtuins require NAD+. Providing a repair system activator can turn on the Sirtuins. The repair system activator is chosen from NAD+, NAD+ precursors NMN, NR, NaMN, NaAD, NAR, analogs of NAD+ that promotes NAD+ use MNM, and cAMP, or any combination thereof. A preferred repair system activator is the NAD+ precursor NMN (to make NAD+, to turn on, and be consumed by Sirtuins, which provides the benefit from Calorie Restriction). In humans, NAD+ typically naturally peaks in the morning and the evening such as at 8 AM and 8 PM, and thus the addition of NAD+ or precursor that would turn into NAD+ would be added, for example, preferentially in the 7 AM to 8 AM and the 7 PM to 8 PM time frame. In certain aspect, preferably one wants the two daily doses 12 hours apart so as not to disrupt the natural cycle of the biological clock. Typical formulations provide greater or equal to 1.19 × 10⁻⁴ moles/kg-of-subject NMN, NAD+, or NAD+ precursor when administered (NMN is 334.22 grams / mole).

One can also administer, typically through injection, NAD+ or use nicotinamide riboside (NR) which can be made into NMN in some cells of the body. Typically administering of NAD+ and NR are less preferred because NAD+ is not absorbed well through the digestive system and the enzymes that make NMN from NR are not found in every cell of the body. Orally delivered NR has also been shown to largely not reach muscle.

In a specific aspect, disclosed are compositions as described herein for use in reducing inflammation in a subject comprising administration of NMN (nicotinamide mononucleotide) to humans in preferred dosages of approximately 0.08 grams / kg total body weight per day divided into two equal doses taken approximately 12 hours apart. In certain embodiments, the dosage can be adjusted for absorption. It is preferred to administer the Repair System Activator such as NMN, through water and drinking.

Compounds and compositions that will activate production of NMN are disclosed. For example, Wang et al., discuss the P7C3 class of aminopropyl carbazole chemicals, compounds, and compositions which act by increasing NAD levels through its NAMPT-mediated salvage. (Wang et al. 2014)

### Category 2, Methyl Donors

When adding a methyl donor for methylation, adding betaine is preferred. Betaine can bypass the need (with the use of choline) for extra NAD+ if used to make S-5'adenosyl-L-methionine (SAM). SAM can provide the methyl group for nicotinamide, which has aging properties by stopping Sirtuin enzymes from working. This methylation of nicotinamide occurs via N-methyltransferase (NNMT) N-methylation to 1-methylnicotinamide. This nicotinamide with a methyl group attached provides competition to the available nicotinamide molecules that can get into the Sirtuin enzyme and decrease the Sirtuin enzyme's reactive ability; thus, preventing this process from happening in proportion to the concentration of each of the two competitors. Typically the timing for giving would be with the Repair System Activator, such as NAD+ or NAD+ precursor.

SAM also provides the methyl groups to reduce the hypo-methylation seen in aging and in the right context it can to be used beneficially to combat aging, example: the need for H3K4me3 methylation (Ulanovskaya OA 2013) of DNA found especially in older people.

Methyl Donors in addition to betaine, which can be used are SAM, methionine, choline, folate, and B 12. Typically these alternatives are less preferred because only about 2% of SAM get into the body when ingested (McMillan JM 2005); choline needs extra NAD+ to be made into betaine, which is in short supply in the body.

Dosages of betaine (trimethyl glycine) can be at least 0.03 grams/kg (3 × 10⁻⁴ mole/kg) of total body weight of the subject (calculated by 0.08 grams (from above NMN calculation) times 0.35 (for molecular weight ratio of betaine / NMN) = 0.03 grams / kg total body weight). This dose can be given over 24 hours, and can be divided into two approximately equal doses taken approximately 12 hours apart. The dose can be dissolved in water and drunk by the subject. The administration can be along with the administration of the category 1 compound or composition.

### Category-3 Antioxidant Defense Activators

When providing a category 3 compound, composition, or formulation the antioxidant defense is turned on. Having the antioxidant defense enzymes working increases the reduction of the thiol (sulfur) groups in the reactive site of Sirtuin enzymes and others with similar regulation. This prevents the Sirtuin enzymes from turning off due to thiol oxidation.

### Hydrogen peroxide (HzOz)

One way to create a generally reducing environment is to "shock" the organism by a pulsed burst of oxidants, such as HzOz. To keep the antioxidant enzymes being made and keeping them working one uses pre-conditioning with oxidants to shock on the system, and one keeps them on by additional timed shock pulses of oxidants prior to the antioxidant enzymes turning off due to their feedback loops that turn them off or down when they are not challenged by oxidants. In doing the pulse of oxidants for the preconditioning one uses a sufficient level of oxidants to turn on and keep on the antioxidant enzymes. The preferred choice for an oxidant to do the preconditioning is hydrogen peroxide (H₂O₂) due to its centrality in the redox signaling pathways and its relative stability for an oxidant and its low level of potential harmful effects compared to other oxidants that the cell deals with in its life cycle. H₂O₂ can oxidize thiol groups on proteins / enzymes thereby changing their enzymatic properties.

This pre-conditioning low level oxidation by H₂O₂ can be given in a pulsed, time controlled, and dose controlled fashion to turn on enzymes and processes without providing oxidation in excess of what is needed to turn on enzymes including anti-oxidant defense and repair systems enzymes, because excess oxidation causes cellular damage and harm. Any small molecule (non-enzyme) anti-oxidants should be taken at other time periods (other than the time period of the oxidative pulse) so as not to diminish this temporal effect of the oxidative pulse.

### Hydrogen peroxide (H₂O₂) oxidation and redox signaling

Hydrogen peroxide (H₂O₂) is a ubiquitous oxidant present in all aerobic organisms (Marino HS 2014). H₂O₂ is now appreciated as a messenger molecule and it provides sensitivity to redox signaling. H₂O₂ provides oxidative modification of amino acid side chains in proteins; in decreasing order of reactivity and biological reversibility, cysteine, methionine, proline, histidine and tryptophan. Thiol modification is key in H₂O₂ sensing and perception in proteins. Hydrogen peroxide has been found to mimic insulin activity, elicit arterial pulmonary relaxation, stimulate cell proliferation, and activate NF-κB and AP-1. The functional consequences of H₂O₂ signaling concern fundamental biological processes. With recognition of the role of low level oxidants stimuli for altering the set point of gene expression for batteries of enzymes, known as Hormesis (Helmut Sies 2014). Transcriptional factors effected by H₂O₂ include: AP-1, Nrf2, CREB, HSF1, HIF-1, TPSS, NF-κB, NOTCH, SP1, and SCREB-1 most involved in regulation of cell damage response, cell proliferation (cell cycle regulation) differentiation and apoptosis (Albrecht SC 2011).

Protein acetylation is regulated by H₂O₂ (Jung S-B 2013). Protein deacetylation is regulated by Sirtuins (Imai, S. 2000). H₂O₂ increase acetylation and Sirtuins decrease acetylation, so H₂O₂ and Sirtuins effects are in pushing acetylation reaction pathways in the opposite directions. Sirt1 is very sensitive to H₂O₂ inhibition of 1 µmol of extracellular H₂O₂ (Jung S-B 2013). Sirt1 is protected by thiol oxidation from (APE1 / Ref-1). It governs the redox state and activity of Sirt1. It reduces the thiol groups in the active site of Sirt1, H₂O₂ oxidizes the thiols in Sirt1's active site. Sirt1 is also regulated by redox-dependent phosphorylation (Caito, S. 2010).

### Need for pulsing of signaling oxidants

Low levels of H₂O₂ increase defenses by preconditioning and thus can ultimately protect against increase of oxidized thiols in Sirtuin's active site and Sirt1's decrease in activity by an oxidative challenge. Adaptation to H₂O₂ decrease H₂O₂ permeability of plasma membranes. Different cell membranes have a full range of permeability to HzOz. Aquaporins also regulate H₂O₂ transport across bio-membranes (Marinho HS 2014).

### Common drugs that change H₂O₂ levels

Metformin, the most widely prescribed antidiabetic drug in the world, increases hydrogen peroxide (H₂O₂); this upregulates peroxiredoxin-2 (PRDX-2). Metformin increases lifespan in *C*. *elegans* and taking away the PRDX-2 gene takes away this effect. PRDX-2 appears to have the role of translating oxidative stress into a downstream prolongevity signal. Treatment with N-acetylcysteine (NAC) and butylated hydroxyanisole (BHA), which are small molecule anti-oxidants, abolished the positive effect of metformin on lifespan (De Haes W 2014). Pharmaceuticals that increase hydrogen peroxide in the body can also be used for this category either in addition to H₂O₂ or as a substitute for adding hydrogen peroxide itself. Pharmaceuticals that increase H₂O₂ in the body include metformin (De Haes W 2014) and acetaminophen (Hinson J 2010).

Pharmaceuticals that increase H₂O₂ in the body need also to be included in the calculation of the oxidative pulse given in category #3. An example is Acetaminophen (the ingredient in Tylenol), which is a pharmaceutical that is known to increase H₂O₂ in the body (Hinson J 2010). N-acetyl-l-cysteine (NAC) is a compound that is known to counter many effects of H₂O₂ in the body.

### Timing, duration, and levels of H₂O₂

Enough oxidation to provide pre-conditioning to signal to the turn on the antioxidant defense and repair systems is desired; but not enough to create oxidized damage like oxidizing the thiol groups in the Sirtuin active site that turns the enzymes activities off. This level has been referred to as the "Goldilocks zone". The APE-1/ Ref-1 is a molecule that protects the thiol groups of the Sirtuin enzymes, which should remain active. The same or similar process for the nicotinamide-N-methyltransferase (NNMT) enzyme is theorized.

In certain embodiments, one can add pulsed low levels of hydrogen peroxide (H₂O₂) transiently to humans to pre-condition the anti-oxidant defense and repair systems to turn on and stay on. In certain preferred embodiments, approximately 100 µM concentration of food grade (commercial grade has acetanilide in it as a stabilizer) H₂O₂ in the 400 to 500 mL of water per individual dose is preferred, which can be taken alone or with Category 1 and Category 2 compounds or compositions. 1 mole of H₂O₂ = 1+1+16+16 = approximately 34 grams. 50% of H₂O₂ is estimated to be absorbed by the gut so a more preferred concentration to take is approximately 200 µM (in the 500 mL). For example, in certain embodiments, one drop of H₂O₂ is 0.05 mL. Food grade H₂O₂ comes in 35% concentrations. Taking 2 drops of 35% H₂O₂ in 500 mL distilled water (with each dose / day), gives approximately 200 µM. H₂O₂ degrades at about 10% / year if no light and no contaminants in deionized / distilled H₂O. H₂O₂ freezes at -11 °C. So in certain embodiments, taking 4 drops / day or 0.2 mL of 35% H₂O₂ / day in 1 liter of water. 35 grams / 100 mL = 0.07 grams / 0.2 mL. In certain embodiments, a quantity of approximately 0.0008 grams of H₂O₂ /kg total body weight dosages can be used.

In one embodiment there is disclosed a composition for use according to any of the aspects herein wherein H₂O₂ is ingested by dissolving H₂O₂ in deionized / distilled water and drinking. A preferred timing of dosage concentration, time taken and length of time taking is to use the same timing as #1 and #2 when in water. In certain embodiments, if H₂O₂ is partially enhanced from endurance exercise do exercise directly before or after.

Administration of metformin (De Haes W 2014), can come in liquid form, Riomet, as well as tablets. In liquid form 5 mL is equal to a 500 mg tablet. It reaches peak plasma concentrations in 1 to 3 hours in immediate release form and a steady state in one to two days. It is typically 50 to 60% bioavailable under fasting conditions. One would need to use this data to time and dose appropriately with Metformin.

### Hydrogen Sulfide (H₂S)

Another way to change the redox potential of oxidation-sensitive protein thiols besides using hydrogen peroxide (H₂O₂) to pre-condition the antioxidant defense system as discussed previously, is by directly augmenting the antioxidant defense system with hydrogen sulfide (H₂S). NaSH (a H₂S donor) (0.025-0.1 millimolar /liter) treatment dose dependently countered H₂O₂ treatment. Plasma H₂S levels decrease in humans over 50 to 80 years of age (Chen Y 2005) and plasma levels of H₂S in patients with cardiovascular disease (CHD) show a significant inverse correlation with severity of CHD and changes in the coronary artery (Jiang H 2005). NaSH decreases ROS and enhances SOD, GPx and GST expression. Lipid and protein oxidation products decrease significantly in plasma samples of healthy volunteers with H₂S rich water (500 mL / day for 2 weeks) (Benedetti S 2009). A 0.1 mM NaSH / Liter can increase Sirt1 in a time dependent manner (Wu D 2015). Exogenous H₂S has a protective effect on maintaining the circadian rhythm of clock genes by changing the NAD+/NADH ratio and enhancing the Sirt1 protein (Shang Z 2012). H₂S is also an important endogenous inhibitor of key elements of acute inflammatory reactions (Zanardo R 2006) by down regulating NF-kB or upregulating heme oxygenase 1 expression (Jin H 2008, Kim K 2008, Oh G 2006, Pan L 2011). H₂S can activate ATP-sensitive, intermediate-conductance and small-conductance potassium channels through cysteine S-sulfhydration (Mustafa A 2011, Yang G 2008) causing endothelial and smooth muscle cell hyperpolarization which intern causes vasorelaxation of vascular endothelium and lowering of blood pressure. H₂S has a direct inhibitory effect on angiotensin-converting enzyme (ACE) activity (Laggner H 2007). NaSH increases the expression of eNOS and PGC-1 Alpha (Wu D 2015), which both play a role in mitochondria biogenesis and function (Wu, CC 2013, Lagouge M 2006). H₂S upregulates the MAPK pathway (Barr LA 2014, Papapetropoulos A 2009, Yong QC 2008). It has been inferred that calorie restriction may help maintain H₂S signaling (Predmore B 2010). GYY4237 a slow releasing H₂S donor can kill seven different human cancer cell lines in a concentration-dependent manner (Lee Z 2011). Sulforaphane, also a H₂S donor, has dose-dependent antiprostate cancer (PC-3) properties (Pei Y 2011).

H₂S is a gasotransmitter. Gasotransmitters are endogenously produced at low levels and are able to freely diffuse through cell membranes to invoke cellular signaling (Calvert JW 2010). The three gasotransmitters are nitric oxide (NO), carbon monoxide (CO), and hydrogen sulfide (H₂S).

Hydrogen sulfide is synthesized from L-cysteine. Cystathionine gama-lyase (CSE), cystathionine beta-synthase (CBS), cysteine aminotransferase (CAT), and 3-mercaptopyruvate sulfurtransferase (MST) are endogenous enzymatic sources of hydrogen sulfide (H₂S). Liver production of H₂S to different extents has been shown by these enzymes and showed H₂S regulates lipid peroxidation and antioxidant enzyme (GPx, T-SOD, Cu/Zn-SOD, and Mn-SOD) activities in the liver, by administration of H₂S donor NaSH to the mice by injection of 0.05 mM of NaSH / kg body weight /day dissolved in 10 mL / kg body weight saline (Wu D 2015). Mitochondria are able to use H₂S under hypoxia and stress conditions to produce ATP (Fu M 2012).

Initial reports of H₂S's antioxidant ability were that H₂S can scavenge superoxide (Geng B 2004) and H₂S can upregulate glutathione (Kimura Y 2004). Later came more detailed reports of its activation of antioxidant enzymes. H₂S has been shown to activate Nuclear factor-erythroid 2-related factor 2 (Nfr2) (Peake BF 2013), which turns on antioxidant genes. Daily administration of Na₂S for 7 days increased Nrf2 expression in both cytosolic and nuclear fractions (Calvert JW 2010). Nrf2, which up regulates expression of antioxidant response element-regulated genes, is upregulated by H₂S (Islam KN 2015). H₂S activation causes Nrf2 to separate itself from its adherent inhibitor, Kelch-like ECHassociated protein 1 in the cytosol (Wakabayashi N 2004) then translocate to the nucleus and bind to a specific enhancer sequence, known as the antioxidant responsive element, in the promoter region of antioxidant genes, including HO-1 and thioredoxin 1 (Calvert JW 2009). H₂S exhibits effects on mitochondria function (Helmy N 2014, Wang CN 2014) antioxidant stress (Bos EM 2013, Du JT 2013) apoptosis (Yao LL 2010), inflammation (Lo Faro ML 2014) angiogenesis (Szabo C 2013, Coletta C 2012, Wang MJ 2010), sepsis and shock (Kida, F. 2015) and blood pressure (Polhemus DJ 2014, Ge SN 2014, Yang G 2008).

H₂S protects against NO₃⁻, as does glutathione. H₂S also significantly reduces the toxic effects of HOCl. H₂S enhances the anti-oxidant effects of N-acetyl-l-cysteine (NAC).

H₂S's therapeutic effects have been most studied to date in regards to heart disease. H₂S effects on heart disease include: macrophages are able to produce H₂S endogenously (Zhu XY 2010). NaHS (a H₂S donor) inhibited pro-atherogenic oxidized low-density lipoproteins induced foam cell formation in macrophages (Wang Y 2009). H₂S is able to down regulate ROS at the mitochondria, providing protection through reduced respiration (Chen Q 2006). H₂S production (10-100 nM) enhanced mitochondrial electron transport and cellular bioenergetics (Modis K 2013) however at high concentrations H₂S is toxic (Hill BC 1984, Nicholls P 1982). H₂S in the diet decreased adverse left ventricle (LV) remodeling during heart failure (Kondo K 2013). H₂S can upregulate endothelial nitric oxide synthase which makes NO (Kondo K 2013) and NO can upregulate the H₂S synthesis enzyme CSE (Zhao, W. 2001). Mice treated with a H₂S donor significantly increase phosphorylation effecting eNOS suggesting active cross talk between H₂S and NO (Kondo K 2013). There also appears to be cross talk between CO and H₂S (Zhange QY 2004, Majid AS 2013). H₂S induces vasodilation, leading to reduced blood pressure (Cheng Y 2004). H₂S in the form of Na₂S (10 minutes prior) prevents reperfusion injury (Sodha NR 2008). Exogenous H₂S also led to improved renal function (Xu Z 2009).

H₂S under in vivo conditions has an extremely short half-life which is estimated to be between seconds and minutes (Wang R 2002, Insko MA 2009). Plasma concentrations of H₂S is in the range of 0.034 to 0.065 mM (Whiteman M 2009), in the brain it is three fold higher than the plasma (Hogg P 2009, Zhao W 2001). H₂S concentration are inversely related to O₂ concentration and H₂S decrease cellular O₂ consumption (Olson K 2015). H₂S concentrations of between 0.030 and 0.300 have also been reported in the blood and plasma (Olson K 2009). H₂S donors NaHS and Na₂S increase H₂S concentration within seconds to minutes.

The physiological range of H₂S is widely variable from 0.005 to 0.300 mM (Predmore BL 2012). Endogenous levels of H₂S in the brains of humans have been detected at from 0.05 to 0.16 mM (Whiteman M 2004); in the brains of Alzheimer's patients, the H₂S concentration is lower (Seshadri S 2002, Tang X 2010). Diallyl trisulfide (DATS) is a stable H₂S donor and shows effects 30 minutes after injection and is longer lasting. NaHS can be taken in drinking water (Givvimani S 2011). NaHS (H₂S donor), in aqueous solution releases H₂S, in drinking water for 6 weeks. There was an increase in plasma H₂S concentration with exogenous supplementation (Peake BF 2013, Kondo K 2013). There was no difference in the consumption of water among the groups of mice treated with NaHS and untreated groups. Other H₂S donors include GYY 4137 (CAS# 106740-09-4) a water soluble H₂S donor that slowly releases H₂S over the course of hours (Li L 2008) and SG 1002 from Sulfagenix, Inc. AP97, AP39, AP67, and AP105 are also H₂S donors with slower release (Whiteman M 2015, Wallace J 2015, Hancock J 2014). H₂S can be ingested with foods containing organosulfides, who's polysulfides can be H₂S donors.

In addition to ingesting H₂S dissolved in water, H₂S can be inhaled and inhalation increases blood H₂S levels (40 ppm for 8 hours for 7 days was used with mice). Inhalation can also be combined with ingestible H₂S donors such as Na₂S and NaHS (Kida K 2011 and 2015). Measurement of H₂S in blood and tissue has been done with a sensitive and reliable means (Wintner E 2010).

H₂S can also be stored in cells in the form of sulfane sulfur and transported and released in response to physiological stimulus (Ishigami M. 2009).

### NRF2 Activators

The transcription factor NF-E2 p45-related factor 2 (Nrf2: gene name NFE212) regulates the expression of networks of genes encoding proteins with diverse cytoprotective activities. Nrf2 itself is controlled primarily at the level of protein stability. Nrf2 is a short lived protein subjected to continuous ubiquitination and protease degradation. There are three known ubiquitin ligase systems that contribute to the degradation of Nrf2 a) Keap-1, a substrate adaptor protein for Cullin-3, b) glycogen synthase kinase, and c) E3 ubiquitin ligase Hrd1. Keap-1 is also a sensor for a wide array of small-molecule activators also called inducers. When Nrf2 is not degraded and is translocated to the nucleus it forms a heterodimer with a small Maf protein, binds to antioxidant-response elements which are the upstream regulatory regions of its target genes and initiates transcription. Nrf2 is a master regulator of cellular redox homeostasis. (Dinkova-Kostova AT 2015). Over 50 genes are regulated by Nrf2 in humans (Pall ML 2015, Choi B-H 2014). In a direct effect of inflammation genes, without a Redox mechanism, Nrf2 also binds to the upstream region of the IL6 gene and when bound can significantly disrupt the recruitment of RNA polymerase II to regulate the transcription of IL6 in human macrophage cells.

Nrf2 signaling is regulated by transcriptional, translational, posttranslational, and epigenetic mechanisms as well as by other protein partners including p62, p21 and IQ motif-containing GTPase activating protein 1 (Huand Y 2015). Nuclear factor erythroid 2 (Nrf2) activators include classes of activators with activities that: induce nuclear translocation of Nrf2, increase Nrf2 mRNA transcription, increase protein expression of Nrf2 and increase Nrf2 downstream target genes. There are also Nrf2 inhibitors (Bach 1, caveolae, TGF-beta) (Gegotek A 2015). The Keap1-Nrf2 pathway acts in concert with autophagy to combat proteotoxicity (Dodson M 2015).

Keap-1 is a zinc metalloprotein that is localized near the plasma membrane. It has three functional domains, at least 25 reactive thiols most of which are found in the intervening linker region. Keap-1 has an Nrf2 binding site on each dimer subunit forming a "latch and hinge." Keap-1 is highly sensitive to oxidation and its different thiol groups have different redox potentials. These different cysteine residues create a sensor system (Suzuki T 2013).

Nrf2 is a 605 amino acid transcription factor composed of six domains. The N-terminal Neh2 domain is the binding site for the inhibitory protein Keap-1. The half-life of Nrf2 when separated from Keap-1 is 20 minutes (Kasper JW 2011). Keap-1 is exported out of the nucleus in 0.5 hours. Nrf2 activations enhances Sirt1 activity in mice fibroblasts cell culture (Jodar L 2010).

When Nrf2 releases Keap-1 it is available to capture IKKBeta thus inhibiting NF-κB target genes. This interaction correlates the expression of antioxidant enzymes by NrF2 and the turning on and off of the immune system by NF-κB. Nrf2 and NF-kB compete for CREB-binding protein (CBP) (Liu GH 2008). There are many phytochemicals that have Nrf2 activation abilities by interacting with Keap-1 in different ways. Immediate alkylators are fast activating. "Michael acceptors", which are acetylene compounds conjugated to an electron-withdrawing group, form reversible alkylating reactions with Keap-1 sensor thiols.

Phenolics that appear to act most directly on Nrf2 are ortho- or paradihydroxyphenols which can be oxidized to quinones (Kumar H 2014). Quinones are oxidized derivatives of aromatic compounds and are often readily made from reactive aromatic compounds with electron-donating substituents such as phenols and catechols, which increase the nucleophilicity of the ring and contributes to the large redox potential needed to break aromaticity. Quinones are conjugated but not aromatic. Quinones are electrophilic Michael acceptors stabilized by conjugation. Depending on the quinone and the site of reduction, reduction can either re-aromatize the compound or break the conjugation. Conjugate addition nearly always breaks the conjugation.

H₂O₂ and H₂S are Nrf2 activators (listed separately above). Everything mentioned that is a Nrf2 activator, is also an antioxidant defense system activator although some things activated by Nrf2 may be seen as additional to antioxidant defense system activation. The activation comes from the multiple ways listed above of keeping the Nrf2 system on. One form of regulation of Nrf2 is reversible phosphorylation. Sirt1 and PARP1 as discussed before can also be reversibly phosphorylated.

Nrf2 activation and the turning on of the antioxidant defense system needs to be correlated in timing to NAD+ availability and methylation availability and be synced with the biological clock NAD+ peaks of the person. The Nrf2 system does need to turn off (example: around 2 pm when NAD+ concentrations normally are at their daily biological clock low) so one's body can do the things it needs to do under a redox balance when that leans towards oxidation.

### Category 3 compounds

Antioxidant defense activators are chosen from HzOz, H₂S, metformin, hydrogen sulfide (H₂S), sodium hydrosulfide (NaHS), sodium sulfide (Na₂S), sulforaphane (in broccoli) (Nallasamy P 2014), curcumin (in turmeric) (Pae H-O 2007, He HJ 2012, Balogun E 2003, quercetin (in onions, apples, tea) (Magesh S 2012, Kimura S 2009), pterostilbene (McCormack D 2013, resveratrol (Cheng L 2015, Mokni M 2007, Kitada M 2011, apigenin (in parsley) (Paredes-Gonzalez X 2015 and 2014, Escande C 2013), zinc (Wang F 2015, Sternberg P 2007, Magesh S 2012) and any combination thereof.

### Specific Compositions

In specific examples, the disclosed nutritional composition can comprise nicotinamide adenine dinucleotide (NAD+), Betaine, and HzOz. In specific examples, the disclosed composition can comprise nicotinamide adenine dinucleotide (NAD+), folate + Vitamin B12, and HzOz. In specific examples, the disclosed nutritional composition can comprise nicotinamide adenine dinucleotide (NAD+), Methionine, and HzOz. In other examples, the disclosed composition can comprise nicotinamide riboside (NR), Methionine, and HzOz. In specific examples, the disclosed composition can comprise nicotinamide adenine dinucleotide (NAD+), Choline, and HzOz.

In specific examples, the disclosed composition can comprise nicotinamide adenine dinucleotide (NAD+), Betaine, and NaHS. In specific examples, the disclosed composition can comprise nicotinamide adenine dinucleotide (NAD+), Folate + Vitamin B12, and NaHS. In specific examples, the disclosed composition can comprise nicotinamide adenine dinucleotide (NAD+), Methionine, and NaHS. In specific examples, the disclosed composition can comprise nicotinamide adenine dinucleotide (NAD+), Choline, and NaHS.

In specific examples, the disclosed composition can comprise nicotinamide adenine dinucleotide (NAD+), Betaine, and Na₂S. In specific examples, the disclosed composition can comprise nicotinamide adenine dinucleotide (NAD+), Folate + Vitamin B12, and Na₂S. In specific examples, the disclosed composition can comprise nicotinamide adenine dinucleotide (NAD+), Methionine, and Na₂S. In specific examples, the disclosed composition can comprise nicotinamide adenine dinucleotide (NAD+), Choline, and Na₂S.

In specific examples, the disclosed composition can comprise nicotinamide adenine dinucleotide (NAD+), Betaine, and any one or more of H₂S, metformin, sulforaphane, curcumin, quercetin, pterostilbene, resveratrol, apigenin, and zinc. In specific examples, the disclosed composition can comprise nicotinamide adenine dinucleotide (NAD+), Folate + Vitamin B12, and any one or more of H₂S, metformin, sulforaphane, curcumin, quercetin, pterostilbene, resveratrol, apigenin, and zinc. In specific examples, the disclosed composition can comprise nicotinamide adenine dinucleotide (NAD+), Methionine, and any one or more of H₂S, metformin, sulforaphane, curcumin, quercetin, pterostilbene, resveratrol, apigenin, and zinc. In specific examples, the disclosed composition can comprise nicotinamide adenine dinucleotide (NAD+), Choline, and any one or more of H₂S, metformin, sulforaphane, curcumin, quercetin, pterostilbene, resveratrol, apigenin, and zinc.

In specific examples, the disclosed nutritional composition can comprise nicotinamide mononucleotide (NMN), Betaine, and H₂O₂. In other examples, the disclosed composition can comprise nicotinamide riboside (NR), Betaine, and HzOz. In other examples, the disclosed composition can comprise one or more of nicotinic acid adenine mononucleotide (NaMN), nicotinic acid adenine dinucleotide (NaAD), and nicotinic acid riboside (NAR), with Betaine, and HzOz. In other examples, the disclosed composition can comprise 1-methylnicotinamide (MNM) and/or cyclic adenosine monophosphate (cAMP), Betaine, and HzOz.

In specific examples, the disclosed composition can comprise nicotinamide mononucleotide (NMN), folate + Vitamin B12, and H₂O₂. In other examples, the disclosed composition can comprise nicotinamide riboside (NR), folate + Vitamin B12, and H₂O₂. In other examples, the disclosed composition can comprise one or more of nicotinic acid adenine mononucleotide (NaMN), nicotinic acid adenine dinucleotide (NaAD), and nicotinic acid riboside (NAR), folate + Vitamin B 12, and H₂O₂. In other examples, the disclosed composition can comprise 1-methylnicotinamide (MNM) and/or cyclic adenosine monophosphate (cAMP), folate + Vitamin B12, and H₂O₂.

In specific examples, the disclosed composition can comprise nicotinamide mononucleotide (NMN), Betaine + Vitamin B12, and H₂O₂. In other examples, the disclosed composition can comprise nicotinamide riboside (NR), Betaine + Vitamin B12, and HzOz. In other examples, the disclosed composition can comprise one or more of nicotinic acid adenine mononucleotide (NaMN), nicotinic acid adenine dinucleotide (NaAD), and nicotinic acid riboside (NAR), Betaine + Vitamin B12, and H₂O₂. In other examples, the disclosed composition can comprise 1-methylnicotinamide (MNM) and/or cyclic adenosine monophosphate (cAMP), Betaine + Vitamin B12, and HzOz.

In specific examples, the disclosed nutritional composition can comprise nicotinamide mononucleotide (NMN), Methionine, and HzOz. In other examples, the disclosed composition can comprise nicotinamide riboside (NR), Methionine, and HzOz. In other examples, the disclosed composition can comprise one or more of nicotinic acid adenine mononucleotide (NaMN), nicotinic acid adenine dinucleotide (NaAD), and nicotinic acid riboside (NAR), with Methionine, and HzOz. In other examples, the disclosed composition can comprise 1-methylnicotinamide (MNM) and/or cyclic adenosine monophosphate (cAMP), Methionine, and HzOz.

In specific examples, the disclosed composition can comprise nicotinamide mononucleotide (NMN), Choline, and HzOz. In other examples, the disclosed composition can comprise nicotinamide riboside (NR), Choline, and HzOz. In other examples, the disclosed composition can comprise one or more of nicotinic acid adenine mononucleotide (NaMN), nicotinic acid adenine dinucleotide (NaAD), and nicotinic acid riboside (NAR), Choline, and HzOz. In other examples, the disclosed composition can comprise 1-methylnicotinamide (MNM) and/or cyclic adenosine monophosphate (cAMP), Choline, and H₂O₂.

In specific examples, the disclosed composition can comprise nicotinamide mononucleotide (NMN), S-Adenosyl-methionine (SAM), and HzOz. In other examples, the disclosed composition can comprise nicotinamide riboside (NR), S-Adenosyl-methionine (SAM), and HzOz. In other examples, the disclosed composition can comprise one or more of nicotinic acid adenine mononucleotide (NaMN), nicotinic acid adenine dinucleotide (NaAD), and nicotinic acid riboside (NAR), S-Adenosyl-methionine (SAM), and HzOz. In other examples, the disclosed composition can comprise 1-methylnicotinamide (MNM) and/or cyclic adenosine monophosphate (cAMP), S-Adenosyl-methionine (SAM), and H₂O₂.

In specific examples, the disclosed composition can comprise nicotinamide mononucleotide (NMN), Betaine, and NaHS. In other examples, the disclosed composition can comprise nicotinamide riboside (NR), Betaine, and NaHS. In other examples, the disclosed composition can comprise one or more of nicotinic acid adenine mononucleotide (NaMN), nicotinic acid adenine dinucleotide (NaAD), and nicotinic acid riboside (NAR), Betaine, and NaHS. In other examples, the disclosed composition can comprise 1-methylnicotinamide (MNM) and/or cyclic adenosine monophosphate (cAMP), Betaine, and NaHS.

In specific examples, the disclosed composition can comprise nicotinamide mononucleotide (NMN), Folate + Vitamin B12, and NaHS. In other examples, the disclosed composition can comprise nicotinamide riboside (NR), Folate + Vitamin B12, and NaHS. In other examples, the disclosed composition can comprise one or more of nicotinic acid adenine mononucleotide (NaMN), nicotinic acid adenine dinucleotide (NaAD), and nicotinic acid riboside (NAR), Folate + Vitamin B 12, and NaHS. In other examples, the disclosed composition can comprise 1-methylnicotinamide (MNM) and/or cyclic adenosine monophosphate (cAMP), Folate + Vitamin B12, and NaHS.

In specific examples, the disclosed composition can comprise nicotinamide mononucleotide (NMN), Betaine + Vitamin B12, and NaHS. In other examples, the disclosed composition can comprise nicotinamide riboside (NR), Betaine + Vitamin B12, and NaHS. In other examples, the disclosed composition can comprise one or more of nicotinic acid adenine mononucleotide (NaMN), nicotinic acid adenine dinucleotide (NaAD), and nicotinic acid riboside (NAR), Betaine + Vitamin B12, and NaHS. In other examples, the disclosed composition can comprise 1-methylnicotinamide (MNM) and/or cyclic adenosine monophosphate (cAMP), Betaine + Vitamin B12, and NaHS.

In specific examples, the disclosed composition can comprise nicotinamide mononucleotide (NMN), Methionine, and NaHS. In other examples, the disclosed composition can comprise nicotinamide riboside (NR), Methionine, and NaHS. In other examples, the disclosed composition can comprise one or more of nicotinic acid adenine mononucleotide (NaMN), nicotinic acid adenine dinucleotide (NaAD), and nicotinic acid riboside (NAR), Methionine, and NaHS. In other examples, the disclosed composition can comprise 1-methylnicotinamide (MNM) and/or cyclic adenosine monophosphate (cAMP), Methionine, and NaHS.

In specific examples, the disclosed composition can comprise nicotinamide mononucleotide (NMN), Choline, and NaHS. In other examples, the disclosed composition can comprise nicotinamide riboside (NR), Choline, and NaHS. In other examples, the disclosed composition can comprise one or more of nicotinic acid adenine mononucleotide (NaMN), nicotinic acid adenine dinucleotide (NaAD), and nicotinic acid riboside (NAR), Choline, and NaHS. In other examples, the disclosed composition can comprise 1-methylnicotinamide (MNM) and/or cyclic adenosine monophosphate (cAMP), Choline, and NaHS.

In specific examples, the disclosed composition can comprise nicotinamide mononucleotide (NMN), S-Adenosyl-methionine (SAM), and NaHS. In other examples, the disclosed composition can comprise nicotinamide riboside (NR), S-Adenosyl-methionine (SAM), and NaHS. In other examples, the disclosed composition can comprise one or more of nicotinic acid adenine mononucleotide (NaMN), nicotinic acid adenine dinucleotide (NaAD), and nicotinic acid riboside (NAR), S-Adenosyl-methionine (SAM), and NaHS. In other examples, the disclosed composition can comprise 1-methylnicotinamide (MNM) and/or cyclic adenosine monophosphate (cAMP), S-Adenosyl-methionine (SAM), and NaHS.

In specific examples, the disclosed composition can comprise nicotinamide mononucleotide (NMN), Betaine, and Na₂S. In other examples, the disclosed composition can comprise nicotinamide riboside (NR), Betaine, and Na₂S. In other examples, the disclosed composition can comprise one or more of nicotinic acid adenine mononucleotide (NaMN), nicotinic acid adenine dinucleotide (NaAD), and nicotinic acid riboside (NAR), Betaine, and Na₂S. In other examples, the disclosed composition can comprise 1-methylnicotinamide (MNM) and/or cyclic adenosine monophosphate (cAMP), Betaine, and Na₂S.

In specific examples, the disclosed composition can comprise nicotinamide mononucleotide (NMN), Folate + Vitamin B12, and Na₂S. In other examples, the disclosed composition can comprise nicotinamide riboside (NR), Folate + Vitamin B12, and Na₂S. In other examples, the disclosed composition can comprise one or more of nicotinic acid adenine mononucleotide (NaMN), nicotinic acid adenine dinucleotide (NaAD), and nicotinic acid riboside (NAR), Folate + Vitamin B 12, and Na₂S. In other examples, the disclosed composition can comprise 1-methylnicotinamide (MNM) and/or cyclic adenosine monophosphate (cAMP), Folate + Vitamin B12, and Na₂S.

In specific examples, the disclosed composition can comprise nicotinamide mononucleotide (NMN), Betaine + Vitamin B12, and Na₂S. In other examples, the disclosed composition can comprise nicotinamide riboside (NR), Betaine + Vitamin B12, and Na₂S. In other examples, the disclosed composition can comprise one or more of nicotinic acid adenine mononucleotide (NaMN), nicotinic acid adenine dinucleotide (NaAD), and nicotinic acid riboside (NAR), Betaine + Vitamin B12, and Na₂S. In other examples, the disclosed composition can comprise 1-methylnicotinamide (MNM) and/or cyclic adenosine monophosphate (cAMP), Betaine + Vitamin B12, and Na₂S.

In specific examples, the disclosed composition can comprise nicotinamide mononucleotide (NMN), Methionine, and Na₂S. In other examples, the disclosed composition can comprise nicotinamide riboside (NR), Methionine, and Na₂S. In other examples, the disclosed composition can comprise one or more of nicotinic acid adenine mononucleotide (NaMN), nicotinic acid adenine dinucleotide (NaAD), and nicotinic acid riboside (NAR), Methionine, and Na₂S. In other examples, the disclosed composition can comprise 1-methylnicotinamide (MNM) and/or cyclic adenosine monophosphate (cAMP), Methionine, and Na₂S.

In specific examples, the disclosed composition can comprise nicotinamide mononucleotide (NMN), Choline, and Na₂S. In other examples, the disclosed composition can comprise nicotinamide riboside (NR), Choline, and Na₂S. In other examples, the disclosed composition can comprise one or more of nicotinic acid adenine mononucleotide (NaMN), nicotinic acid adenine dinucleotide (NaAD), and nicotinic acid riboside (NAR), Choline, and Na₂S. In other examples, the disclosed composition can comprise 1-methylnicotinamide (MNM) and/or cyclic adenosine monophosphate (cAMP), Choline, and Na₂S.

In specific examples, the disclosed composition can comprise nicotinamide mononucleotide (NMN), S-Adenosyl-methionine (SAM), and Na₂S. In other examples, the disclosed composition can comprise nicotinamide riboside (NR), S-Adenosyl-methionine (SAM), and Na₂S. In other examples, the disclosed composition can comprise one or more of nicotinic acid adenine mononucleotide (NaMN), nicotinic acid adenine dinucleotide (NaAD), and nicotinic acid riboside (NAR), S-Adenosyl-methionine (SAM), and Na₂S. In other examples, the disclosed composition can comprise 1-methylnicotinamide (MNM) and/or cyclic adenosine monophosphate (cAMP), S-Adenosyl-methionine (SAM), and Na₂S.

In specific examples, the disclosed composition can comprise nicotinamide mononucleotide (NMN), Betaine, and any one or more of H₂S, metformin, sulforaphane, curcumin, quercetin, pterostilbene, resveratrol, apigenin, and zinc. In other examples, the disclosed composition can comprise nicotinamide riboside (NR), Betaine, and any one or more of H₂S, metformin, sulforaphane, curcumin, quercetin, pterostilbene, resveratrol, apigenin, and zinc. In other examples, the disclosed composition can comprise one or more of nicotinic acid adenine mononucleotide (NaMN), nicotinic acid adenine dinucleotide (NaAD), and nicotinic acid riboside (NAR), Betaine, and any one or more of H₂S, metformin, sulforaphane, curcumin, quercetin, pterostilbene, resveratrol, apigenin, and zinc. In other examples, the disclosed composition can comprise 1-methylnicotinamide (MNM) and/or cyclic adenosine monophosphate (cAMP), Betaine, and any one or more of H₂S, metformin, sulforaphane, curcumin, quercetin, pterostilbene, resveratrol, apigenin, and zinc.

In specific examples, the disclosed composition can comprise nicotinamide mononucleotide (NMN), Folate + Vitamin B12, and any one or more of H₂S, metformin, sulforaphane, curcumin, quercetin, pterostilbene, resveratrol, apigenin, and zinc. In other examples, the disclosed composition can comprise nicotinamide riboside (NR), Folate + Vitamin B12, and any one or more of H₂S, metformin, sulforaphane, curcumin, quercetin, pterostilbene, resveratrol, apigenin, and zinc. In other examples, the disclosed composition can comprise one or more of nicotinic acid adenine mononucleotide (NaMN), nicotinic acid adenine dinucleotide (NaAD), and nicotinic acid riboside (NAR), Folate + Vitamin B12, and any one or more of H₂S, metformin, sulforaphane, curcumin, quercetin, pterostilbene, resveratrol, apigenin, and zinc. In other examples, the disclosed composition can comprise 1-methylnicotinamide (MNM) and/or cyclic adenosine monophosphate (cAMP), Folate + Vitamin B12, and any one or more of H₂S, metformin, sulforaphane, curcumin, quercetin, pterostilbene, resveratrol, apigenin, and zinc.

In specific examples, the disclosed composition can comprise nicotinamide mononucleotide (NMN), Betaine + Vitamin B12, and any one or more of H₂S, metformin, sulforaphane, curcumin, quercetin, pterostilbene, resveratrol, apigenin, and zinc. In other examples, the disclosed composition can comprise nicotinamide riboside (NR), Betaine + Vitamin B12, and any one or more of H₂S, metformin, sulforaphane, curcumin, quercetin, pterostilbene, resveratrol, apigenin, and zinc. In other examples, the disclosed composition can comprise one or more of nicotinic acid adenine mononucleotide (NaMN), nicotinic acid adenine dinucleotide (NaAD), and nicotinic acid riboside (NAR), Betaine + Vitamin B12, and any one or more of H₂S, metformin, sulforaphane, curcumin, quercetin, pterostilbene, resveratrol, apigenin, and zinc. In other examples, the disclosed composition can comprise 1-methylnicotinamide (MNM) and/or cyclic adenosine monophosphate (cAMP), Betaine + Vitamin B12, and any one or more of H₂S, metformin, sulforaphane, curcumin, quercetin, pterostilbene, resveratrol, apigenin, and zinc.

In specific examples, the disclosed composition can comprise nicotinamide mononucleotide (NMN), Methionine, and any one or more of H₂S, metformin, sulforaphane, curcumin, quercetin, pterostilbene, resveratrol, apigenin, and zinc. In other examples, the disclosed composition can comprise nicotinamide riboside (NR), Methionine, and any one or more of H₂S, metformin, sulforaphane, curcumin, quercetin, pterostilbene, resveratrol, apigenin, and zinc. In other examples, the disclosed composition can comprise one or more of nicotinic acid adenine mononucleotide (NaMN), nicotinic acid adenine dinucleotide (NaAD), and nicotinic acid riboside (NAR), Methionine, and any one or more of H₂S, metformin, sulforaphane, curcumin, quercetin, pterostilbene, resveratrol, apigenin, and zinc. In other examples, the disclosed composition can comprise 1-methylnicotinamide (MNM) and/or cyclic adenosine monophosphate (cAMP), Methionine, and any one or more of H₂S, metformin, sulforaphane, curcumin, quercetin, pterostilbene, resveratrol, apigenin, and zinc.

In specific examples, the disclosed composition can comprise nicotinamide mononucleotide (NMN), Choline, and any one or more of H₂S, metformin, sulforaphane, curcumin, quercetin, pterostilbene, resveratrol, apigenin, and zinc. In other examples, the disclosed composition can comprise nicotinamide riboside (NR), Choline, and any one or more of H₂S, metformin, sulforaphane, curcumin, quercetin, pterostilbene, resveratrol, apigenin, and zinc. In other examples, the disclosed composition can comprise one or more of nicotinic acid adenine mononucleotide (NaMN), nicotinic acid adenine dinucleotide (NaAD), and nicotinic acid riboside (NAR), Choline, and any one or more of H₂S, metformin, sulforaphane, curcumin, quercetin, pterostilbene, resveratrol, apigenin, and zinc. In other examples, the disclosed composition can comprise 1-methylnicotinamide (MNM) and/or cyclic adenosine monophosphate (cAMP), Choline, and any one or more of H₂S, metformin, sulforaphane, curcumin, quercetin, pterostilbene, resveratrol, apigenin, and zinc.

In specific examples, the disclosed composition can comprise nicotinamide mononucleotide (NMN), S-Adenosyl-methionine (SAM), and any one or more of H₂S, metformin, sulforaphane, curcumin, quercetin, pterostilbene, resveratrol, apigenin, and zinc. In other examples, the disclosed composition can comprise nicotinamide riboside (NR), S-Adenosyl-methionine (SAM), and any one or more of H₂S, metformin, sulforaphane, curcumin, quercetin, pterostilbene, resveratrol, apigenin, and zinc. In other examples, the disclosed composition can comprise one or more of nicotinic acid adenine mononucleotide (NaMN), nicotinic acid adenine dinucleotide (NaAD), and nicotinic acid riboside (NAR), S-Adenosyl-methionine (SAM), and any one or more of H₂S, metformin, sulforaphane, curcumin, quercetin, pterostilbene, resveratrol, apigenin, and zinc. In other examples, the disclosed composition can comprise 1-methylnicotinamide (MNM) and/or cyclic adenosine monophosphate (cAMP), S-Adenosyl-methionine (SAM), and any one or more of H₂S, metformin, sulforaphane, curcumin, quercetin, pterostilbene, resveratrol, apigenin, and zinc.

In the disclosed compositions, the combined amount of compounds of category 1, 2, and 3 in the composition can be at least 5 wt.% of the composition. For example, the repair system activator, the methyl donor, and the antioxidant defense activator can be at least 5 wt.% of the composition. In other example, the combined amount of compounds of category 1, 2, and 3 in the composition can be at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, or 100 wt.% of the composition, where any of the stated values can form an upper or lower endpoint of a range.

### Delivery system for ingredients of category 1, 2, and 3

Formulations, which can be packaged in a powder or lyophilized form, which can then have either hot or cold liquid added to them for reconstituting into a solution are disclosed. For example, the disclosed compositions could be mixed with compositions, such as is done in personal beverage systems, which make hot or cold coffee or tea or hot chocolate from individually packaged components and the addition of water. The disclosed compositions for use can be administered in vivo either alone or in a pharmaceutically acceptable carrier. By "pharmaceutically acceptable" is meant a material that is not biologically or otherwise undesirable, i.e., the material can be administered to a subject, along with the composition disclosed herein, without causing any undesirable biological effects. The carrier would naturally be selected to minimize any degradation of the active ingredient and to minimize any adverse side effects in the subject, as would be well known to one of skill in the art. The materials can be in solution, suspension (for example, incorporated into microparticles, liposomes, or cells).

### Microbiome interaction with delivery via digestive tract or skin

The mammalian intestinal microbiota is composed of up to 100 trillion microbes from over 500 genera of bacteria from two main phyla, namely *Bacteroidetes* and *Firmicutes.* A well-studied mammalian probiotic *Lactobacillus rhamnosus* GG is a potent inducer of ROS (Jones R 2014). Redox signaling mediates symbiosis between the gut microbiota and the intestine. In flies, increase in life span is correlated to increase formation of the oxidant H₂O₂ in the gut. H₂S protects the mucosal lining of the gastrointestinal tract against oxidative stress as well as regulates various functions including fluid transport, inflammation, acid induced HCO₃⁻ secretion (Yonezawa D 2007, Ise F 2011, Wallace J 2009+2010, Fiorucci S 2006, Kasparek M 2008, Takeuchi K 2011+2015). Gut microbiota composition in the elderly has been correlated to plasma Il-6 levels (Claesson MJ 2012).

A fasting molecule Crtc enhances immunity by making the gut barrier less permeable to bacteria. Gut bacteria that get across the gut barrier cause inflammation. This Crtc is a genetic switch in the brain that controls energy balance. This constant communication between the brain and the GI tract allows the body to keep tract of energy expenditures and stores. Crtc interacts with CREB (cAMP response element-binding protein). A partner of Crtc in the human brain is neuropeptide Y, which causes mammals to search for food. CREB activity is regulated by energy sensing Sirt1 and its ability to deacetylate CREB (Paz JC 2014). This links the level of NAD+ and the feeling of hunger. The glucose-regulated antagonism between (yet coordinated with) CREB and Sirt1 for Hes-1 transcription participates in the metabolic regulation of neurogenesis, this is important since a decline in neurogenesis accompanies brain aging (Bondolfi L 2004) and CREB transcription factor is activated by nutrient deprivation which is correlated to Sirtuin enzyme activity.

TNF in the circulation of humans that occurs as part of the aging process impairs inflammatory monocyte development function and is detrimental to anti-pneumococcal immunity. This is reversed with pharmacological reduction of TNF.

The formulation could have organisms such as bacteria in the microbiome extrude any or all of these three categories of compounds that are desired and add them directly into the gut. These organisms could extrude the desired compounds in the quantity and with the timing desired. These organisms could be introduced to the microbiome either from a selection of organisms that naturally occur in the microbiome or by the engineering of organisms that naturally occurs in the microbiome. The engineered organisms could be engineered to extrude these compounds in accordance to the introduced organism's and or the host's biological clock. The introduced organism could be engineered to extrude the desired amount of compound or compounds. Gene-drive could be used to switch all of the species in the gut of this type used to the introduced organism's gene type desired. A kill switch could be engineered into this introduced species as well to allow an elimination of these engineered species if they were not desired at a later date.

### Pharmaceutically Acceptable Carriers

The compositions disclosed herein can be for use in therapy in combination with a pharmaceutically acceptable carrier.

Suitable carriers and their formulations are described in Remington: The Science and Practice of Pharmacy (22nd ed.) ed. L.V. Loyd Jr., CBS Publishers & Distributors Grandville MI USA 2012. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers, which matrices are in the form of shaped articles, e.g., films, liposomes or microparticles. It will be apparent to those persons skilled in the art that certain carriers can be more preferable depending upon, for instance, the route of administration and concentration of composition being administered.

Pharmaceutical carriers are known to those skilled in the art. These most typically would be standard carriers for administration of drugs to humans, including solutions such as sterile water, saline, and buffered solutions at physiological pH. The compositions for use can be administered intramuscularly or subcutaneously. Other compounds will be administered according to standard procedures used by those skilled in the art.

Pharmaceutical compositions can include carriers, thickeners, diluents, buffers, preservatives, surface active agents and the like in addition to the molecule of choice. Pharmaceutical compositions can also include one or more active ingredients such as antimicrobial agents, anti-inflammatory agents, anesthetics, and the like.

The pharmaceutical composition for use can be administered in a number of ways depending on whether local or systemic treatment is desired, and on the area to be treated. Administration can be topically (including ophthalmically, vaginally, rectally, intranasally), orally, by inhalation, or parenterally, for example by intravenous drip, subcutaneous, intraperitoneal or intramuscular injection. The disclosed compounds can be administered intravenously, intraperitoneally, intramuscularly, subcutaneously, intracavity, or transdermally.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives can also be present such as, for example, antimicrobials, chelating agents, and inert gases and the like.

Formulations for topical administration can include ointments, lotions, creams, gels, drops, suppositories, sprays, liquids and powders. Conventional pharmaceutical carriers, aqueous, powder or oily bases, thickeners and the like may be necessary or desirable.

Compositions for oral administration include powders or granules, suspensions or solutions in water or non-aqueous media, capsules, sachets, or tablets. Thickeners, flavorings, diluents, emulsifiers, dispersing aids or binders may be desirable.

Some of the compositions for use can be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

The various compounds and compositions of categories 1, 2, and 3, can be taken at the same time or in proximity, such as within 1, 5, 10, 30, 60, 90, or 120 minutes.

Dosages of each item or items from category 1, 2, and 3 that is sufficient but not in excess (described in molar terms to body weight) and the ingredients are such that the interrelationship of these doses is balanced.

A delivery system in water is preferable if the preferred ingredient of category 1, 2 and 3 are used. This will help elicit the correct timing (all 3 preferred ingredients are easily absorbed and soluble in water). For some other less preferred ingredients, which are not as water soluble or are not as easily absorbed their delivery would result in a reduced benefit with respect to the pulse timing of these three categories of ingredients.

Disclosed are compositions according to any of the aspects described herein for use in reducing inflammation in a subject.

In one embodiment, compositions for use as described herein are disclosed wherein the first compound, the second compound, and the third compound are administered at approximately the same time.

Also disclosed are compositions for use, wherein the first compound is administered within 15, 30, 60, 90, or 120 minutes of the subject's biological clock NAD+ peak.

Also disclosed are compositions for use, wherein the compositions are administered to a subject a dosage of at least 1 × 10⁻⁸ moles of the first compound to the subject, 1 × 10⁻⁸ moles of the second compound to the subject, and 1 × 10⁻⁹ moles of the third compound to the subject.

Also disclosed are compositions for use, wherein the composition is injected over 8-12 days.

Also disclosed are compositions for use, wherein the composition is an aerosol, lyophilization, powder, or emulsion.

Also disclosed are compositions for use, wherein the subject is a human.

Also disclosed are compositions for use, wherein the human is treated for at least two months.

Also disclosed are compositions for use, wherein the composition is a tablet that is administered orally at least once daily.

Also disclosed are compositions for use, wherein the composition is administered once daily.

The disclosed compositions for use can be administered at a variety of dosages. For example category 1 compounds like Nicotinamide Mononucleotide (NMN), can be at dosages per day of 1 × 10⁻⁶ moles/kg to 1 × 10⁻² moles/kg or 1 × 10⁻⁵ moles/kg to 1 × 10⁻³ moles/kg or 1 × 10⁻⁴ moles/kg to 1 × 10⁻³ moles/kg or 2 × 10⁻⁴ moles/kg to 7 × 10⁻⁴ moles/kg. In certain embodiments, the dosages per day of the category 1 molecule can be at least 1 × 10⁻⁶ moles/kg, 1 × 10⁻⁵ moles/kg, 1 × 10⁻⁴ moles/kg, 1 × 10⁻³ moles/kg or 1 × 10⁻² moles/kg. The dosages can also be at least 2.38 moles/kg per day. The same dosages are contemplated herein for other category 1 compounds NAD+, NR, NaMN, NaAD, NAR, MNM, and cAMP.

The dosage of category 2 compounds, such as betaine, can be at dosages per day of 1 × 10⁻⁶ moles/kg to 1 × 10⁻² moles/kg or 1 × 10⁻⁵ moles/kg to 1 × 10⁻³ moles/kg or 1 × 10⁻⁴ moles/kg to 1 × 10⁻³ moles/kg or 2 × 10⁻⁴ moles/kg to 7 × 10⁻⁴ moles/kg. In certain embodiments, the dosages per day of the category 2 compound can be at least 1 × 10⁻⁶ moles/kg, 1 × 10⁻⁵ moles/kg, 1 × 10⁻⁴ moles/kg, 1 × 10⁻³ moles/kg or 1 × 10⁻² moles/kg. The dosages can also be at least 5.82 × 10⁻⁴ moles / kg body weight / day.

The dosages of category 3 compounds, such as HzOz, can be at dosages per day of 1 × 10⁻⁷ moles/kg to 1 × 10⁻² moles/kg or 1 × 10⁻⁶ moles/kg to 1 × 10⁻³ moles/kg or 1 × 10⁻⁵ moles/kg to 1 × 10⁻⁴ moles/kg or 1 × 10⁻⁵ moles/kg to 7 × 10⁻⁵ moles/kg. In certain embodiments, the dosages per day of the category 3 compound can be at least 1 × 10⁻⁷ moles/kg, 1 × 10⁻⁶ moles/kg, 1 × 10⁻⁵ moles/kg, 1 × 10⁻⁴ moles/kg or 1 × 10⁻³ moles/kg. The dosages can also be at least dosage 2.34 × 10⁻⁵ moles / kg body weight / day.

The dosages of category 3 compounds, such as NaSH, can be at dosages per day of 1 × 10⁻⁸ moles/kg to 1 × 10⁻³ moles/kg or 1 × 10⁻⁷ moles/kg to 1 × 10⁻⁴ moles/kg or 1 × 10⁻⁶ moles/kg to 1 × 10⁻⁵ moles/kg or 1 × 10⁻⁶ moles/kg to 7 × 10⁻⁶ moles/kg. In certain embodiments, the dosages per day of the category 3 compound can be at least 1 × 10⁻⁸ moles/kg, 1 × 10⁻⁷ moles/kg, 1 × 10⁻⁶ moles/kg, 1 × 10⁻⁴ moles/kg or 1 × 10⁻³ moles/kg. In certain embodiments, the dosages can also be at least 3.02 × 10⁻⁶ moles / Kg body weight / day.

### Specific Compositions for use

Disclosed are compositions and products for use in reducing inflammation in a subject. In specific examples, the disclosed compositions/products for use can comprise nicotinamide adenine dinucleotide (NAD+), S-Adenosyl-methionine (SAM), and HzOz. In specific examples, the disclosed compositions/products for use can comprise nicotinamide adenine dinucleotide (NAD+), S-Adenosyl-methionine (SAM), and NaSH. In specific examples, the disclosed compositions/products for use can comprise nicotinamide adenine dinucleotide (NAD+), S-Adenosyl-methionine (SAM), and Na₂S. In specific examples, the disclosed compositions/products for use can comprise nicotinamide adenine dinucleotide (NAD+), S-Adenosyl-methionine (SAM), and any one or more of H₂S, metformin, sulforaphane, curcumin, quercetin, pterostilbene, resveratrol, apigenin, and zinc.

In specific examples, the disclosed compositions/products for use can comprise nicotinamide adenine dinucleotide (NAD+), Betaine, and HzOz. In specific examples, the disclosed compositions/products for use can comprise nicotinamide adenine dinucleotide (NAD+), folate + Vitamin B12, and H₂O₂. In specific examples, the disclosed compositions/products for use can comprise nicotinamide adenine dinucleotide (NAD+), Methionine, and HzOz. In other examples, the disclosed compositions/products for use can comprise nicotinamide riboside (NR), Methionine, and HzOz. In specific examples, the disclosed compositions/products for use can comprise nicotinamide adenine dinucleotide (NAD+), Choline, and H₂O₂.

In specific examples, the disclosed compositions/products for use can comprise nicotinamide adenine dinucleotide (NAD+), Betaine, and NaHS. In specific examples, the disclosed compositions/products for use can comprise nicotinamide adenine dinucleotide (NAD+), Folate + Vitamin B12, and NaHS. In specific examples, the disclosed compositions/products for use can comprise nicotinamide adenine dinucleotide (NAD+), Methionine, and NaHS. In specific examples, the disclosed compositions/products for use can comprise nicotinamide adenine dinucleotide (NAD+), Choline, and NaHS.

In specific examples, the disclosed compositions/products for use can comprise nicotinamide adenine dinucleotide (NAD+), Betaine, and Na₂S. In specific examples, the disclosed compositions/products for use can comprise nicotinamide adenine dinucleotide (NAD+), Folate + Vitamin B12, and Na₂S. In specific examples, the disclosed compositions/products for use can comprise nicotinamide adenine dinucleotide (NAD+), Methionine, and Na₂S. In specific examples, the disclosed compositions/products for use can comprise nicotinamide adenine dinucleotide (NAD+), Choline, and Na₂S.

In specific examples, the disclosed compositions/products for use can comprise nicotinamide adenine dinucleotide (NAD+), Betaine, and any one or more of H₂S, metformin, sulforaphane, curcumin, quercetin, pterostilbene, resveratrol, apigenin, and zinc. In specific examples, the disclosed compositions/products for use can comprise nicotinamide adenine dinucleotide (NAD+), Folate + Vitamin B12, and any one or more of H₂S, metformin, sulforaphane, curcumin, quercetin, pterostilbene, resveratrol, apigenin, and zinc. In specific examples, the disclosed compositions/products for use can comprise nicotinamide adenine dinucleotide (NAD+), Methionine, and any one or more of H₂S, metformin, sulforaphane, curcumin, quercetin, pterostilbene, resveratrol, apigenin, and zinc. In specific examples, the disclosed compositions/products for use can comprise nicotinamide adenine dinucleotide (NAD+), Choline, and any one or more of H₂S, metformin, sulforaphane, curcumin, quercetin, pterostilbene, resveratrol, apigenin, and zinc.

In specific examples, the disclosed compositions/products for use can comprise nicotinamide mononucleotide (NMN), Betaine, and H₂O₂. In other examples, the disclosed compositions/products for use can comprise nicotinamide riboside (NR), Betaine, and H₂O₂. In other examples, the disclosed compositions/products for use can comprise one or more of nicotinic acid adenine mononucleotide (NaMN), nicotinic acid adenine dinucleotide (NaAD), and nicotinic acid riboside (NAR), with Betaine, and H₂O₂. In other examples, the disclosed compositions/products for use can comprise 1-methylnicotinamide (MNM) and/or cyclic adenosine monophosphate (cAMP), Betaine, and HzOz.

In specific examples, the disclosed compositions/products for use can comprise nicotinamide mononucleotide (NMN), folate + Vitamin B12, and H₂O₂. In other examples, the disclosed compositions/products for use can comprise nicotinamide riboside (NR), folate + Vitamin B 12, and H₂O₂. In other examples, the disclosed compositions/products for use can comprise one or more of nicotinic acid adenine mononucleotide (NaMN), nicotinic acid adenine dinucleotide (NaAD), and nicotinic acid riboside (NAR), folate + Vitamin B 12, and HzOz. In other examples, the disclosed compositions/products for use can comprise 1-methylnicotinamide (MNM) and/or cyclic adenosine monophosphate (cAMP), folate + Vitamin B 12, and H₂O₂.

In specific examples, the disclosed compositions/products for use can comprise nicotinamide mononucleotide (NMN), Betaine + Vitamin B 12, and HzOz. In other examples, the disclosed compositions/products for use can comprise nicotinamide riboside (NR), Betaine + Vitamin B12, and HzOz. In other examples, the disclosed compositions/products for use can comprise one or more of nicotinic acid adenine mononucleotide (NaMN), nicotinic acid adenine dinucleotide (NaAD), and nicotinic acid riboside (NAR), Betaine + Vitamin B12, and HzOz. In other examples, the disclosed compositions/products for use can comprise 1-methylnicotinamide (MNM) and/or cyclic adenosine monophosphate (cAMP), Betaine + Vitamin B 12, and HzOz.

In specific examples, the disclosed compositions/products for use can comprise nicotinamide mononucleotide (NMN), Methionine, and HzOz. In other examples, the disclosed compositions/products for use can comprise nicotinamide riboside (NR), Methionine, and HzOz. In other examples, the disclosed compositions/products for use can comprise one or more of nicotinic acid adenine mononucleotide (NaMN), nicotinic acid adenine dinucleotide (NaAD), and nicotinic acid riboside (NAR), with Methionine, and HzOz. In other examples, the disclosed compositions/products for use can comprise 1-methylnicotinamide (MNM) and/or cyclic adenosine monophosphate (cAMP), Methionine, and H₂O₂.

In specific examples, the disclosed compositions/products for use can comprise nicotinamide mononucleotide (NMN), Choline, and HzOz. In other examples, the disclosed compositions/products for use can comprise nicotinamide riboside (NR), Choline, and HzOz. In other examples, the disclosed compositions/products for use can comprise one or more of nicotinic acid adenine mononucleotide (NaMN), nicotinic acid adenine dinucleotide (NaAD), and nicotinic acid riboside (NAR), Choline, and HzOz. In other examples, the disclosed compositions/products for use can comprise 1-methylnicotinamide (MNM) and/or cyclic adenosine monophosphate (cAMP), Choline, and HzOz.

In specific examples, the disclosed compositions/products for use can comprise nicotinamide mononucleotide (NMN), S-Adenosyl-methionine (SAM), and HzOz. In other examples, the disclosed compositions/products for use can comprise nicotinamide riboside (NR), S-Adenosyl-methionine (SAM), and H₂O₂. In other examples, the disclosed compositions/products for use can comprise one or more of nicotinic acid adenine mononucleotide (NaMN), nicotinic acid adenine dinucleotide (NaAD), and nicotinic acid riboside (NAR), S-Adenosyl-methionine (SAM), and HzOz. In other examples, the disclosed compositions/products for use can comprise 1-methylnicotinamide (MNM) and/or cyclic adenosine monophosphate (cAMP), S-Adenosyl-methionine (SAM), and HzOz.

In specific examples, the disclosed compositions/products for use can comprise nicotinamide mononucleotide (NMN), Betaine, and NaHS. In other examples, the disclosed compositions/products for use can comprise nicotinamide riboside (NR), Betaine, and NaHS. In other examples, the disclosed compositions/products for use can comprise one or more of nicotinic acid adenine mononucleotide (NaMN), nicotinic acid adenine dinucleotide (NaAD), and nicotinic acid riboside (NAR), Betaine, and NaHS. In other examples, the disclosed compositions/products for use can comprise 1-methylnicotinamide (MNM) and/or cyclic adenosine monophosphate (cAMP), Betaine, and NaHS.

In specific examples, the disclosed compositions/products for use can comprise nicotinamide mononucleotide (NMN) , Folate + Vitamin B12, and NaHS. In other examples, the disclosed compositions/products for use can comprise nicotinamide riboside (NR), Folate + Vitamin B12, and NaHS. In other examples, the disclosed compositions/products for use can comprise one or more of nicotinic acid adenine mononucleotide (NaMN), nicotinic acid adenine dinucleotide (NaAD), and nicotinic acid riboside (NAR), Folate + Vitamin B12, and NaHS. In other examples, the disclosed compositions/products for use can comprise 1-methylnicotinamide (MNM) and/or cyclic adenosine monophosphate (cAMP), Folate + Vitamin B12, and NaHS.

In specific examples, the disclosed compositions/products for use can comprise nicotinamide mononucleotide (NMN), Betaine + Vitamin B12, and NaHS. In other examples, the disclosed compositions/products for use can comprise nicotinamide riboside (NR), Betaine + Vitamin B12, and NaHS. In other examples, the disclosed compositions/products for use can comprise one or more of nicotinic acid adenine mononucleotide (NaMN), nicotinic acid adenine dinucleotide (NaAD), and nicotinic acid riboside (NAR), Betaine + Vitamin B12, and NaHS. In other examples, the disclosed compositions/products for use can comprise 1-methylnicotinamide (MNM) and/or cyclic adenosine monophosphate (cAMP), Betaine + Vitamin B12, and NaHS.

In specific examples, the disclosed compositions/products for use can comprise nicotinamide mononucleotide (NMN), Methionine, and NaHS. In other examples, the disclosed compositions/products for use can comprise nicotinamide riboside (NR), Methionine, and NaHS. In other examples, the disclosed compositions/products for use can comprise one or more of nicotinic acid adenine mononucleotide (NaMN), nicotinic acid adenine dinucleotide (NaAD), and nicotinic acid riboside (NAR), Methionine, and NaHS. In other examples, the disclosed compositions/products for use can comprise 1-methylnicotinamide (MNM) and/or cyclic adenosine monophosphate (cAMP), Methionine, and NaHS.

In specific examples, the disclosed compositions/products for use can comprise nicotinamide mononucleotide (NMN), Choline, and NaHS. In other examples, the disclosed compositions/products for use can comprise nicotinamide riboside (NR), Choline, and NaHS. In other examples, the disclosed compositions/products for use can comprise one or more of nicotinic acid adenine mononucleotide (NaMN), nicotinic acid adenine dinucleotide (NaAD), and nicotinic acid riboside (NAR), Choline, and NaHS. In other examples, the disclosed compositions/products for use can comprise 1-methylnicotinamide (MNM) and/or cyclic adenosine monophosphate (cAMP), Choline, and NaHS.

In specific examples, the disclosed compositions/products for use can comprise nicotinamide mononucleotide (NMN), S-Adenosyl-methionine (SAM), and NaHS. In other examples, the disclosed compositions/products for use can comprise nicotinamide riboside (NR), S-Adenosyl-methionine (SAM), and NaHS. In other examples, the disclosed compositions/products for use can comprise one or more of nicotinic acid adenine mononucleotide (NaMN), nicotinic acid adenine dinucleotide (NaAD), and nicotinic acid riboside (NAR), S-Adenosyl-methionine (SAM), and NaHS. In other examples, the disclosed compositions/products for use can comprise 1-methylnicotinamide (MNM) and/or cyclic adenosine monophosphate (cAMP), S-Adenosyl-methionine (SAM), and NaHS.

In specific examples, the disclosed compositions/products for use can comprise nicotinamide mononucleotide (NMN), Betaine, and Na₂S. In other examples, the disclosed compositions/products for use can comprise nicotinamide riboside (NR), Betaine, and Na₂S. In other examples, the disclosed compositions/products for use can comprise one or more of nicotinic acid adenine mononucleotide (NaMN), nicotinic acid adenine dinucleotide (NaAD), and nicotinic acid riboside (NAR), Betaine, and Na₂S. In other examples, the disclosed compositions/products for use can comprise 1-methylnicotinamide (MNM) and/or cyclic adenosine monophosphate (cAMP), Betaine, and Na₂S.

In specific examples, the disclosed compositions/products for use can comprise nicotinamide mononucleotide (NMN), Folate + Vitamin B12, and Na₂S. In other examples, the disclosed compositions/products for use can comprise nicotinamide riboside (NR), Folate + Vitamin B12, and Na₂S. In other examples, the disclosed compositions/products for use can comprise one or more of nicotinic acid adenine mononucleotide (NaMN), nicotinic acid adenine dinucleotide (NaAD), and nicotinic acid riboside (NAR), Folate + Vitamin B12, and Na₂S. In other examples, the disclosed compositions/products for use can comprise 1-methylnicotinamide (MNM) and/or cyclic adenosine monophosphate (cAMP), Folate + Vitamin B12, and Na₂S.

In specific examples, the disclosed compositions/products for use can comprise nicotinamide mononucleotide (NMN), Betaine + Vitamin B12, and Na₂S. In other examples, the disclosed compositions/products for use can comprise nicotinamide riboside (NR), Betaine + Vitamin B12, and Na₂S. In other examples, the disclosed compositions/products for use can comprise one or more of nicotinic acid adenine mononucleotide (NaMN), nicotinic acid adenine dinucleotide (NaAD), and nicotinic acid riboside (NAR), Betaine + Vitamin B12, and Na₂S. In other examples, the disclosed compositions/products for use can comprise 1-methylnicotinamide (MNM) and/or cyclic adenosine monophosphate (cAMP), Betaine + Vitamin B12, and Na₂S.

In specific examples, the disclosed compositions/products for use can comprise nicotinamide mononucleotide (NMN), Methionine, and Na₂S. In other examples, the disclosed compositions/products for use can comprise nicotinamide riboside (NR), Methionine, and Na₂S. In other examples, the disclosed compositions/products for use can comprise one or more of nicotinic acid adenine mononucleotide (NaMN), nicotinic acid adenine dinucleotide (NaAD), and nicotinic acid riboside (NAR), Methionine, and Na₂S. In other examples, the disclosed compositions/products for use can comprise 1-methylnicotinamide (MNM) and/or cyclic adenosine monophosphate (cAMP), Methionine, and Na₂S.

In specific examples, the disclosed compositions/products for use can comprise nicotinamide mononucleotide (NMN), Choline, and Na₂S. In other examples, the disclosed compositions/products for use can comprise nicotinamide riboside (NR), Choline, and Na₂S. In other examples, the disclosed compositions/products for use can comprise one or more of nicotinic acid adenine mononucleotide (NaMN), nicotinic acid adenine dinucleotide (NaAD), and nicotinic acid riboside (NAR), Choline, and Na₂S. In other examples, the disclosed compositions/products for use can comprise 1-methylnicotinamide (MNM) and/or cyclic adenosine monophosphate (cAMP), Choline, and Na₂S.

In specific examples, the disclosed compositions/products for use can comprise nicotinamide mononucleotide (NMN), S-Adenosyl-methionine (SAM), and Na₂S. In other examples, the disclosed compositions/products for use can comprise nicotinamide riboside (NR), S-Adenosyl-methionine (SAM), and Na₂S. In other examples, the disclosed compositions/products for use can comprise one or more of nicotinic acid adenine mononucleotide (NaMN), nicotinic acid adenine dinucleotide (NaAD), and nicotinic acid riboside (NAR), S-Adenosyl-methionine (SAM), and Na₂S. In other examples, the disclosed compositions/products for use can comprise 1-methylnicotinamide (MNM) and/or cyclic adenosine monophosphate (cAMP), S-Adenosyl-methionine (SAM), and Na₂S.

In specific examples, the disclosed compositions/products for use can comprise nicotinamide mononucleotide (NMN), Betaine, and any one or more of H₂S, metformin, sulforaphane, curcumin, quercetin, pterostilbene, resveratrol, apigenin, and zinc. In other examples, the disclosed compositions/products for use can comprise nicotinamide riboside (NR), Betaine, and any one or more of H₂S, metformin, sulforaphane, curcumin, quercetin, pterostilbene, resveratrol, apigenin, and zinc. In other examples, the disclosed compositions/products for use can comprise one or more of nicotinic acid adenine mononucleotide (NaMN), nicotinic acid adenine dinucleotide (NaAD), and nicotinic acid riboside (NAR), Betaine, and any one or more of H₂S, metformin, sulforaphane, curcumin, quercetin, pterostilbene, resveratrol, apigenin, and zinc. In other examples, the disclosed compositions/products for use can comprise 1-methylnicotinamide (MNM) and/or cyclic adenosine monophosphate (cAMP), Betaine, and any one or more of H₂S, metformin, sulforaphane, curcumin, quercetin, pterostilbene, resveratrol, apigenin, and zinc.

In specific examples, the disclosed compositions/products for use can comprise nicotinamide mononucleotide (NMN), Folate + Vitamin B12, and any one or more of H₂S, metformin, sulforaphane, curcumin, quercetin, pterostilbene, resveratrol, apigenin, and zinc. In other examples, the disclosed compositions/products for use can comprise nicotinamide riboside (NR), Folate + Vitamin B12, and any one or more of H₂S, metformin, sulforaphane, curcumin, quercetin, pterostilbene, resveratrol, apigenin, and zinc. In other examples, the disclosed compositions/products for use can comprise one or more of nicotinic acid adenine mononucleotide (NaMN), nicotinic acid adenine dinucleotide (NaAD), and nicotinic acid riboside (NAR), Folate + Vitamin B12, and any one or more of H₂S, metformin, sulforaphane, curcumin, quercetin, pterostilbene, resveratrol, apigenin, and zinc. In other examples, the disclosed compositions/products for use can comprise 1-methylnicotinamide (MNM) and/or cyclic adenosine monophosphate (cAMP), Folate + Vitamin B12, and any one or more of H₂S, metformin, sulforaphane, curcumin, quercetin, pterostilbene, resveratrol, apigenin, and zinc.

In specific examples, the disclosed compositions/products for use can comprise nicotinamide mononucleotide (NMN), Betaine + Vitamin B12, and any one or more of H₂S, metformin, sulforaphane, curcumin, quercetin, pterostilbene, resveratrol, apigenin, and zinc. In other examples, the disclosed compositions/products for use can comprise nicotinamide riboside (NR), Betaine + Vitamin B12, and any one or more of H₂S, metformin, sulforaphane, curcumin, quercetin, pterostilbene, resveratrol, apigenin, and zinc. In other examples, the disclosed compositions/products for use can comprise one or more of nicotinic acid adenine mononucleotide (NaMN), nicotinic acid adenine dinucleotide (NaAD), and nicotinic acid riboside (NAR), Betaine + Vitamin B12, and any one or more of H₂S, metformin, sulforaphane, curcumin, quercetin, pterostilbene, resveratrol, apigenin, and zinc. In other examples, the disclosed compositions/products for use can comprise 1-methylnicotinamide (MNM) and/or cyclic adenosine monophosphate (cAMP), Betaine + Vitamin B12, and any one or more of H₂S, metformin, sulforaphane, curcumin, quercetin, pterostilbene, resveratrol, apigenin, and zinc.

In specific examples, the disclosed compositions/products for use can comprise nicotinamide mononucleotide (NMN), Methionine, and any one or more of H₂S, metformin, sulforaphane, curcumin, quercetin, pterostilbene, resveratrol, apigenin, and zinc. In other examples, the disclosed compositions/products for use can comprise nicotinamide riboside (NR), Methionine, and any one or more of H₂S, metformin, sulforaphane, curcumin, quercetin, pterostilbene, resveratrol, apigenin, and zinc. In other examples, the disclosed compositions/products for use can comprise one or more of nicotinic acid adenine mononucleotide (NaMN), nicotinic acid adenine dinucleotide (NaAD), and nicotinic acid riboside (NAR), Methionine, and any one or more of H₂S, metformin, sulforaphane, curcumin, quercetin, pterostilbene, resveratrol, apigenin, and zinc. In other examples, the disclosed compositions/products for use can comprise 1-methylnicotinamide (MNM) and/or cyclic adenosine monophosphate (cAMP), Methionine, and any one or more of H₂S, metformin, sulforaphane, curcumin, quercetin, pterostilbene, resveratrol, apigenin, and zinc.

In specific examples, the disclosed compositions/products for use can comprise nicotinamide mononucleotide (NMN), Choline, and any one or more of H₂S, metformin, sulforaphane, curcumin, quercetin, pterostilbene, resveratrol, apigenin, and zinc. In other examples, the disclosed compositions/products for use can comprise nicotinamide riboside (NR), Choline, and any one or more of H₂S, metformin, sulforaphane, curcumin, quercetin, pterostilbene, resveratrol, apigenin, and zinc. In other examples, the disclosed compositions/products for use can comprise one or more of nicotinic acid adenine mononucleotide (NaMN), nicotinic acid adenine dinucleotide (NaAD), and nicotinic acid riboside (NAR), Choline, and any one or more of H₂S, metformin, sulforaphane, curcumin, quercetin, pterostilbene, resveratrol, apigenin, and zinc. In other examples, the disclosed compositions/products for use can comprise 1-methylnicotinamide (MNM) and/or cyclic adenosine monophosphate (cAMP), Choline, and any one or more of H₂S, metformin, sulforaphane, curcumin, quercetin, pterostilbene, resveratrol, apigenin, and zinc.

In specific examples, the disclosed compositions/products for use can comprise nicotinamide mononucleotide (NMN), S-Adenosyl-methionine (SAM), and any one or more of H₂S, metformin, sulforaphane, curcumin, quercetin, pterostilbene, resveratrol, apigenin, and zinc. In other examples, the disclosed compositions/products for use can comprise nicotinamide riboside (NR), S-Adenosyl-methionine (SAM), and any one or more of H₂S, metformin, sulforaphane, curcumin, quercetin, pterostilbene, resveratrol, apigenin, and zinc. In other examples, the disclosed compositions/products for use can comprise one or more of nicotinic acid adenine mononucleotide (NaMN), nicotinic acid adenine dinucleotide (NaAD), and nicotinic acid riboside (NAR), S-Adenosyl-methionine (SAM), and any one or more of H₂S, metformin, sulforaphane, curcumin, quercetin, pterostilbene, resveratrol, apigenin, and zinc. In other examples, the disclosed compositions/products for use can comprise 1-methylnicotinamide (MNM) and/or cyclic adenosine monophosphate (cAMP), S-Adenosyl-methionine (SAM), and any one or more of H₂S, metformin, sulforaphane, curcumin, quercetin, pterostilbene, resveratrol, apigenin, and zinc.

### Surrogate markers for aging

A variety of markers can be used as surrogates for monitoring aging.

### DNA Methylation levels

DNA methylation levels change with age. Studies have identified biomarkers of chronological age based on DNA methylation levels called an "epigenetic clock" (Horvath S 2013 based on 353 dinucleotide CpG markers). Differences between DNA methylation age and chronological age led to the conclusion that DNA methylation-derived measures of biological aging are traits that predict mortality independently of health status, lifestyle factors, and known genetic factors (Marioni RE 2015). This epigenetic clock is tissue specific since some tissues age faster than others. The cerebellum ages more slowly than other parts of the body (Horvath S 2015). HIV-1-infected individuals show accelerated aging with this epigenetic clock (Rickabaugh TM 2015). Methylation data can be collected from circulating T cells and monocytes and was done so in a population cohort of 1264 participants (Reynolds LM 2014).

### DNA breakage

Single stranded and double stranded DNA breakage has not been used as methylation has for a biological clock but it is correlated to aging (Yu Q 2015) with older age having more breakage on average. Companies such as Exogen Biotechnology are able to test for single stranded and double stranded DNA breakage. NAD+ is used in DNA repair by PARP and Sirtuin enzymes, thus seeing less DNA breakage is an indication that these enzyme systems are working.

### Inflammation markers

Inflammation markers can be analyzed for aging including those markers found in the study by Arai in 2015. Arai found inflammation markers that were predictive of who would continue to live (life-span) and who would be physically and cognitively healthy (health-span).The markers used were CMV IgG, IL-6, TNF-alpha and CRP.

### Other markers associated with aging

Global loss of H3K9me3 or the resulting heterochromatin architecture changes correlate to biological aging as was shown in the human aging caused by Werner syndrome's premature aging and this can also be analyzed (Zhang W 2015).

A variety of compounds in blood correlate to age, as well as effect age and can be measure. An example is TGF-beta, which is lower in younger individuals than older individuals.

Metabalomic measurements have been correlated to aging using a nonlinear regression technique and a 13 year follow up.

Peripheral blood leukocyte telomere length can be measured and compared to 64,637 individuals of known age (Rode L 2015), although telomere length is only modestly correlated to age (r=0.5) and cellular aging continues regardless of telomere length.

### Definitions

In this specification and in the claims that follow, reference will be made to a number of terms, which shall be defined to have the following meanings:
Throughout the description and claims of this specification the word "comprise" and other forms of the word, such as "comprising" and "comprises," means including but not limited to, and is not intended to exclude, for example, other additives, components, integers, or steps.

As used in the description and the appended claims, the singular forms "a," "an," and "the" include plural referents unless the context clearly dictates otherwise. Thus, for example, reference to "a composition" includes mixtures of two or more such compositions, reference to "the compound" includes mixtures of two or more such compounds, reference to "an agent" includes mixture of two or more such agents, and the like.

"Optional" or "optionally" means that the subsequently described event or circumstance can or cannot occur, and that the description includes instances where the event or circumstance occurs and instances where it does not.

As used herein, by a "subject" is meant an individual. Thus, the "subject" can include domesticated animals (*e.g.,* cats, dogs, etc.), livestock (*e.g.,* cattle, horses, pigs, sheep, goats, etc.), laboratory animals (*e.g*., mouse, rabbit, rat, guinea pig, etc.), and birds. "Subject" can also include a mammal, such as a primate or a human.

### References

Abdelmohsen, K., R. Pullmann, Jr., A. Lal, H. H. Kim, S. Galban, X. Yang, J. D. Blethrow, M. Walker, J. Shubert, D. A. Gillespie, H. Furneaux and M. Gorospe (2007). "Phosphorylation of HuR by Chk2 regulates SIRT1 expression." Mol Cell 25(4): 543-557.
Adebanjo, O. A., H. K. Anandatheerthavarada, A. P. Koval, B. S. Moonga, G. Biswas, L. Sun, B. R. Sodam, et al. "A New Function for Cd38/Adp-Ribosyl Cyclase in Nuclear Ca2+ Homeostasis." Nat Cell Biol 1, no. 7 (Nov 1999): 409-14.
Albrecht, S. C., A. G. Barata, J. Grosshans, A. A. Teleman and T. P. Dick (2011). "In vivo mapping of hydrogen peroxide and oxidized glutathione reveals chemical and regional specificity of redox homeostasis." Cell Metab 14(6): 819-829.
Alic N, Partridge L. Myc mouse and anti-ageing therapy. Trends Endocrinol Metab. 2015;26(4): 163-4.
Ame, J. C., V. Rolli, V. Schreiber, C. Niedergang, F. Apiou, P. Decker, S. Muller, T. Hoger, J. Menissier-de Murcia and G. de Murcia (1999). "PARP-2, A novel mammalian DNA damage-dependent poly(ADP-ribose) polymerase." J Biol Chem 274(25): 17860-17868.
Arai, Y., C. M. Martin-Ruiz, M. Takayama, Y. Abe, T. Takebayashi, S. Koyasu, M. Suematsu, N. Hirose, and T. von Zglinicki. "Inflammation, but Not Telomere Length, Predicts Successful Ageing at Extreme Old Age: A Longitudinal Study of Semi-Supercentenarians." EBioMedicine 2, no. 10 (Oct 2015): 1549-58.
Armah CN, Traka MH, Dainty JR, Defernez M, Janssens A, Leung W, et al. A diet rich in high-glucoraphanin broccoli interacts with genotype to reduce discordance in plasma metabolite profiles by modulating mitochondrial function. Am J Clin Nutr. 2013;98(3):712-22.
Astrakhantseva IV, Efimov GA, Drutskaya MS, Kruglov AA, Nedospasov SA. Modern anti-cytokine therapy of autoimmune diseases. Biochemistry (Mosc). 2014;79(12):1308-21.
Bai P, Canto C, Oudart H, Brunyanszki A, Cen Y, Thomas C, et al. PARP-1 inhibition increases mitochondrial metabolism through SIRT1 activation. Cell Metab. 2011;13(4):461-8.
Bai, P. and C. Canto (2012). "The role of PARP-1 and PARP-2 enzymes in metabolic regulation and disease." Cell Metab 16(3): 290-295.
Balogun, E., M. Hoque, P. Gong, E. Killeen, C. J. Green, R. Foresti, J. Alam and R. Motterlini (2003). "Curcumin activates the haem oxygenase-1 gene via regulation of Nrf2 and the antioxidant-responsive element." Biochem J 371(Pt 3): 887-895.
Barata H, Thompson M, Zielinska W, Han YS, Mantilla CB, Prakash YS, et al. The role of cyclic-ADP-ribose-signaling pathway in oxytocin-induced Ca2+ transients in human myometrium cells. Endocrinology. 2004;145(2):881-9.
Barber MF, Michishita-Kioi E, Xi Y, Tasselli L, Kioi M, Moqtaderi Z, et al. SIRT7 links H3K18 deacetylation to maintenance of oncogenic transformation. Nature. 2012;487(7405): 114-8.
Barr, L. A. and J. W. Calvert (2014). "Discoveries of Hydrogen Sulfide as a Novel Cardiovascular Therapeutic." Circulation Journal 78(9): 2111-2118.
Bellizzi D, Rose G, Cavalcante P, Covello G, Dato S, De Rango F, et al. A novel VNTR enhancer within the SIRT3 gene, a human homologue of SIR2, is associated with survival at oldest ages. Genomics. 2005;85(2):258-63.
Belsky, D. W., A. Caspi, R. Houts, H. J. Cohen, D. L. Corcoran, A. Danese, H. Harrington, S. Israel, M. E. Levine, J. D. Schaefer, K. Sugden, B. Williams, A. I. Yashin, R. Poulton and T. E. Moffitt (2015). "Quantification of biological aging in young adults." Proc Natl Acad Sci U S A 112(30): E4104-4110.
Ben Mosbah, I., Y. Mouchel, J. Pajaud, C. Ribault, C. Lucas, A. Laurent, K. Boudjema, F. Morel, A. Corlu and P. Compagnon (2012). "Pretreatment with mangafodipir improves liver graft tolerance to ischemia/reperfusion injury in rat." PLoS One 7(11): e50235.
Bettcher BM, Kramer JH. Longitudinal inflammation, cognitive decline, and Alzheimer's disease: a mini-review. Clin Pharmacol Ther. 2014;96(4):464-9.
Bettcher BM, Watson CL, Walsh CM, Lobach IV, Neuhaus J, Miller JW, et al. Interleukin-6, age, and corpus callosum integrity. PLoS One. 2014;9(9):e106521.
Bettcher BM, Yaffe K, Boudreau RM, Neuhaus J, Aizenstein H, Ding J, et al. Declines in inflammation predict greater white matter microstructure in older adults. Neurobiol Aging. 2015;36(2):948-54.
Bocklandt, S., W. Lin, M. E. Sehl, F. J. Sanchez, J. S. Sinsheimer, S. Horvath and E. Vilain (2011). "Epigenetic predictor of age." PLoS One 6(6): e14821.
Bos, E. M., R. Wang, P. M. Snijder, M. Boersema, J. Damman, M. Fu, J. Moser, J. L. Hillebrands, R. J. Ploeg, G. Yang, H. G. Leuvenink and H. van Goor (2013). "Cystathionine gamma-lyase protects against renal ischemia/reperfusion by modulating oxidative stress." J Am Soc Nephrol 24(5): 759-770.
Braidy, N., G. J. Guillemin and R. Grant (2011). "Effects of Kynurenine Pathway Inhibition on NAD Metabolism and Cell Viability in Human Primary Astrocytes and Neurons." Int J Tryptophan Res 4: 29-37.
Braidy, N., G. J. Guillemin, H. Mansour, T. Chan-Ling, A. Poljak and R. Grant (2011). "Age related changes in NAD+ metabolism oxidative stress and Sirt1 activity in wistar rats." PLoS One 6(4): e19194.
Butchart, J., L. Brook, V. Hopkins, J. Teeling, U. Puntener, D. Culliford, R. Sharples, et al. "Etanercept in Alzheimer Disease: A Randomized, Placebo-Controlled, Double-Blind, Phase 2 Trial." Neurology 84, no. 21 (May 26 2015): 2161-8.
Calvert, J. W., M. Elston, C. K. Nicholson, S. Gundewar, S. Jha, J. W. Elrod, A. Ramachandran and D. J. Lefer (2010). "Genetic and pharmacologic hydrogen sulfide therapy attenuates ischemia-induced heart failure in mice." Circulation 122(1): 11-19.
Calvert, J. W., S. Jha, S. Gundewar, J. W. Elrod, A. Ramachandran, C. B. Pattillo, C. G. Kevil and D. J. Lefer (2009). "Hydrogen sulfide mediates cardioprotection through Nrf2 signaling." Circ Res 105(4): 365-374.
Canto, C., A. A. Sauve and P. Bai (2013). "Crosstalk between poly(ADP-ribose) polymerase and sirtuin enzymes." Mol Aspects Med 34(6): 1168-1201.
Cao Z, Tsang M, Zhao H, Li Y. Induction of endogenous antioxidants and phase 2 enzymes by α-lipoic acid in rat cardiac H9C2 cells: protection against oxidative injury. Biochemical and Biophysical Research Communications. 2003;310(3):979-85.
Cardus A, Uryga AK, Walters G, Erusalimsky JD. SIRT6 protects human endothelial cells from DNA damage, telomere dysfunction, and senescence. Cardiovasc Res. 2013;97(3):571-9.
Cesari, M., B. W. Penninx, M. Pahor, F. Lauretani, A. M. Corsi, G. Rhys Williams, J. M. Guralnik, and L. Ferrucci. "Inflammatory Markers and Physical Performance in Older Persons: The Inchianti Study." J Gerontol A Biol Sci Med Sci 59, no. 3 (Mar 2004): 242-8.
Chaix A, Zarrinpar A, Miu P, Panda S. Time-restricted feeding is a preventative and therapeutic intervention against diverse nutritional challenges. Cell Metab. 2014;20(6):991-1005.
Chen S, Seiler J, Santiago-Reichelt M, Felbel K, Grummt I, Voit R. Repression of RNA polymerase I upon stress is caused by inhibition of RNA-dependent deacetylation of PAF53 by SIRT7. Mol Cell. 2013;52(3):303-13.
Chen, D., J. Bruno, E. Easlon, S. J. Lin, H. L. Cheng, F. W. Alt and L. Guarente (2008). "Tissue-specific regulation of SIRT1 by calorie restriction." Genes Dev 22(13): 1753-1757.
Chen, Q., A. K. Camara, D. F. Stowe, C. L. Hoppel and E. J. Lesnefsky (2007). "Modulation of electron transport protects cardiac mitochondria and decreases myocardial injury during ischemia and reperfusion." Am J Physiol Cell Physiol 292(1): C137-147.
Cheng L. Resveratrol attenuates inflammation and oxidative stress induced by myocardialischemia-reperfusion injury: role of Nrf2/ARE pathway. 2015.
Cheng, Y., J. F. Ndisang, G. Tang, K. Cao and R. Wang (2004). "Hydrogen sulfide-induced relaxation of resistance mesenteric artery beds of rats." Am J Physiol Heart Circ Physiol 287(5): H2316-2323.
Cho EH. SIRT3 as a Regulator of Non-alcoholic Fatty Liver Disease. J Lifestyle Med. 2014;4(2):80-5.
Chouliaras, L., D. L. van den Hove, G. Kenis, S. Keitel, P. R. Hof, J. van Os, H. W. Steinbusch, C. Schmitz and B. P. Rutten (2012). "Prevention of age-related changes in hippocampal levels of 5-methylcytidine by caloric restriction." Neurobiol Aging 33(8): 1672-1681.
Coletta, C., A. Papapetropoulos, K. Erdelyi, G. Olah, K. Modis, P. Panopoulos, A. Asimakopoulou, D. Gero, I. Sharina, E. Martin and C. Szabo (2012). "Hydrogen sulfide and nitric oxide are mutually dependent in the regulation of angiogenesis and endothelium-dependent vasorelaxation." Proc Natl Acad Sci U S A 109(23): 9161-9166.
Colman, R. J., T. M. Beasley, J. W. Kemnitz, S. C. Johnson, R. Weindruch and R. M. Anderson (2014). "Caloric restriction reduces age-related and all-cause mortality in rhesus monkeys." Nat Commun 5: 3557.
Cremers, C. M., and U. Jakob. "Oxidant Sensing by Reversible Disulfide Bond Formation." J Biol Chem 288, no. 37 (Sep 13 2013): 26489-96.
De Haes, W., L. Frooninckx, R. Van Assche, A. Smolders, G. Depuydt, J. Billen, B. P. Braeckman, L. Schoofs and L. Temmerman (2014). "Metformin promotes lifespan through mitohormesis via the peroxiredoxin PRDX-2." Proc Natl Acad Sci U S A 111(24): E2501-2509.
Deaglio S, Vaisitti T, Billington R, Bergui L, Omede P, Genazzani AA, et al. CD38/CD19: a lipid raft-dependent signaling complex in human B cells. Blood. 2007;109(12):5390-8.
Derhovanessian, E., A. B. Maier, R. Beck, G. Jahn, K. Hahnel, P. E. Slagboom, A. J. de Craen, R. G. Westendorp and G. Pawelec (2010). "Hallmark features of immunosenescence are absent in familial longevity." J Immunol 185(8): 4618-4624.
Dinkova-Kostova AT, Abramov AY. The emerging role of Nrf2 in mitochondrial function. Free Radic Biol Med. 2015;88(Pt B):179-88.
Dinkova-Kostova, A. T., K. T. Liby, K. K. Stephenson, W. D. Holtzclaw, X. Gao, N. Suh, C. Williams, R. Risingsong, T. Honda, G. W. Gribble, M. B. Sporn and P. Talalay (2005). "Extremely potent triterpenoid inducers of the phase 2 response: correlations of protection against oxidant and inflammatory stress." Proc Natl Acad Sci U S A 102(12): 4584-4589.
Dipp, M., and A. M. Evans. "Cyclic Adp-Ribose Is the Primary Trigger for Hypoxic Pulmonary Vasoconstriction in the Rat Lung in Situ." Circ Res 89, no. 1 (Jul 6 2001): 77-83.
Dodson M, Redmann M, Rajasekaran NS, Darley-Usmar V, Zhang J. KEAP1-NRF2 signalling and autophagy in protection against oxidative and reductive proteotoxicity. Biochem J. 2015;469(3):347-55.
Du J, Zhou Y, Su X, Yu JJ, Khan S, Jiang H, et al. Sirt5 is a NAD-dependent protein lysine demalonylase and desuccinylase. Science. 2011;334(6057):806-9.
Escande, C., V. Nin, N. L. Price, V. Capellini, A. P. Gomes, M. T. Barbosa, L. O'Neil, et al. "Flavonoid Apigenin Is an Inhibitor of the Nad+ Ase Cd38: Implications for Cellular Nad+ Metabolism, Protein Acetylation, and Treatment of Metabolic Syndrome." Diabetes 62, no. 4 (Apr 2013): 1084-93.
Fernandez, A. F., et al. (2015). "H3K4me1 marks DNA regions hypomethylated during aging in human stem and differentiated cells." Genome Res 25(1): 27-40.
Ferrero, E., and F. Malavasi. "The Metamorphosis of a Molecule: From Soluble Enzyme to the Leukocyte Receptor Cd38." J Leukoc Biol 65, no. 2 (Feb 1999): 151-61.
Fifel K. Sirtuin 3: a molecular pathway linking sleep deprivation to neurological diseases. J Neurosci. 2014;34(28):9179-81.
Fiorucci S, Distrutti E, Cirino G, Wallace JL. The emerging roles of hydrogen sulfide in the gastrointestinal tract and liver. Gastroenterology. 2006;131(1):259-71.
Flier J, Van Muiswinkel FL, Jongenelen CA, Drukarch B. The neuroprotective antioxidant alpha-lipoic acid induces detoxication enzymes in cultured astroglial cells. Free Radic Res. 2002;36(6):695-9.
Ford E, Voit R, Liszt G, Magin C, Grummt I, Guarente L. Mammalian Sir2 homolog SIRT7 is an activator of RNA polymerase I transcription. Genes Dev. 2006;20(9):1075-80.
Ford, J., S. Ahmed, S. Allison, M. Jiang and J. Milner (2014). "JNK2-dependent regulation of SIRT1 protein stability." Cell Cycle 7(19): 3091-3097.
Forman, H. J., J. M. Fukuto, T. Miller, H. Zhang, A. Rinna and S. Levy (2008). "The chemistry of cell signaling by reactive oxygen and nitrogen species and 4-hydroxynonenal." Arch Biochem Biophys 477(2): 183-195.
Franceschi C, Campisi J. Chronic inflammation (inflammaging) and its potential contribution to age-associated diseases. J Gerontol A Biol Sci Med Sci. 2014;69 Suppl 1:S4-9.
Franceschi C, Capri M, Monti D, Giunta S, Olivieri F, Sevini F, et al. Inflammaging and anti-inflammaging: a systemic perspective on aging and longevity emerged from studies in humans. Mech Ageing Dev. 2007;128(1):92-105.
Franceschi C. Inflammaging as a Major Characteristic of Old People: Can It Be Prevented or Cured? Nutrition Reviews. 2007;65(12):173-6.
Fu, M., W. Zhang, L. Wu, G. Yang, H. Li and R. Wang (2012). "Hydrogen sulfide (H2S) metabolism in mitochondria and its regulatory role in energy production." Proc Natl Acad Sci U S A 109(8): 2943-2948.
Fusco S, Leone L, Barbati SA, Samengo D, Piacentini R, Maulucci G, et al. A CREB-Sirt1-Hes1 Circuitry Mediates Neural Stem Cell Response to Glucose Availability. Cell Rep. 2016;14(5):1195-205.
Ge Y, Jiang W, Gan L, Wang L, Sun C, Ni P, et al. Mouse embryonic fibroblasts from CD38 knockout mice are resistant to oxidative stresses through inhibition of reactive oxygen species production and Ca(2+) overload. Biochem Biophys Res Commun. 2010;399(2):167-72.
Ge, S. N., M. M. Zhao, D. D. Wu, Y. Chen, Y. Wang, J. H. Zhu, W. J. Cai, Y. Z. Zhu and Y. C. Zhu (2014). "Hydrogen sulfide targets EGFR Cys797/Cys798 residues to induce Na(+)/K(+)-ATPase endocytosis and inhibition in renal tubular epithelial cells and increase sodium excretion in chronic salt-loaded rats." Antioxid Redox Signal 21(15): 2061-2082.
Gegotek A, Skrzydlewska E. The role of transcription factor Nrf2 in skin cells metabolism. Arch Dermatol Res. 2015;307(5):385-96.
Geng, B., H. Yan, G. Z. Zhong, C. Y. Zhang, X. B. Chen, H. F. Jiang, C. S. Tang and J. B. Du (2004). "[Hydrogen sulfide: a novel cardiovascular functional regulatory gas factor]." Beijing Da Xue Xue Bao 36(1): 106.
Gerhart-Hines Z, Dominy JE, Jr., Blattler SM, Jedrychowski MP, Banks AS, Lim JH, et al. The cAMP/PKA pathway rapidly activates SIRT1 to promote fatty acid oxidation independently of changes in NAD(+). Mol Cell. 2011;44(6):851-63.
Giralt A, Villarroya F. SIRT3, a pivotal actor in mitochondrial functions: metabolism, cell death and aging. Biochem J. 2012;444(1):1-10.
Givvimani, S., C. Munjal, R. Gargoum, U. Sen, N. Tyagi, J. C. Vacek and S. C. Tyagi (2011). "Hydrogen sulfide mitigates transition from compensatory hypertrophy to heart failure." J Appl Physiol (1985) 110(4): 1093-1100.
Goel, A., D. R. Spitz and G. J. Weiner (2012). "Manipulation of cellular redox parameters for improving therapeutic responses in B-cell lymphoma and multiple myeloma." J Cell Biochem 113(2): 419-425.
Gomes P, Outeiro TF, Cavadas C. Emerging Role of Sirtuin 2 in the Regulation of Mammalian Metabolism. Trends Pharmacol Sci. 2015;36(11):756-68.
Gomes, A. P., N. L. Price, A. J. Ling, J. J. Moslehi, M. K. Montgomery, L. Rajman, J. P. White, J. S. Teodoro, C. D. Wrann, B. P. Hubbard, E. M. Mercken, C. M. Palmeira, R. de Cabo, A. P. Rolo, N. Turner, E. L. Bell and D. A. Sinclair (2013). "Declining NAD(+) induces a pseudohypoxic state disrupting nuclear-mitochondrial communication during aging." Cell 155(7): 1624-1638.
Goodson NJ, Symmons DP, Scott DG, Bunn D, Lunt M, Silman AJ. Baseline levels of C-reactive protein and prediction of death from cardiovascular disease in patients with inflammatory polyarthritis: a ten-year follow up study of a primary care-based inception cohort. Arthritis Rheum. 2005;52(8):2293-9.
Grob A, Roussel P, Wright JE, McStay B, Hernandez-Verdun D, Sirri V. Involvement of SIRT7 in resumption of rDNA transcription at the exit from mitosis. J Cell Sci. 2009;122(Pt 4):489-98.
Grozio A, Sociali G, Sturla L, Caffa I, Soncini D, Salis A, et al. CD73 protein as a source of extracellular precursors for sustained NAD+ biosynthesis in FK866-treated tumor cells. J Biol Chem. 2013;288(36):25938-49.
Guarente L. The many faces of sirtuins: Sirtuins and the Warburg effect. Nat Med. 2014;20(1):24-5.
Guarente, L. (2000). "Sir2 links chromatin silencing, metabolism, and aging." Genes Dev 14(9): 1021-1026.
Haigis MC, Mostoslavsky R, Haigis KM, Fahie K, Christodoulou DC, Murphy AJ, et al. SIRT4 inhibits glutamate dehydrogenase and opposes the effects of calorie restriction in pancreatic beta cells. Cell. 2006;126(5):941-54.
Halaschek-Wiener J, Amirabbasi-Beik M, Monfared N, Pieczyk M, Sailer C, Kollar A, et al. Genetic variation in healthy oldest-old. PLoS One. 2009;4(8):e6641.
Han L, Ge J, Zhang L, Ma R, Hou X, Li B, et al. Sirt6 depletion causes spindle defects and chromosome misalignment during meiosis of mouse oocyte. Sci Rep. 2015;5:15366.
Harman, D. "Aging: A Theory Based on Free Radical and Radiation Chemistry." J Gerontol 11, no. 3 (Jul 1956): 298-300.
He, C. and D. J. Klionsky (2009). "Regulation mechanisms and signaling pathways of autophagy." Annu Rev Genet 43: 67-93.
He, H. J., G. Y. Wang, Y. Gao, W. H. Ling, Z. W. Yu and T. R. Jin (2012). "Curcumin attenuates Nrf2 signaling defect, oxidative stress in muscle and glucose intolerance in high fat diet-fed mice." World J Diabetes 3(5): 94-104.
Hill, B. C., T. C. Woon, P. Nicholls, J. Peterson, C. Greenwood and A. J. Thomson (1984). "Interactions of sulphide and other ligands with cytochrome c oxidase. An electron-paramagnetic-resonance study." Biochem J 224(2): 591-600.
Hinson, J. A., D. W. Roberts and L. P. James (2010). "Mechanisms of acetaminophen-induced liver necrosis." Handb Exp Pharmacol(196): 369-405.
Hirschey MD, Shimazu T, Goetzman E, Jing E, Schwer B, Lombard DB, et al. SIRT3 regulates mitochondrial fatty-acid oxidation by reversible enzyme deacetylation. Nature. 2010;464(7285):121-5.
Hirschey MD, Shimazu T, Huang JY, Schwer B, Verdin E. SIRT3 regulates mitochondrial protein acetylation and intermediary metabolism. Cold Spring Harb Symp Quant Biol. 2011;76:267-77.
Hoffman-Liebermann, B., and D. A. Liebermann. "Interleukin-6- and Leukemia Inhibitory Factor-Induced Terminal Differentiation of Myeloid Leukemia Cells Is Blocked at an Intermediate Stage by Constitutive C-Myc." Mol Cell Biol 11, no. 5 (May 1991): 2375-81.
Hokari F, Kawasaki E, Sakai A, Koshinaka K, Sakuma K, Kawanaka K. Muscle contractile activity regulates Sirt3 protein expression in rat skeletal muscles. J Appl Physiol (1985). 2010;109(2):332-40.
Holmes C, Butchart J. Systemic inflammation and Alzheimer's disease. Biochem Soc Trans. 2011;39(4):898-901.
Horvath, S. (2013). "DNA methylation age of human tissues and cell types." Genome Biol 14(10): R115.
Horvath, S. (2015). "Erratum to: DNA methylation age of human tissues and cell types." Genome Biol 16: 96.
Horvath, S., V. Mah, A. T. Lu, J. S. Woo, O. W. Choi, A. J. Jasinska, J. A. Riancho, S. Tung, N. S. Coles, J. Braun, H. V. Vinters and L. S. Coles (2015). "The cerebellum ages slowly according to the epigenetic clock." Aging (Albany NY) 7(5): 294-306.
Huang Y, Li W, Su ZY, Kong AN. The complexity of the Nrf2 pathway: beyond the antioxidant response. J Nutr Biochem. 2015;26(12):1401-13.
Hussain, A. M., H. C. Lee, and C. F. Chang. "Functional Expression of Secreted Mouse Bst-1 in Yeast." Protein Expr Purif 12, no. 1 (Feb 1998): 133-7.
Imai, S. (2009). "SIRT1 and caloric restriction: an insight into possible trade-offs between robustness and frailty." Curr Opin Clin Nutr Metab Care 12(4): 350-356.
Insko, M. A., T. L. Deckwerth, P. Hill, C. F. Toombs and C. Szabo (2009). "Detection of exhaled hydrogen sulphide gas in rats exposed to intravenous sodium sulphide." Br J Pharmacol 157(6): 944-951.
Ise F, Takasuka H, Hayashi S, Takahashi K, Koyama M, Aihara E, et al. Stimulation of duodenal HCO(3)(-) secretion by hydrogen sulphide in rats: relation to prostaglandins, nitric oxide and sensory neurones. Acta Physiol (Oxf). 2011;201(1): 117-26.
Iyer SS, He Q, Janczy JR, Elliott EI, Zhong Z, Olivier AK, et al. Mitochondrial cardiolipin is required for Nlrp3 inflammasome activation. Immunity. 2013;39(2):311-23.
Jackson, M. J. (2011). "Control of reactive oxygen species production in contracting skeletal muscle." Antioxid Redox Signal 15(9): 2477-2486.
Jain, S. K., J. Rains, J. Croad, B. Larson and K. Jones (2009). "Curcumin supplementation lowers TNF-alpha, IL-6, IL-8, and MCP-1 secretion in high glucose-treated cultured monocytes and blood levels of TNF-alpha, IL-6, MCP-1, glucose, and glycosylated hemoglobin in diabetic rats." Antioxid Redox Signal 11(2): 241-249.
Jiang, H. L., H. C. Wu, Z. L. Li, B. Geng and C. S. Tang (2005). "[Changes of the new gaseous transmitter H2S in patients with coronary heart disease]." Di Yi Jun Yi Da Xue Xue Bao 25(8): 951-954.
Jin, H. F., J. B. Du, X. H. Li, Y. F. Wang, Y. F. Liang and C. S. Tang (2006). "Interaction between hydrogen sulfide/cystathionine gamma-lyase and carbon monoxide/heme oxygenase pathways in aortic smooth muscle cells." Acta Pharmacol Sin 27(12): 1561-1566.
Jodar, L., E. M. Mercken, J. Ariza, C. Younts, J. A. Gonzalez-Reyes, F. J. Alcain, I. Buron, R. de Cabo and J. M. Villalba (2011). "Genetic deletion of Nrf2 promotes immortalization and decreases life span of murine embryonic fibroblasts." J Gerontol A Biol Sci Med Sci 66(3): 247-256.
Jung, K. J., A. Dasgupta, K. Huang, S. J. Jeong, C. Pise-Masison, K. V. Gurova and J. N. Brady (2008). "Small-molecule inhibitor which reactivates p53 in human T-cell leukemia virus type 1-transformed cells." J Virol 82(17): 8537-8547.
Jung, S. B., C. S. Kim, Y. R. Kim, A. Naqvi, T. Yamamori, S. Kumar, A. Kumar and K. Irani (2013). "Redox factor-1 activates endothelial SIRTUIN1 through reduction of conserved cysteine sulfhydryls in its deacetylase domain." PLoS One 8(6): e65415.
Kanfi Y, Naiman S, Amir G, Peshti V, Zinman G, Nahum L, et al. The sirtuin SIRT6 regulates lifespan in male mice. Nature. 2012;483(7388):218-21.
Kang, B. Y., S. Kim, K. H. Lee, Y. S. Lee, I. Hong, M. O. Lee, D. Min, et al. "Transcriptional Profiling in Human Hacat Keratinocytes in Response to Kaempferol and Identification of Potential Transcription Factors for Regulating Differential Gene Expression." Exp Mol Med 40, no. 2 (Apr 30 2008): 208-19.
Kaspar, J. W., S. K. Niture and A. K. Jaiswal (2012). "Antioxidant-induced INrf2 (Keap1) tyrosine 85 phosphorylation controls the nuclear export and degradation of the INrf2-Cul3-Rbx1 complex to allow normal Nrf2 activation and repression." J Cell Sci 125(Pt 4): 1027-1038.
Kasparek MS, Linden DR, Kreis ME, Sarr MG. Gasotransmitters in the gastrointestinal tract. Surgery. 2008;143(4):455-9.
Kellenberger E, Kuhn I, Schuber F, Muller-Steffner H. Flavonoids as inhibitors of human CD38. Bioorg Med Chem Lett. 2011;21(13):3939-42.
Kennedy, S. R., L. A. Loeb and A. J. Herr (2012). "Somatic mutations in aging, cancer and neurodegeneration." Mech Ageing Dev 133(4): 118-126.
Kida, K., E. Marutani, R. K. Nguyen and F. Ichinose (2015). "Inhaled hydrogen sulfide prevents neuropathic pain after peripheral nerve injury in mice." Nitric Oxide 46: 87-92.
Kida, K., M. Yamada, K. Tokuda, E. Marutani, M. Kakinohana, M. Kaneki and F. Ichinose (2011). "Inhaled hydrogen sulfide prevents neurodegeneration and movement disorder in a mouse model of Parkinson's disease." Antioxid Redox Signal 15(2): 343-352.
Kim HS, Vassilopoulos A, Wang RH, Lahusen T, Xiao Z, Xu X, et al. SIRT2 maintains genome integrity and suppresses tumorigenesis through regulating APC/C activity. Cancer Cell. 2011;20(4):487-99.
Kim SC, Sprung R, Chen Y, Xu Y, Ball H, Pei J, et al. Substrate and functional diversity of lysine acetylation revealed by a proteomics survey. Mol Cell. 2006;23(4):607-18.
Kimura S, Warabi E, Yanagawa T, Ma D, Itoh K, Ishii Y, et al. Essential role of Nrf2 in keratinocyte protection from UVA by quercetin. Biochem Biophys Res Commun. 2009;387(1):109-14.
Kimura, Y. and H. Kimura (2004). "Hydrogen sulfide protects neurons from oxidative stress." FASEB J 18(10): 1165-1167.
Kincaid B, Bossy-Wetzel E. Forever young: SIRT3 a shield against mitochondrial meltdown, aging, and neurodegeneration. Front Aging Neurosci. 2013;5:48.
Kirkwood, T. B. and M. R. Rose (1991). "Evolution of senescence: late survival sacrificed for reproduction." Philos Trans R Soc Lond B Biol Sci 332(1262): 15-24.
Kirkwood, T. B. (2005). "Understanding the odd science of aging." Cell 120(4): 437-447.
Kirkwood, T. L., P. Kapahi and D. P. Shanley (2000). "Evolution, stress, and longevity." J Anat 197 Pt 4: 587-590.
Kitada, M., S. Kume, N. Imaizumi and D. Koya (2011). "Resveratrol improves oxidative stress and protects against diabetic nephropathy through normalization of Mn-SOD dysfunction in AMPK/SIRT1-independent pathway." Diabetes 60(2): 634-643.
Kondo, K., S. Bhushan, A. L. King, S. D. Prabhu, T. Hamid, S. Koenig, T. Murohara, B. L. Predmore, G. Gojon, Sr., G. Gojon, Jr., R. Wang, N. Karusula, C. K. Nicholson, J. W. Calvert and D. J. Lefer (2013). "H(2)S protects against pressure overload-induced heart failure via upregulation of endothelial nitric oxide synthase." Circulation 127(10): 1116-1127.
Kong X, Wang R, Xue Y, Liu X, Zhang H, Chen Y, et al. Sirtuin 3, a new target of PGC-1alpha, plays an important role in the suppression of ROS and mitochondrial biogenesis. PLoS One. 2010;5(7):e11707.
Kugel S, Mostoslavsky R. Chromatin and beyond: the multitasking roles for SIRT6. Trends Biochem Sci. 2014;39(2):72-81.
Kumar, S. and A. K. Pandey (2013). "Chemistry and biological activities of flavonoids: an overview." ScientificWorldJournal 2013: 162750.
Kumasaka, S., et al. (1999). "Novel mechanisms involved in superoxide anion radical-triggered Ca2+ release from cardiac sarcoplasmic reticulum linked to cyclic ADP-ribose stimulation." Antioxid Redox Signal 1(1): 55-69.
Labbadia, J. and R. I. Morimoto (2015). "The biology of proteostasis in aging and disease." Annu Rev Biochem 84: 435-464.
Lagouge, M., C. Argmann, Z. Gerhart-Hines, H. Meziane, C. Lerin, F. Daussin, N. Messadeq, J. Milne, P. Lambert, P. Elliott, B. Geny, M. Laakso, P. Puigserver and J. Auwerx (2006). "Resveratrol improves mitochondrial function and protects against metabolic disease by activating SIRT1 and PGC-1alpha." Cell 127(6): 1109-1122.
Landis, G. N., M. P. Salomon, D. Keroles, N. Brookes, T. Sekimura and J. Tower (2015). "The progesterone antagonist mifepristone/RU486 blocks the negative effect on life span caused by mating in female Drosophila." Aging (Albany NY) 7(1): 53-69.
Laurent G, German NJ, Saha AK, de Boer VC, Davies M, Koves TR, et al. SIRT4 coordinates the balance between lipid synthesis and catabolism by repressing malonyl CoA decarboxylase. Mol Cell. 2013;50(5):686-98.
Lee BT, Ahmed FA, Hamm LL, Teran FJ, Chen CS, Liu Y, et al. Association of C-reactive protein, tumor necrosis factor-alpha, and interleukin-6 with chronic kidney disease. BMC Nephrol. 2015;16:77.
Lee CU, Song EK, Yoo CH, Kwak YK, Han MK. Lipopolysaccharide induces CD38 expression and solubilization in J774 macrophage cells. Mol Cells. 2012;34(6):573-6.
Lee JK, Bettencourt R, Brenner D, Le TA, Barrett-Connor E, Loomba R. Association between serum interleukin-6 concentrations and mortality in older adults: the Rancho Bernardo study. PLoS One. 2012;7(4):e34218.
Lee S, Paudel O, Jiang Y, Yang XR, Sham JS. CD38 mediates angiotensin II-induced intracellular Ca(2+) release in rat pulmonary arterial smooth muscle cells. Am J Respir Cell Mol Biol. 2015;52(3):332-41.
Lee, Z. W., J. Zhou, C. S. Chen, Y. Zhao, C. H. Tan, L. Li, P. K. Moore and L. W. Deng (2011). "The slow-releasing hydrogen sulfide donor, GYY4137, exhibits novel anticancer effects in vitro and in vivo." PLoS One 6(6): e21077.
Leruez S, Milea D, Defoort-Dhellemmes S, Colin E, Crochet M, Procaccio V, et al. Sensorineural hearing loss in OPA1-linked disorders. Brain. 2013;136(Pt 7):e236.
Li, L., M. Whiteman, Y. Y. Guan, K. L. Neo, Y. Cheng, S. W. Lee, Y. Zhao, R. Baskar, C. H. Tan and P. K. Moore (2008). "Characterization of a novel, water-soluble hydrogen sulfide-releasing molecule (GYY4137): new insights into the biology of hydrogen sulfide." Circulation 117(18): 2351-2360.
Liu GH, Qu J, Shen X. NF-kappaB/p65 antagonizes Nrf2-ARE pathway by depriving CBP from Nrf2 and facilitating recruitment of HDAC3 to MafK. Biochim Biophys Acta. 2008;1783(5):713-27.
Liu R, Dang W, Du Y, Zhou Q, Jiao K, Liu Z. SIRT2 is involved in the modulation of depressive behaviors. Sci Rep. 2015;5:8415.
Liu R, Liu H, Ha Y, Tilton RG, Zhang W. Oxidative stress induces endothelial cell senescence via downregulation of Sirt6. Biomed Res Int. 2014;2014:902842.
Lombard DB, Alt FW, Cheng HL, Bunkenborg J, Streeper RS, Mostoslavsky R, et al. Mammalian Sir2 homolog SIRT3 regulates global mitochondrial lysine acetylation. Mol Cell Biol. 2007;27(24):8807-14.
Long AN, Owens K, Schlappal AE, Kristian T, Fishman PS, Schuh RA. Effect of nicotinamide mononucleotide on brain mitochondrial respiratory deficits in an Alzheimer's disease-relevant murine model. BMC Neurol. 2015;15:19.
Lupoli, R., A. Di Minno, G. Spadarella, M. Franchini, R. Sorrentino, G. Cirino and G. Di Minno (2015). "Methylation reactions, the redox balance and atherothrombosis: the search for a link with hydrogen sulfide." Semin Thromb Hemost 41(4): 423-432.
Magesh, S., Y. Chen and L. Hu (2012). "Small molecule modulators of Keap1-Nrf2-ARE pathway as potential preventive and therapeutic agents." Med Res Rev 32(4): 687-726.
Marinho, H. S., C. Real, L. Cyrne, H. Soares and F. Antunes (2014). "Hydrogen peroxide sensing, signaling and regulation of transcription factors." Redox Biol 2: 535-562.
Marioni, R. E., et al. (2015). "DNA methylation age of blood predicts all-cause mortality in later life." Genome Biol 16: 25.
Martin GR, McKnight GW, Dicay MS, Coffin CS, Ferraz JG, Wallace JL. Hydrogen sulphide synthesis in the rat and mouse gastrointestinal tract. Dig Liver Dis. 2010;42(2): 103-9.
Massudi, H., R. Grant, N. Braidy, J. Guest, B. Farnsworth and G. J. Guillemin (2012). "Age-associated changes in oxidative stress and NAD+ metabolism in human tissue." PLoS One 7(7): e42357.
Mathias RA, Greco TM, Oberstein A, Budayeva HG, Chakrabarti R, Rowland EA, et al. Sirtuin 4 is a lipoamidase regulating pyruvate dehydrogenase complex activity. Cell. 2014;159(7): 1615-25.
McCay C. The Effect of Retarded Growth Upon The Length of Life Span And Upon The Ultimate Body Size Â. 1935.
McCormack D, McFadden D. A review of pterostilbene antioxidant activity and disease modification. Oxid Med Cell Longev. 2013;2013:575482.
Medzhitov R. Origin and physiological roles of inflammation. Nature. 2008;454(7203):428-35.
Merino, M. M., C. Rhiner, J. M. Lopez-Gay, D. Buechel, B. Hauert and E. Moreno (2015). "Elimination of unfit cells maintains tissue health and prolongs lifespan." Cell 160(3): 461-476.
Metes-Kosik, N., I. Luptak, P. M. Dibello, D. E. Handy, S. S. Tang, H. Zhi, F. Qin, D. W. Jacobsen, J. Loscalzo and J. Joseph (2012). "Both selenium deficiency and modest selenium supplementation lead to myocardial fibrosis in mice via effects on redox-methylation balance." Mol Nutr Food Res 56(12): 1812-1824.
Michishita E, McCord RA, Berber E, Kioi M, Padilla-Nash H, Damian M, et al. SIRT6 is a histone H3 lysine 9 deacetylase that modulates telomeric chromatin. Nature. 2008;452(7186):492-6.
Minagawa S, Araya J, Numata T, Nojiri S, Hara H, Yumino Y, et al. Accelerated epithelial cell senescence in IPF and the inhibitory role of SIRT6 in TGF-beta-induced senescence of human bronchial epithelial cells. Am J Physiol Lung Cell Mol Physiol. 2011;300(3):L391-401.
Mokni M, Elkahoui S, Limam F, Amri M, Aouani E. Effect of resveratrol on antioxidant enzyme activities in the brain of healthy rat. Neurochem Res. 2007;32(6):981-7.
Mokni, M., S. Hamlaoui, I. Karkouch, M. Amri, L. Marzouki, F. Limam and E. Aouani (2013). "Resveratrol Provides Cardioprotection after Ischemia/reperfusion Injury via Modulation of Antioxidant Enzyme Activities." Iran J Pharm Res 12(4): 867-875.
Moriarty-Craige, S. E., K. N. Ha, P. Sternberg, Jr., M. Lynn, S. Bressler, G. Gensler and D. P. Jones (2007). "Effects of long-term zinc supplementation on plasma thiol metabolites and redox status in patients with age-related macular degeneration." Am J Ophthalmol 143(2): 206-211.
Moscardo A, Valles J, Latorre A, Jover R, Santos MT. The histone deacetylase sirtuin 2 is a new player in the regulation of platelet function. J Thromb Haemost. 2015;13(7):1335-44.
Mustafa, A. K., G. Sikka, S. K. Gazi, J. Steppan, S. M. Jung, A. K. Bhunia, V. M. Barodka, F. K. Gazi, R. K. Barrow, R. Wang, L. M. Amzel, D. E. Berkowitz and S. H. Snyder (2011). "Hydrogen sulfide as endothelium-derived hyperpolarizing factor sulfhydrates potassium channels." Circ Res 109(11): 1259-1268.
Nallasamy, P., H. Si, P. V. Babu, D. Pan, Y. Fu, E. A. Brooke, H. Shah, W. Zhen, H. Zhu, D. Liu, Y. Li and Z. Jia (2014). "Sulforaphane reduces vascular inflammation in mice and prevents TNF-alpha-induced monocyte adhesion to primary endothelial cells through interfering with the NF-kappaB pathway." J Nutr Biochem 25(8): 824-833.
Nathan, C. and A. Cunningham-Bussel (2013). "Beyond oxidative stress: an immunologist's guide to reactive oxygen species." Nat Rev Immunol 13(5): 349-361.
Neish AS, Jones RM. Redox signaling mediates symbiosis between the gut microbiota and the intestine. Gut Microbes. 2014;5(2):250-3.
Nie, H., et al. (2014). "SIRT2 plays a key role in both cell cycle regulation and cell survival of BV2 microglia." Int J Physiol Pathophysiol Pharmacol 6(3): 166-171.
Nogueiras R, Habegger KM, Chaudhary N, Finan B, Banks AS, Dietrich MO, et al. Sirtuin 1 and sirtuin 3: physiological modulators of metabolism. Physiol Rev. 2012;92(3):1479-514.
Noriega LG, Feige JN, Canto C, Yamamoto H, Yu J, Herman MA, et al. CREB and ChREBP oppositely regulate SIRT1 expression in response to energy availability. EMBO Rep. 2011;12(10):1069-76.
North BJ, Rosenberg MA, Jeganathan KB, Hafner AV, Michan S, Dai J, et al. SIRT2 induces the checkpoint kinase BubR1 to increase lifespan. EMBO J. 2014;33(13):1438-53.
Okabe, E., et al. (2000). "Calmodulin and cyclic ADP-ribose interaction in Ca2+ signaling related to cardiac sarcoplasmic reticulum: superoxide anion radical-triggered Ca2+ release." Antioxid Redox Signal 2(1): 47-54.
Oliver, A. W., J. C. Ame, S. M. Roe, V. Good, G. de Murcia and L. H. Pearl (2004). "Crystal structure of the catalytic fragment of murine poly(ADP-ribose) polymerase-2." Nucleic Acids Res 32(2): 456-464.
Otto, H., P. A. Reche, F. Bazan, K. Dittmar, F. Haag and F. Koch-Nolte (2005). "In silico characterization of the family of PARP-like poly(ADP-ribosyl)transferases (pARTs)." BMC Genomics 6: 139.
Pae, H. O., G. S. Jeong, S. O. Jeong, H. S. Kim, S. A. Kim, Y. C. Kim, S. J. Yoo, H. D. Kim and H. T. Chung (2007). "Roles of heme oxygenase-1 in curcumin-induced growth inhibition in rat smooth muscle cells." Exp Mol Med 39(3): 267-277.
Palacios, O. M., et al. (2009). "Diet and exercise signals regulate SIRT3 and activate AMPK and PGC-1alpha in skeletal muscle." Aging (Albany NY) 1(9): 771-783.
Pall, M. L. and S. Levine (2015). "Nrf2, a master regulator of detoxification and also antioxidant, anti-inflammatory and other cytoprotective mechanisms, is raised by health promoting factors." Sheng Li Xue Bao 67(1): 1-18.
Pan, L. L., X. H. Liu, Q. H. Gong, D. Wu and Y. Z. Zhu (2011). "Hydrogen sulfide attenuated tumor necrosis factor-alpha-induced inflammatory signaling and dysfunction in vascular endothelial cells." PLoS One 6(5): e19766.
Papapetropoulos, A., A. Pyriochou, Z. Altaany, G. Yang, A. Marazioti, Z. Zhou, M. G. Jeschke, L. K. Branski, D. N. Herndon, R. Wang and C. Szabo (2009). "Hydrogen sulfide is an endogenous stimulator of angiogenesis." Proc Natl Acad Sci U S A 106(51): 21972-21977.
Paredes-Gonzalez X, Fuentes F, Jeffery S, Saw CL, Shu L, Su ZY, et al. Induction of NRF2-mediated gene expression by dietary phytochemical flavones apigenin and luteolin. Biopharm Drug Dispos. 2015;36(7):440-51.
Paredes-Gonzalez X, Fuentes F, Su ZY, Kong AN. Apigenin reactivates Nrf2 antioxidative stress signaling in mouse skin epidermal JB6 P + cells through epigenetics modifications. AAPS J. 2014;16(4):727-35.
Paz JC, Park S, Phillips N, Matsumura S, Tsai WW, Kasper L, et al. Combinatorial regulation of a signal-dependent activator by phosphorylation and acetylation. Proc Natl Acad Sci USA. 2014;111(48):17116-21.
Peake, B. F., C. K. Nicholson, J. P. Lambert, R. L. Hood, H. Amin, S. Amin and J. W. Calvert (2013). "Hydrogen sulfide preconditions the db/db diabetic mouse heart against ischemia-reperfusion injury by activating Nrf2 signaling in an Erk-dependent manner." Am J Physiol Heart Circ Physiol 304(9): H1215-1224.
Pearl, R., et al. (1928). "The Form of the Growth Curve of the Canteloup (Cucumis Melo) under Field Conditions." Proc Natl Acad Sci U S A 14(12): 895-901.
Peng, C., et al. (2011). "The first identification of lysine malonylation substrates and its regulatory enzyme." Mol Cell Proteomics 10(12): M111 012658.
Perls, T. T., et al. (1997). "Middle-aged mothers live longer." Nature 389(6647): 133.
Polhemus, D. J. and D. J. Lefer (2014). "Emergence of hydrogen sulfide as an endogenous gaseous signaling molecule in cardiovascular disease." Circ Res 114(4): 730-737.
Pollak N, Niere M, Ziegler M. NAD kinase levels control the NADPH concentration in human cells. J Biol Chem. 2007;282(46):33562-71.
Polletta L, Vernucci E, Carnevale I, Arcangeli T, Rotili D, Palmerio S, et al. SIRT5 regulation of ammonia-induced autophagy and mitophagy. Autophagy. 2015;11(2):253-70.
Powers, S. K. and M. J. Jackson (2008). "Exercise-induced oxidative stress: cellular mechanisms and impact on muscle force production." Physiol Rev 88(4): 1243-1276.
Prasad, G. S., et al. (1996). "Crystal structure of Aplysia ADP ribosyl cyclase, a homologue of the bifunctional ectozyme CD38." Nat Struct Biol 3(11): 957-964.
Predmore, B. L., D. J. Lefer and G. Gojon (2012). "Hydrogen sulfide in biochemistry and medicine." Antioxid Redox Signal 17(1): 119-140.
Predmore, B. L., K. Kondo, S. Bhushan, M. A. Zlatopolsky, A. L. King, J. P. Aragon, D. B. Grinsfelder, M. E. Condit and D. J. Lefer (2012). "The polysulfide diallyl trisulfide protects the ischemic myocardium by preservation of endogenous hydrogen sulfide and increasing nitric oxide bioavailability." Am J Physiol Heart Circ Physiol 302(11): H2410-2418.
Predmore, B. L., M. J. Alendy, K. I. Ahmed, C. Leeuwenburgh and D. Julian (2010). "The hydrogen sulfide signaling system: changes during aging and the benefits of caloric restriction." Age (Dordr) 32(4): 467-481.
Ramsey, K. M. and J. Bass (2009). "Obeying the clock yields benefits for metabolism." Proc Natl Acad Sci U S A 106(11): 4069-4070.
Ramsey, K. M., J. Yoshino, C. S. Brace, D. Abrassart, Y. Kobayashi, B. Marcheva, H. K. Hong, J. L. Chong, E. D. Buhr, C. Lee, J. S. Takahashi, S. Imai and J. Bass (2009). "Circadian clock feedback cycle through NAMPT-mediated NAD+ biosynthesis." Science 324(5927): 651-654.
Ramsey, K. M., K. F. Mills, A. Satoh and S. Imai (2008). "Age-associated loss of Sirt1-mediated enhancement of glucose-stimulated insulin secretion in beta cell-specific Sirt1-overexpressing (BESTO) mice." Aging Cell 7(1): 78-88.
Ravussin, E., L. M. Redman, J. Rochon, S. K. Das, L. Fontana, W. E. Kraus, S. Romashkan, D. A. et al. (2015). "A 2-Year Randomized Controlled Trial of Human Caloric Restriction: Feasibility and Effects on Predictors of Health Span and Longevity." J Gerontol A Biol Sci Med Sci 70(9): 1097-1104.
Reuben, D. B., et al. (2002). "Peripheral blood markers of inflammation predict mortality and functional decline in high-functioning community-dwelling older persons." J Am Geriatr Soc 50(4): 638-644.
Rey, G. and A. B. Reddy (2013). "Protein acetylation links the circadian clock to mitochondrial function." Proc Natl Acad Sci U S A 110(9): 3210-3211.
Reynolds, L. M., J. R. Taylor, J. Ding, K. Lohman, C. Johnson, D. Siscovick, G. Burke, W. Post, S. Shea, D. R. Jacobs, Jr., H. Stunnenberg, S. B. Kritchevsky, I. Hoeschele, C. E. McCall, D. M. Herrington, R. P. Tracy and Y. Liu (2014). "Age-related variations in the methylome associated with gene expression in human monocytes and T cells." Nat Commun 5: 5366.
Rickabaugh, T. M., R. M. Baxter, M. Sehl, J. S. Sinsheimer, P. M. Hultin, L. E. Hultin, A. Quach, O. Martinez-Maza, S. Horvath, E. Vilain and B. D. Jamieson (2015). "Acceleration of age-associated methylation patterns in HIV-1-infected adults." PLoS One 10(3): e0119201.
Ridker, P. M., et al. (2000). "Elevation of tumor necrosis factor-alpha and increased risk of recurrent coronary events after myocardial infarction." Circulation 101(18): 2149-2153.
Roadmap Epigenomics, C., et al. (2015). "Integrative analysis of 111 reference human epigenomes." Nature 518(7539): 317-330.
Rose G, Dato S, Altomare K, Bellizzi D, Garasto S, Greco V, et al. Variability of the SIRT3 gene, human silent information regulator Sir2 homologue, and survivorship in the elderly. Exp Gerontol. 2003;38(10):1065-70.
Sahu, R. P., S. Batra and S. K. Srivastava (2009). "Activation of ATM/Chk1 by curcumin causes cell cycle arrest and apoptosis in human pancreatic cancer cells." Br J Cancer 100(9): 1425-1433.
Sauve, A. A. and V. L. Schramm (2002). "Mechanism-based inhibitors of CD38: a mammalian cyclic ADP-ribose synthetase." Biochemistry 41(26): 8455-8463.
Sauve, A. A., et al. (1998). "The reaction mechanism for CD38. A single intermediate is responsible for cyclization, hydrolysis, and base-exchange chemistries." Biochemistry 37(38): 13239-13249.
Schmeisser, K., J. Mansfeld, D. Kuhlow, S. Weimer, S. Priebe, I. Heiland, M. Birringer, M. Groth, A. Segref, Y. Kanfi, N. L. Price, S. Schmeisser, S. Schuster, A. F. Pfeiffer, R. Guthke, M. Platzer, T. Hoppe, H. Y. Cohen, K. Zarse, D. A. Sinclair and M. Ristow (2013). "Role of sirtuins in lifespan regulation is linked to methylation of nicotinamide." Nat Chem Biol 9(11): 693-700.
Seshadri, S., A. Beiser, J. Selhub, P. F. Jacques, I. H. Rosenberg, R. B. D'Agostino, P. W. Wilson and P. A. Wolf (2002). "Plasma homocysteine as a risk factor for dementia and Alzheimer's disease." N Engl J Med 346(7): 476-483.
Shang, Z., C. Lu, S. Chen, L. Hua and R. Qian (2012). "Effect of H(2)S on the circadian rhythm of mouse hepatocytes." Lipids Health Dis 11: 23.
Sharma A, Diecke S, Zhang WY, Lan F, He C, Mordwinkin NM, et al. The role of SIRT6 protein in aging and reprogramming of human induced pluripotent stem cells. J Biol Chem. 2013;288(25):18439-47.
Shen J, Ma W, Liu Y. Deacetylase SIRT6 deaccelerates endothelial senescence. Cardiovasc Res. 2013;97(3):391-2.
Sies, H. (2014). "Role of metabolic H2O2 generation: redox signaling and oxidative stress." J Biol Chem 289(13): 8735-8741.
Sodha, N. R., R. T. Clements, J. Feng, Y. Liu, C. Bianchi, E. M. Horvath, C. Szabo and F. W. Sellke (2008). "The effects of therapeutic sulfide on myocardial apoptosis in response to ischemia-reperfusion injury." Eur J Cardiothorac Surg 33(5): 906-913.
Sohal RS, Orr WC. The redox stress hypothesis of aging. Free Radic Biol Med. 2012;52(3):539-55.
Someya S, Yu W, Hallows WC, Xu J, Vann JM, Leeuwenburgh C, et al. Sirt3 mediates reduction of oxidative damage and prevention of age-related hearing loss under caloric restriction. Cell. 2010;143(5):802-12.
Stefanson, A. L. and M. Bakovic (2014). "Dietary regulation of Keap1/Nrf2/ARE pathway: focus on plant-derived compounds and trace minerals." Nutrients 6(9): 3777-3801.
Suh JH, Wang H, Liu RM, Liu J, Hagen TM. (R)-alpha-lipoic acid reverses the age-related loss in GSH redox status in post-mitotic tissues: evidence for increased cysteine requirement for GSH synthesis. Arch Biochem Biophys. 2004;423(1):126-35.
Sun, L., et al. (2003). "Disordered osteoclast formation and function in a CD38 (ADP-ribosyl cyclase)-deficient mouse establishes an essential role for CD38 in bone resorption." FASEB J 17(3): 369-375.
Suzuki T, Motohashi H, Yamamoto M. Toward clinical application of the Keap1-Nrf2 pathway. Trends Pharmacol Sci. 2013;34(6):340-6.
Suzuki, T., et al. (2013). "Regulatory nexus of synthesis and degradation deciphers cellular Nrf2 expression levels." Mol Cell Biol 33(12): 2402-2412.
Szabo, C., C. Coletta, C. Chao, K. Modis, B. Szczesny, A. Papapetropoulos and M. R. Hellmich (2013). "Tumor-derived hydrogen sulfide, produced by cystathionine-beta-synthase, stimulates bioenergetics, cell proliferation, and angiogenesis in colon cancer." Proc Natl Acad Sci U S A 110(30): 12474-12479.
Szilard, L. (1959). "On the Nature of the Aging Process." Proc Natl Acad Sci U S A 45(1): 30-45.
Taaffe, D. R., et al. (2000). "Cross-sectional and prospective relationships of interleukin-6 and C-reactive protein with physical performance in elderly persons: MacArthur studies of successful aging." J Gerontol A Biol Sci Med Sci 55(12): M709-715.
Tachibana, T., S. Okazaki, A. Murayama, A. Naganuma, A. Nomoto and S. Kuge (2009). "A major peroxiredoxin-induced activation of Yap1 transcription factor is mediated by reduction-sensitive disulfide bonds and reveals a low level of transcriptional activation." J Biol Chem 284(7): 4464-4472.
Takeuchi K, Kita K, Hayashi S, Aihara E. Regulatory mechanism of duodenal bicarbonate secretion: Roles of endogenous prostaglandins and nitric oxide. Pharmacol Ther. 2011; 130(1):59-70.
Takeuchi, K., et al. (2015). "Muscarinic acetylcholine receptor subtype 4 is essential for cholinergic stimulation of duodenal bicarbonate secretion in mice - relationship to D cell/somatostatin." J Physiol Pharmacol 66(3): 391-401.
Tan M, Peng C, Anderson KA, Chhoy P, Xie Z, Dai L, et al. Lysine glutarylation is a protein posttranslational modification regulated by SIRT5. Cell Metab. 2014;19(4):605-17.
Tang, X. Q., R. Q. Chen, Y. K. Ren, P. D. Soldato, A. Sparatore, Y. Y. Zhuang, H. R. Fang and C. Y. Wang (2011). "ACS6, a Hydrogen sulfide-donating derivative of sildenafil, inhibits homocysteine-induced apoptosis by preservation of mitochondrial function." Med Gas Res 1(1): 20.
Tirumurugaan KG, Kang BN, Panettieri RA, Foster DN, Walseth TF, Kannan MS. Regulation of the cd38 promoter in human airway smooth muscle cells by TNF-alpha and dexamethasone. Respir Res. 2008;9:26.
Tocmo, R., D. Liang, Y. Lin and D. Huang (2015). "Chemical and biochemical mechanisms underlying the cardioprotective roles of dietary organopolysulfides." Front Nutr 2: 1.
Tomczyk, S., K. Fischer, S. Austad and B. Galliot (2015). ", a powerful model for aging studies." Invertebr Reprod Dev 59(sup1): 11-16.
Tu, H. C., D. Ren, G. X. Wang, D. Y. Chen, T. D. Westergard, H. Kim, S. Sasagawa, J. J. Hsieh and E. H. Cheng (2009). "The p53-cathepsin axis cooperates with ROS to activate programmed necrotic death upon DNA damage." Proc Natl Acad Sci U S A 106(4): 1093-1098.
Tyagi, N., K. S. Moshal, U. Sen, T. P. Vacek, M. Kumar, W. M. Hughes, Jr., S. Kundu and S. C. Tyagi (2009). "H2S protects against methionine-induced oxidative stress in brain endothelial cells." Antioxid Redox Signal 11(1): 25-33.
van den Bogaard EH, Bergboer JG, Vonk-Bergers M, van Vlijmen-Willems IM, Hato SV, van der Valk PG, et al. Coal tar induces AHR-dependent skin barrier repair in atopic dermatitis. J Clin Invest. 2013;123(2):917-27.
Vanyushin, B. F. (2005). "Enzymatic DNA methylation is an epigenetic control for genetic functions of the cell." Biochemistry (Mosc) 70(5): 488-499.
Varadhan R, Yao W, Matteini A, Beamer BA, Xue QL, Yang H, et al. Simple biologically informed inflammatory index of two serum cytokines predicts 10 year all-cause mortality in older adults. J Gerontol A Biol Sci Med Sci. 2014;69(2):165-73.
Wakabayashi, N., A. T. Dinkova-Kostova, W. D. Holtzclaw, M. I. Kang, A. Kobayashi, M. Yamamoto, T. W. Kensler and P. Talalay (2004). "Protection against electrophile and oxidant stress by induction of the phase 2 response: fate of cysteines of the Keap1 sensor modified by inducers." Proc Natl Acad Sci U S A 101(7): 2040-2045.
Wallace, J. L. (2010). "Physiological and pathophysiological roles of hydrogen sulfide in the gastrointestinal tract." Antioxid Redox Signal 12(9): 1125-1133.
Wallace, J. L. and R. Wang (2015). "Hydrogen sulfide-based therapeutics: exploiting a unique but ubiquitous gasotransmitter." Nat Rev Drug Discov 14(5): 329-345.
Wang F, Li Y, Cao Y, Li C. Zinc might prevent heat-induced hepatic injury by activating the Nrf2-antioxidant in mice. Biol Trace Elem Res. 2015;165(1):86-95.
Wang Z, Zhang L, Liang Y, Zhang C, Xu Z, Zhang L, et al. Cyclic AMP Mimics the Anti-ageing Effects of Calorie Restriction by Up-Regulating Sirtuin. Sci Rep. 2015;5:12012.
Wang, J., S. Zhang, Y. Wang, L. Chen and X. S. Zhang (2009). "Disease-aging network reveals significant roles of aging genes in connecting genetic diseases." PLoS Comput Biol 5(9): e1000521.
Wang, G., et al. (2014) "P7C3 Neuroprotective Chemicals Function by Activating the Rate-Limiting Enzyme in NAD Salvage." Cell 158: 1324-1334.
Wang, R. (2002). "Two's company, three's a crowd: can H2S be the third endogenous gaseous transmitter?" FASEB J 16(13): 1792-1798.
Wenger, Y. and B. Galliot (2013). "RNAseq versus genome-predicted transcriptomes: a large population of novel transcripts identified in an Illumina-454 Hydra transcriptome." BMC Genomics 14: 204.
Whiteman, M. and P. K. Moore (2009). "Hydrogen sulfide and the vasculature: a novel vasculoprotective entity and regulator of nitric oxide bioavailability?" J Cell Mol Med 13(3): 488-507.
Wilson HL, Dipp M, Thomas JM, Lad C, Galione A, Evans AM. Adp-ribosyl cyclase and cyclic ADP-ribose hydrolase act as a redox sensor. a primary role for cyclic ADP-ribose in hypoxic pulmonary vasoconstriction. J Biol Chem. 2001;276(14):11180-8.
Wintner, E. A., T. L. Deckwerth, W. Langston, A. Bengtsson, D. Leviten, P. Hill, M. A. Insko, R. Dumpit, E. VandenEkart, C. F. Toombs and C. Szabo (2010). "A monobromobimane-based assay to measure the pharmacokinetic profile of reactive sulphide species in blood." Br J Pharmacol 160(4): 941-957.
Wu Y, Chen L, Wang Y, Li W, Lin Y, Yu D, et al. Overexpression of Sirtuin 6 suppresses cellular senescence and NF-kappaB mediated inflammatory responses in osteoarthritis development. Sci Rep. 2015;5:17602.
Wu, C. C. and S. B. Bratton (2013). "Regulation of the intrinsic apoptosis pathway by reactive oxygen species." Antioxid Redox Signal 19(6): 546-558.
Wu, D., Q. Hu, X. Liu, L. Pan, Q. Xiong and Y. Z. Zhu (2015). "Hydrogen sulfide protects against apoptosis under oxidative stress through SIRT1 pathway in H9c2 cardiomyocytes." Nitric Oxide 46: 204-212.
Wu, L. and R. Derynck (2009). "Essential role of TGF-beta signaling in glucose-induced cell hypertrophy." Dev Cell 17(1): 35-48.
Xu, Z., G. Prathapasinghe, N. Wu, S. Y. Hwang, Y. L. Siow and K. O (2009). "Ischemia-reperfusion reduces cystathionine-beta-synthase-mediated hydrogen sulfide generation in the kidney." Am J Physiol Renal Physiol 297(1): F27-35.
Yang, G., L. Wu, B. Jiang, W. Yang, J. Qi, K. Cao, Q. Meng, A. K. Mustafa, W. Mu, S. Zhang, S. H. Snyder and R. Wang (2008). "H2S as a physiologic vasorelaxant: hypertension in mice with deletion of cystathionine gamma-lyase." Science 322(5901): 587-590.
Yao, L. L., X. W. Huang, Y. G. Wang, Y. X. Cao, C. C. Zhang and Y. C. Zhu (2010). "Hydrogen sulfide protects cardiomyocytes from hypoxia/reoxygenation-induced apoptosis by preventing GSK-3beta-dependent opening of mPTP." Am J Physiol Heart Circ Physiol 298(5): H1310-1319.
Yonezawa D, Sekiguchi F, Miyamoto M, Taniguchi E, Honjo M, Masuko T, et al. A protective role of hydrogen sulfide against oxidative stress in rat gastric mucosal epithelium. Toxicology. 2007;241(1-2):11-8.
Yong, Q. C., S. W. Lee, C. S. Foo, K. L. Neo, X. Chen and J. S. Bian (2008). "Endogenous hydrogen sulphide mediates the cardioprotection induced by ischemic postconditioning." Am J Physiol Heart Circ Physiol 295(3): H1330-H1340.
Yoshino, J. and S. Imai (2013). "Accurate measurement of nicotinamide adenine dinucleotide (NAD(+)) with high-performance liquid chromatography." Methods Mol Biol 1077: 203-215.
Yu, Q., Y. V. Katlinskaya, C. J. Carbone, B. Zhao, K. V. Katlinski, H. Zheng, M. Guha, N. Li, Q. Chen, T. Yang, C. J. Lengner, R. A. Greenberg, F. B. Johnson and S. Y. Fuchs (2015). "DNA-damage-induced type I interferon promotes senescence and inhibits stem cell function." Cell Rep 11(5): 785-797.
Yudoh K, Karasawa R, Ishikawa J. Age-related Decrease of Sirtuin 2 Protein in Human Peripheral Blood Mononuclear Cells. Curr Aging Sci. 2015;8(3):256-8.
Zee, R. S., C. B. Yoo, D. R. Pimentel, D. H. Perlman, J. R. Burgoyne, X. Hou, M. E. McComb, C. E. Costello, R. A. Cohen and M. M. Bachschmid (2010). "Redox regulation of sirtuin-1 by S-glutathiolation." Antioxid Redox Signal 13(7): 1023-1032.
Zhang AY, Yi F, Teggatz EG, Zou AP, Li PL. Enhanced production and action of cyclic ADP-ribose during oxidative stress in small bovine coronary arterial smooth muscle. Microvasc Res. 2004;67(2):159-67.
Zhao, W., J. Zhang, Y. Lu and R. Wang (2001). "The vasorelaxant effect of H(2)S as a novel endogenous gaseous K(ATP) channel opener." EMBO J 20(21): 6008-6016.
Zhao, Y., S. Bhushan, C. Yang, H. Otsuka, J. D. Stein, A. Pacheco, B. Peng, N. O. Devarie-Baez, H. C. Aguilar, D. J. Lefer and M. Xian (2013). "Controllable hydrogen sulfide donors and their activity against myocardial ischemia-reperfusion injury." ACS Chem Biol 8(6): 1283-1290.
Zhou T, Kurnasov O, Tomchick DR, Binns DD, Grishin NV, Marquez VE, et al. Structure of human nicotinamide/nicotinic acid mononucleotide adenylyltransferase. Basis for the dual substrate specificity and activation of the oncolytic agent tiazofurin. J Biol Chem. 2002;277(15): 13148-54.

### EXAMPLES

The following examples are set forth below to illustrate the methods, compositions, and results according to the disclosed subject matter. These examples are not intended to be inclusive of all aspects of the subject matter disclosed herein, but rather to illustrate representative methods, compositions, and results.

A 61 year old Caucasian male weighing 88 kg at the beginning of the treatment was treated with a regimine of category 1, category 2, and category 3 molecules as noted below.
Nicotinamide mononucleotide (NMN) (MW=334.22)
Betaine (trimethyl glycine) (MW =117.14)
H₂O₂ (MW=34.01)
NaSH (MW=56.06)

Solutions of various compounds were produced for administering to the subject by mixing a set number of grams with 500 mL of water.

Typical final concentrations of NMN taken by subject were 3.5 grams in 500 mL H₂O, betaine were 3 grams in 500 mL H₂O, H₂O₂ were (2 drops of 35% concentration in 500 mL H₂O), and NaSH were (drops of 2 at 66uM per drop concentration in 500 mL H₂O).

The amounts of each composition were set so that by the subject drinking the full 500 mL a final dosage approximately 1.19 × 10⁻⁴ moles NMN/ kg body weight per dose, 2.91 × 10⁻⁴ moles betaine / kg body weight per dose, 1.17 × 10⁻⁵ moles of H₂O₂ / kg of body weight per dose, and 1.51 × 10⁻⁶ moles of NaSH / kg of body weight per dose was given to the subject through drinking the 500 mL solution.

By taking two similar dosages per day, the sum of the two daily equal allotments was
- Nicotinamide Mononucleotide (NMN) dosage -- 2.38 × 10⁻⁴ moles / Kg body weight / day
- The betaine dosage -- 5.82 × 10⁻⁴ moles / Kg body weight / day
- The Hydrogen Peroxide (H₂O₂) dosage -- 2.34 × 10⁻⁵ moles / Kg body weight / day
- The Sodium Hydrogen Sulfide (NaSH) dosage -- 3.02 × 10⁻⁶ moles / Kg body weight / day

The subject was weighed each day.

The subject self-administered the formulations orally through drinking the solution at approximately 7AM and 7 PM each day. These times were chosen because they approximated the subjects' biological clock peaks of NAD+ as determined by Ramsey K 2009. This had the effect of pulsing the ingredients into the body twice a day, approximately timed with the biological clock of the subject.

LabCor Inc. performed the marker testing using standard protocols on a monthly basis. Blood draw times ranged between 8:19 am and 8:54 am. Inflammatory measurements are correlated to the biological clock. LabCor tested levels of CMV IgG, C-Reactive Protein, Tumor Necrosis Factor-Alpha, and Interleukin-6 in Serum.

The subject also had the following data collected monthly at LabCorp, including Serum Glucose, Serum Uric Acid, BUN, Serum Creatinine, eGRF if non African American, BUN/Creatinine Ratio, Serum Sodium, Serum Potassium, Serum Chloride, Total Carbon Dioxide, Serum Calcium, Serum Phosphorus, Serum Total Protein, Serum Albumin, Serum, Total Globulin, A/G Ratio, Total Bilirubin, Serum Alkaline Phosphatase, LDH, AST (SGOT), ALT (SGPT), Serum Iron, Total Cholesterol, Triglycerides, HDL Cholesterol, Calculation VLDL cholesterol Calculation LDL Cholesterol, Total Cholesterol/ HDL ratio, Estimated CHD risk, White Blood Cells, Red Blood Cells, Hemoglobin, Hematocrit, MCV, MCH, MCHC, RDW, Platelets, Neutrophils, Lymphs, Monocytes, Eos, Basos, Immature Cells, Neutrophils (Absolute). Lymphs (Absolute), Monocytes (Absolute), Eos (Absolute), Baso (Absolute), Immature Granulocytes, Immature Grans (Absolute), NRBC, VAP Cholesterol Profile, LDL Cholesterol, HDL Cholesterol, VLDL Cholesterol, Cholesterol total, Triglycerides, Non HDL Cholesterol (LDL+VLDL), ApoB 100=Calculation, LDL-R (Real)-C, Lp(a) Cholesterol, IDL Cholesterol, Remnant Lipo (IDL+VLDL3), Probable Metabolic Syndrome, HDL-2 (most Protective), HDL-3 (Less Protective), VLDL-3 (Small Remnant), LDL1 Pattern A, LDL2 Pattern A, LDL3 Pattern B, LDL4 Pattern B, LDL Density Pattern, Glucose Tolerance (4 Sp Blood), Glucose Fasting, Glucose 1 hour, Glucose 2 hours, Glucose 3 hours, Insulin Fasting, Insulin 1 hour, Insulin 2 hours, Insulin 3 hours, Cortisol AM, Cortisol PM, IL-1b (Serum), Hemoglobin A1c, Rheumatoid Arthritis Factor, IGF-1, Cardiac, Tumor Interleukin-8 (Serum), Homocyst(e)ine (Plasma), Antinuclear Antibodies direct, Sedimentation Rate-Westergren Cortisol, (Urinary Free), Cortisol, F, ug, L, U, Cortisol, Fug, 24hr,U, Serum Immunoglobulin G, Qn, Serum Immunoglobulin A, Qn, Serum Immunoglobulin M, Qn, oxLDL, CMV IgM, Ferritin, and H. pylori IgG.

University of California, San Diego measured:
a. Spectral 3 tesla MRI of right calf leg muscle before, during, and after exercise
b. Spectral 3 tesla MRI of Liver
c. Structural 3 tesla MRI of Liver
d. Spectral 3 tesla MRI of Brain (front and back)
e. A structural 3 tesla MRI of Brain
f. A structural 3 tesla MRI of the right knee (showing Arthritis)
g. 3-Nitrotyrosine (a marker for oxidative / nitrative stress)
h. Coagulation Tests (a marker for oxidative stress)
i. F2-isoprostanes (a marker for oxidative / nitrative stress,)
j. GSH: GSSH (a marker for and protection from oxidative / nitrative stress)
k. Urine Organic Acids
l. 8-hydroxydeooxyguanosine (8-OHDG) (a marker for oxidative / nitrative stress)
m. Malondialdehyde (a marker for oxidative / nitrative stress)
n. hsCRP ( a marker that can be adversely affected by oxidative stress)
o. Proteomic profile (a marker for oxidative / nitrative stress)

A list of medical history questions (UCSD) were answered. Body fat and mineral testing was performed at private MD's office. Treadmill testing was performed at private MD's office. 4 tissue biopsy types (liver (needle biopsy), skin; adipose, muscle) were obtained (stored at -80 C at UCLA). A log of daily exercise and weight was obtained. Also weekly glucose monitoring before and after NMN and BP monitoring before and after NMN was obtained.

### Results

**Table 1.**

| | | | | | | 61 year old Male Caucasian | | | | |
|---|---|---|---|---|---|---|---|---|---|---|
| With the additions of | | | | | | | | | | |
| NMN | | | | | X | X | X | X | X | X |
| Betaine | | | | | | | | X | X | X |
| H₂O₂ | | | | | | | | | X | |
| NaSH | | | | | | | | | | X |

| | | Normal Range Low | Normal Range High | Baseline | | | | | | |
|---|---|---|---|---|---|---|---|---|---|---|
| CMV IgG | | 0 | 0 | 0 | 0 | 0 | 0 | 0 | 0 | 0 |
| C-Reactive Protein | mg/L | 0 | 3 | 2.77 | 3.25 | 0.43 | 0.53 | 0.85 | 0.21 | 0.40 |
| Tumor Necrosis Factor-Alpha | pg/mL | 0 | 8.1 | 1.1 | 0.9 | 1.1 | 1.1 | 1 | 0.5 | 0.3 |
| Interleukin-6 Serum | pg/mL | 0 | 15.5 | 1.3 | 4.4 | <0.7 | 0.9 | 3.1 | <0.7 | <0.7 |
| Inflammation Score | | 0 | 26.6 | 5.17 | 8.55 | 2.23 | 2.53 | 4.95 | 1.41 | 1.40 |

The results of the monthly administration schedule and testing for the subject are presented in Table 1. Table 1 shows that the subject was provided a formulation on a monthly basis, where the formulation included NMN alone for 3 months (comparative), NMN+ betaine for one month (comparative), NMN+ betaine + H₂O₂ for one month and NMN+ betaine +NaSH for one month.

Other observations of interest during study are that the subject was healthy during the full duration of the study. Photos depicted that aged skin cells on hand became youthful in appearance. The subject's complexion of facial skin improved during study. The subject had significant weight loss and apatite was lowered during study. The subject had an elimination of pain from arthritis in right knee during study. The subject had more restful sleep during study. The subject had increased energy during study. The subject had better vision at eye exam.

### Discussion

The age of 61 correlates to the age of unrelated and offspring families in the Arai Y 2015 study detailed herein. The results of this study, in light of the Arai Y 2015 study, show that the triple therapy with the three categories of compounds change the predicted outcome, as identified by Arai 2015, of this 61 year old 88 kg Caucasian male from unsuccessful aging to a prediction of successful aging. In the baseline condition for the subject, both C-reactive Protein (2.77 mg/L) and Interleukin-6 (1.3 pg/mL) measurements were above the "unrelated family" level (0.7 mg/l and 1.13 pg/mL) (Arai Y 2015, Table 1) as well as the "offspring" level (0.7 mg/l and 1.03 pg/mL) (Arai Y. 2015, Table 1) respectively. The 61 male subject of this study has a similar age to the "offspring" group and the "unrelated family" group of Arai. These two inflammation test scores effect the prediction algorithm to predict a worse aging outcome for the 61 year old subject than the "offspring" or "unrelated family" groups of Arai at baseline.

After two months of treatment with NMN, however, the markers of the 61 year old subject were brought to levels better than the "offspring" group of Arai (CRP, 0.43 mg/l and IL-6, less than 0.7 pg/mL). While both of these markers do rise slightly in month one, the overall effect of the NMN treatment is to reduce the levels of these markers. The lower or approximately similar levels to the "offspring" group of Arai continued to be produced by administration of NMN through months 3, but the effect seemingly plateaus in the 61 year old male.

All three inflammatory markers drop to their lowest level with the addition of all three categories of ingredients. IL-6 drops to undetectable levels, TNF-alpha drops by over 50%, and CRP drops to about a tenth of the original value. When H₂O₂ is used for the category 3 ingredient in this example CRP drops more than when NaSH is used and when NaSH is used as the category 3 ingredient TNF-alpha dropped more than when H₂O₂ is used. In both cases of triple therapy the results are far below the necessary levels to predict very successful aging. CMV titers were not discussed here since this 61 year old male had no or undetectable levels of CMV IgG and this is as good as the measured value of this variable can get.

When the interventional therapy for this 61 year old male in this experiment is compared to the results gained by one or two years of calorie restriction one can see that the results are far greater with this triple category therapy and they are far easier to obtain (Di Francesco A 2015, Ravussin E 2015).

### Correlations by other authors to human health improvements from the lowering TNF-alpha and IL-6 which were lowered in this example;

### Other studies (similar to Arai Y 2015)

Immune markers (a simple index of serum interleukin-6 (IL-6) and tumor necrosis factor alpha (TNF-alpha) two of the Arai 4 markers) were found to be the best predictor of mortality in 1,155 older adults in a 10 year all-cause mortality study after adjusting for variables already known to cause death (Varadhan R 2014). A single immune marker (Serum IL-6) predicted all-cause mortality, cancer, cardiovascular disease and liver disease in a 1843 person prospective cohort study (Lee JK 2012). These studies confirmed results in smaller prior studies (Derhovanessian E 2010, Reuben DB 2002, Taaffe DR 2000).

### Possible mechanism of action:

In December 2013, A. Gomes et al, published a study demonstrating that raising the levels of NAD+ with precursor NMN in old mice restores mitochondrial function to that of a young mouse. C. Correia-Melo showed with age mitochondria drive a cellular proinflammatory phenotype including IL-6 secretion.

### Immune Dysfunction:

In July 2014, I. V. Astrakhantseva et al, issued a report showing the benefits of reducing the levels of TNF and IL-6 as effective ways to control inflammation symptoms such as joint destruction and autoimmune diseases. A. Puchta et al, hypothesized a molecular mechanism using these two inflammation variables (TNF and IL-6) for predictive effects on life span and health span. The study showed how TNF increasingly drives immune dysfunction with age and that lowering the levels of TNF decrease this impairment.

### Brain Disease:

In September 2014 Brianne Bettcher et al, published a study indicating that at older ages, there is a positive correlation between increased levels of IL-6 and lowered white matter function in the brain. In February 2015, Brianne Bettcher et al, published a study showing that reducing systemic inflammation had a positive effects on cognition and brain structure which may reverse neurodegenerative disease processes.

### Heart disease:

In 2000, Paul Ridker et al, published 2 studies concluding that in apparently healthy men, elevated levels of IL-6 is associated in increased risk of future Myocardial Infarction and TNF increases the risk of recurrent coronary events after Myocardial Infarction. In August 2005, NJ Goodson et al, published a study linking increased levels of C-Reactive Protein with a prediction of death from cardiovascular disease.

### Kidney Disease:

In 2015, Belinda Lee et al, published a study demonstrating the association between elevated levels of CRP, TNF and IL-6 with chronic kidney disease.

### Alzheimer's Disease:

Lowering TNF-alpha and IL-6 lowers the chance of getting Alzheimer's disease and lowers the negative effects of Alzheimer's disease (Butchart J 2015, Holmes C 2011). Adding NMN in a mouse Alzheimer's disease model was beneficial (Long AN 2015).

### Research into the potential benefits of lowering TNF-alpha and IL-6 for a more effective immune response to viruses and bacteria:

McElroy AK, after analyzing the kinetics of inflammatory signaling in life threatening human Ebola Virus disease, proposed the possible therapeutic benefit of lowering the proinflammatory signaling of IL-6 for clinical intervention of these patients. A. Puchta proposed the possible therapeutic benefit of lowering IL-6 and TNF alpha to increase the ability to fight Streptococcus pneumoniae.

### Research correlation of the potential benefits of lowering TNF-alpha and IL-6 to better physical performance.

Cesari M in 2004 concluded higher levels of IL-6 was correlated to lower physical performance in older adults and a target for intervention. Puzianowska-Kuznicka M showed Il-6 and CRP were good predictors of physical and cognitive performance and the risk of mortality in 3496 individuals.

### Sleep:

Irwin MR correlated sleep disturbances to increased CRP and IL-6 but not TNF in a meta-analysis of 72 previous sleep studies.

## Claims

1. A nutritional composition for administering to a subject, composition, comprising:
a repair system activator chosen from, nicotinamide adenine dinucleotide (NAD+), nicotinamide mononucleotide (NMN), nicotinamide riboside (NR), nicotinic acid adenine mononucleotide (NaMN), nicotinic acid adenine dinucleotide (NaAD), nicotinic acid riboside (NAR), 1-methylnicotinamide (MNM), cyclic adenosine monophosphate (cAMP), and any combination thereof;
a methyl donor chosen from, S-5'-adenosyl-L-methionine (SAM), methionine, betaine, choline, folate, vitamin B12, and any combination thereof; and
an antioxidant defense activator chosen from H₂O₂, H₂S, NaSH, Na₂S, metformin, curcumin, sulforaphane, quercetin, apigenin, pterostilbene, resveratrol, zinc, and any combination thereof; and optionally
water.

2. The composition of claim 1, wherein the repair system activator, the methyl donor, and the antioxidant defense activator are at least 5 wt.% of the composition.

3. The composition of claim 1, wherein the repair system activator is nicotinamide mononucleotide (NMN), nicotinamide riboside (NR), or both.

4. The composition of claim 1, wherein the methyl donor is methionine, betaine, or both.

5. The composition of claim 1, wherein the antioxidant defense activator is H₂O₂, H₂S, or NaSH.

6. The composition of claim 1, wherein the repair system activator, methyl donor, and antioxidant defense activator are in an amount sufficient to beneficially change a surrogate marker for aging level in a human when compared to the surrogate marker level prior to administration.

7. The composition of claim 6 wherein the change in the level of the surrogate marker is lowered.

8. The composition of claim 7, wherein the surrogate marker is CMV IgG, C-Reactive Protein, Tumor Necrosis Factor-Alpha, or Interleukin-6.

9. The composition of claim 6, wherein the change in the level of the surrogate marker is increased.

10. The composition of claim 9, wherein the surrogate marker is DNA methylation.

11. The composition of claim 1, wherein the composition comprises at least 1 × 10⁻⁸ moles of the repair system activator, at least 1 × 10⁻⁸ moles of the methyl donor, and at least 1 × 10⁻⁹ moles of the antioxidant defense activator.

12. The composition of claim 1, wherein the composition comprises nicotinamide mononucleotide (NMN), Betaine, and H₂O₂.

13. A tablet comprising the composition of claim 1.

14. The composition of claims 1 to 12 for use in reducing inflammation in a subject, preferably a human subject.

15. The composition for use according to claim 14, wherein the composition is administered to a subject at a dosage of at least 1 × 10⁻⁶ moles /kg of the repair system activator to the subject, 1 × 10⁻⁶ moles /kg of the methyl donor to the subject, and 1 × 10⁻⁷ moles /kg of the antioxidant defense activator to the subject.

16. The composition for use according to claim 14, wherein the composition is injected over 8-12 days.

17. The composition for use according to claim 14, wherein the composition is in an aerosol, lyophilized, powder, or emulsion form.

18. The composition for use according to claim 14, wherein the composition is administered to a human subject for at least two months.

19. The composition for use according to claim 14, wherein the composition is in a tablet that is administered orally at least once daily.

20. The composition for use according to claim 14, wherein the composition is administered to the subject once daily.

21. A product comprising:
a repair system activator chosen from nicotinamide adenine dinucleotide (NAD+), nicotinamide mononucleotide (NMN), nicotinamide riboside (NR), nicotinic acid adenine mononucleotide (NaMN), nicotinic acid adenine dinucleotide (NaAD), nicotinic acid riboside (NAR), 1-methylnicotinamide (MNM), cyclic adenosine monophosphate (cAMP), and any combination thereof;
a methyl donor chosen from, S-5'-adenosyl-L-methionine (SAM), methionine, betaine, choline, folate, vitamin B12, and any combination thereof; and
an antioxidant defense activator chosen from H₂O₂, H₂S, NaSH, Na₂S, metformin, curcumin, sulforaphane, quercetin, apigenin, pterostilbene, resveratrol, zinc, and any combination thereof,
for use in reducing inflammation in a subject, preferably a human, wherein the repair system activator, the methyl donor, and the antioxidant defense activator are administered at approximately the same time or at different times.

22. The product for use according to claim 21, wherein the repair system activator is administered within 15, 30, 60, 90, or 120 minutes of the subject's biological clock NAD+ peak.

23. The product for use according to claim 21, wherein the repair system activator, the methyl donor, and the antioxidant defense activator are administered to a human subject for at least two months.

24. The product for use according to claim 21, wherein the repair system activator, the methyl donor, and the antioxidant defense activator are administered to a human subject once daily.

## Patentansprüche

1. Ernährungszusammensetzung zur Verabreichung an ein Subjekt, wobei die Zusammensetzung umfasst:
einen Reparatursystemaktivator, der aus Nicotinamid-Adenin-Dinukleotid (NAD+), Nicotinamid-Mononukleotid (NMN), Nicotinamid-Ribosid (NR), Nicotinsäure-Adenin-Mononukleotid (NaMN), Nicotinsäure-Adenin-Dinukleotid (NaAD), Nicotinsäure-Ribosid (NAR), 1-Methylnicotinamid (MNM), zyklischem Adenosin-Monophosphat (cAMP) und einer beliebigen Kombination davon gewählt wird;
einen Methyldonor, der aus S-5'-Adenosyl-L-Methionin (SAM), Methionin, Betain, Cholin, Folsäure, Vitamin B12 und einer beliebigen Kombination davon gewählt wird; und
ein Antioxidansabwehraktivator, der aus H₂O₂, H₂S, NaSH, Na₂S, Metformin, Curcumin, Sulforaphan, Quercetin, Apigenin, Pterostilben, Resveratrol, Zink und einer beliebigen Kombination davon gewählt wird; und optional
Wasser.

2. Zusammensetzung nach Anspruch 1, wobei der Reparatursystemaktivator, der Methyldonor und der Antioxidansabwehraktivator mindestens 5 Gew.-% der Zusammensetzung bilden.

3. Zusammensetzung nach Anspruch 1, wobei der Reparatursystemaktivator Nicotinamid-Mononukleotid (NMN), Nicotinamid-Ribosid (NR) oder beides ist.

4. Zusammensetzung nach Anspruch 1, wobei der Methyldonor Methionin, Betain oder beides ist.

5. Zusammensetzung nach Anspruch 1, wobei der Antioxidansabwehraktivator H₂O₂, H₂S oder NaSH ist.

6. Zusammensetzung nach Anspruch 1, wobei der Reparatursystemaktivator, Methyldonor und Antioxidansabwehraktivator in einer Menge vorliegen, die ausreicht, um einen Surrogatmarker für einen Alterungsgrad bei einem Menschen verglichen mit dem Level des Surrogatmarkers vor Verabreichung vorteilhaft zu verändern.

7. Zusammensetzung nach Anspruch 6, wobei die Änderung des Levels des Surrogatmarkers verringert ist.

8. Zusammensetzung nach Anspruch 7, wobei der Surrogatmarker CMV IgG, C-reaktives Protein, Tumor-Nekrose-Faktor-Alpha oder Interleukin-6 ist.

9. Zusammensetzung nach Anspruch 6, wobei die Änderung des Levels des Surrogatmarkers erhöht ist.

10. Zusammensetzung nach Anspruch 9, wobei der Surrogatmarker DNA-Methylierung ist.

11. Zusammensetzung nach Anspruch 1, wobei die Zusammensetzung mindestens 1 × 10⁻⁸ Mol des Reparatursystemaktivators, mindestens 1 × 10⁻⁸ Mol des Methyldonors und mindestens 1 × 10⁻⁹ Mol des Antioxidansabwehraktivators umfasst.

12. Zusammensetzung nach Anspruch 1, wobei die Zusammensetzung Nicotinamid-Mononukleotid (NMN), Betain und H₂O₂ umfasst.

13. Tablette, die die Zusammensetzung nach Anspruch 1 umfasst.

14. Zusammensetzung nach den Ansprüchen 1 bis 12 zur Verwendung beim Verringern von Entzündungen in einem Subjekt, bevorzugt einem menschlichen Subjekt.

15. Zusammensetzung zur Verwendung nach Anspruch 14, wobei die Zusammensetzung einem Subjekt mit einer Dosierung von mindestens 1 × 10⁻⁶ Mol/kg des Reparatursystemaktivators zu dem Subjekt, 1 × 10⁻⁶ Mol/kg des Methyldonors zu dem Subjekt und 1 × 10⁻⁷ Mol/kg des Antioxidansabwehraktivators zu dem Subjekt verabreicht wird.

16. Zusammensetzung zur Verwendung nach Anspruch 14, wobei die Zusammensetzung über 8-12 Tage injiziert wird.

17. Zusammensetzung zur Verwendung nach Anspruch 14, wobei die Zusammensetzung in Aerosol-, lyophilisierter, Pulver- oder Emulsionsform ist.

18. Zusammensetzung zur Verwendung nach Anspruch 14, wobei die Zusammensetzung einem menschlichen Subjekt für mindestens zwei Monate verabreicht wird.

19. Zusammensetzung zur Verwendung nach Anspruch 14, wobei die Zusammensetzung in einer Tablette ist, die mindestens einmal täglich oral verabreicht wird.

20. Zusammensetzung zur Verwendung nach Anspruch 14, wobei die Zusammensetzung dem Subjekt einmal täglich verabreicht wird.

21. Produkt, umfassend:
einen Reparatursystemaktivator, der aus Nicotinamid-Adenin-Dinukleotid (NAD+), Nicotinamid-Mononukleotid (NMN), Nicotinamid-Ribosid (NR), Nicotinsäure-Adenin-Mononukleotid (NaMN), Nicotinsäure-Adenin-Dinukleotid (NaAD), Nicotinsäure-Ribosid (NAR), 1-Methylnicotinamid (MNM), zyklischem Adenosin-Monophosphat (cAMP) und einer beliebigen Kombination davon gewählt wird;
einen Methyldonor, der aus S-5'-Adenosyl-L-Methionin (SAM), Methionin, Betain, Cholin, Folsäure, Vitamin B12 und einer beliebigen Kombination davon gewählt wird; und
ein Antioxidansabwehraktivator, der aus H₂O₂, H₂S, NaSH, Na₂S, Metformin, Curcumin, Sulforaphan, Quercetin, Apigenin, Pterostilben, Resveratrol, Zink und einer beliebigen Kombination davon gewählt wird,
zur Verwendung beim Verringern einer Entzündung in einem Subjekt, bevorzugt einem Menschen, wobei der Reparatursystemaktivator, der Methyldonor und der Antioxidansabwehraktivator etwa zur gleichen Zeit oder zu unterschiedlichen Zeiten verabreicht werden.

22. Produkt zur Verwendung nach Anspruch 21, wobei der Reparatursystemaktivator innerhalb von 15, 30, 60, 90 oder 120 Minuten der NAD+ Spitze in der biologischen Uhr des Subjekts verabreicht wird.

23. Produkt zur Verwendung nach Anspruch 21, wobei der Reparatursystemaktivator, der Methyldonor und der Antioxidansabwehraktivator einem menschlichen Subjekt für mindestens zwei Monate verabreicht werden.

24. Produkt zur Verwendung nach Anspruch 21, wobei der Reparatursystemaktivator, der Methyldonor und der Antioxidansabwehraktivator einem menschlichen Subjekt einmal täglich verabreicht werden.

## Revendications

1. Composition nutritionnelle à administrer à un sujet, composition comprenant :
un activateur de système de réparation choisi parmi, la nicotinamide adénine dinucléotide (NAD+), le nicotinamide mononucléotide (NMN), la nicotinamide riboside (NR), l'acide nicotinique adénine mononucléotide (NaMN), l'acide nicotinique adénine dinucléotide (NaAD), l'acide nicotinique riboside (NAR), le 1-méthylnicotinamide (MNM), l'adénosine monophosphate cyclique (cAMP), et une quelconque combinaison de ceux-ci ; et
un donneur de méthyle choisi parmi la S-5'-adénosyl-L-méthionine (SAM), la méthionine, la bétaïne, la choline, le folate, la vitamine B12, ou une quelconque combinaison de ceux-ci ; et
un activateur de défense antioxydante choisi parmi H₂O₂, H₂S, NaSH, Na₂S, la métformine, la curcumine, le sulforaphane, la quercétine, l'apigénine, le ptérostilbène, le resvératrol, le zinc, et une quelconque combinaison de ceux-ci ; et éventuellement
de l'eau.

2. Composition selon la revendication 1, dans laquelle l'activateur de système de réparation, le donneur de méthyle et l'activateur de défense antioxydante représentent au moins 5 % en poids de la composition.

3. Composition selon la revendication 1, dans laquelle l'activateur de système de réparation est le nicotinamide mononucléotide (NMN), la nicotinamide riboside (NR), ou les deux.

4. Composition selon la revendication 1, dans laquelle le donneur de méthyle est la méthionine, la bétaïne, ou les deux.

5. Composition selon la revendication 1, dans laquelle l'activateur de défense antioxydante est H₂O₂, H₂S, ou NaSH.

6. Composition selon la revendication 1, dans laquelle l'activateur de système de réparation, le donneur de méthyle et l'activateur de défense antioxydante sont en une quantité suffisante pour modifier de façon bénéfique un niveau de marqueur de substitution relatif au vieillissement chez un humain par rapport au niveau de marquer de substitution avant administration.

7. Composition selon la revendication 6, dans laquelle la modification du niveau du marqueur de substitution est abaissée.

8. Composition selon la revendication 7, dans laquelle le marqueur de substitution est le CMV IgG, la protéine C-réactive, le facteur de nécrose tumorale alpha, ou l'interleukine-6.

9. Composition selon la revendication 6, dans laquelle la modification du niveau du marqueur de substitution est accrue.

10. Composition selon la revendication 9, dans laquelle le marqueur de substitution est la méthylation de l'ADN.

11. Composition selon la revendication 1, dans laquelle la composition comprend au moins 1 × 10⁻⁸ mole de l'activateur de système de réparation, au moins 1×10⁻⁸ mole du donneur de méthyle et au moins 1 × 10⁻⁹ mole de l'activateur de défense antioxydante.

12. Composition selon la revendication 1, dans laquelle la composition comprend le nicotinamide mononucléotide (NMN), la bétaïne, et le H₂O₂.

13. Comprimé comprenant la composition 1.

14. Composition selon les revendications 1 à 12, pour utilisation dans la réduction de l'inflammation chez un sujet, de préférence un sujet humain.

15. Composition pour utilisation selon la revendication 14, dans laquelle la composition est administrée à un sujet à raison d'au moins 1 × 10⁻⁶ mole/kg de l'activateur de système de réparation au sujet, 1 × 10⁻⁶ mole/kg du donneur de méthyle au sujet, et 1 × 10⁻⁷ mole/kg de l'activateur de défense antioxydante au sujet.

16. Composition pour utilisation selon la revendication 14, dans laquelle la composition est injectée sur 8-12 jours.

17. Composition pour utilisation selon la revendication 14, dans laquelle la composition est sous forme aérosol, lyophilisée, pulvérulente ou d'émulsion.

18. Composition pour utilisation selon la revendication 14, dans laquelle la composition est administrée à un sujet humain pendant au moins deux mois.

19. Composition pour utilisation selon la revendication 14, dans laquelle la composition est sous forme de comprimé qui est administré par voie orale au moins une fois par jour.

20. Composition pour utilisation selon la revendication 14, dans laquelle la composition est administrée au sujet une fois par jour.

21. Produit comprenant :
un activateur de système de réparation choisi parmi la nicotinamide adénine dinucléotide (NAD+), le nicotinamide mononucléotide (NMN), la nicotinamide riboside (NR), l'acide nicotinique adénine mononucléotide (NaMN), l'acide nicotinique adénine dinucléotide (NaAD), l'acide nicotinique riboside (NAR), le 1-méthylnicotinamide (MNM), l'adénosine monophosphate cyclique (cAMP), et une quelconque combinaison de ceux-ci ;
un donneur de méthyle choisi parmi la S-5'-adénosyl-L-méthionine (SAM), la méthionine, la bétaïne, la choline, le folate, la vitamine B12, et une quelconque combinaison de ceux-ci ;
un activateur de défense antioxydante choisi parmi H₂O₂, H₂S, NaSH, Na₂S, la métformine, la curcumine, le sulforaphane, la quercétine, l'apigénine, le ptérostilbène, le resvératrol, le zinc, et une quelconque combinaison de ceux-ci,
pour utilisation dans la réduction de l'inflammation chez un sujet, de préférence un humain, dans lequel l'activateur de système de réparation, le donneur de méthyle et l'activateur de défense antioxydante sont administrés approximativement au même moment ou à des moments différents.

22. Produit pour utilisation selon la revendication 21, dans lequel l'activateur de système de réparation est administré dans les 15, 30, 60, 90, ou 120 minutes du pic de NAD+ d'horloge biologique du sujet.

23. Produit pour utilisation selon la revendication 21, dans lequel l'activateur de système de réparation, le donneur de méthyle et l'activateur de défense antioxydante sont administrés à un sujet humain pendant au moins deux mois.

24. Produit pour utilisation selon la revendication 21, dans lequel l'activateur de système de réparation, le donneur de méthyle et l'activateur de défense antioxydante sont administrés à un sujet humain une fois par jour.
